# EUROPEAN PATENT SPECIFICATION

(11) **EP 1 203 077 B1**
(45) Date of publication and mention of the grant of the patent: **07.01.2009**
(21) Application number: 00954055.0
(22) Date of filing: 11.08.2000
(51) Int. Cl.: C12N 9/52, C12N 15/82, C07K 14/24, C12N 15/11

(54) **TRANSGENIC PLANTS EXPRESSING i PHOTORHABDUS /i TOXIN**
I(PHOTORHABDUS) TOXIN-EXPRIMIERENDE TRANSGENE PFLANZEN
PLANTES TRANSGENIQUES EXPRIMANT LA TOXINE i PHOTORHABDUS /i

(30) Priority: 11.08.1999 US 148356 P
(43) Date of publication of application: 08.05.2002
(73) Proprietor: Dow AgroSciences LLC, Indianapolis, Indiana 46268 (US)
(72) Inventor: PETELL, James, K., Grass Valley, CA 95949 (US); MERLO, Donald, J., Carmel, IN 46032 (US); HERMAN, Rod, A., New Ross, IN 47968 (US); ROBERTS, Jean, L., Arcadia, IN 46030 (US); GUO, Lining, Morrissville, NC 27560 (US); SCHAFER, Barry, W., Cicero, IN 46034 (US); SUKHAPINDA, Kitisri, Zionsville, IN 46077 (US); MERLO, Ann, Owens, Carmel, IN 46032 (US)
(74) Representative: Raynor, John
(86) International application number: PCT/US2000/022237
(87) International publication number: WO 2001/011029

(56) References cited:
- WO-A-97/13402
- WO-A-98/08932

### SEQUENCE LISTING

<110> Petell, Jim
   Merlo, Donald
   Herman, Rod
   Roberts, Jean
   Guo, Lining
   Schafer, Barry
   Sukhapinda, Kitisri
   Owens Merlo, Ann
<120> Transgenic Plants Expressing Photorhabdus Toxin
<130> 50698
<140>
   <141>
<150> US 60/148,356
   <151> 1999-08-11
<160> 8
<170> PatentIn Ver. 2.0
<210> 1
   <211> 7551
   <212> DNA
   <213> Photorhabdus luminescens
<220>
   <221> CDS
   <222> (1)..(7598)
<400> 1
   atg aac gag tct gta aaa gag ata cct gat gta tta aaa agc cag tgt    48
   Met Asn Glu Ser Val Lys Glu Ile Pro Asp Val Leu Lys Ser Gln Cys
     1               5                  10                  15
   ggt ttt aat tgt ctg aca gat att agc cac agc tct ttt aat gaa ttt    96
   Gly Phe Asn Cys Leu Thr Asp Ile Ser His Ser Ser Phe Asn Glu Phe
                20                  25                  30
   cgc cag caa gta tct gag cac ctc tcc tgg tcc gaa aca cac gac tta   144
   Arg Gln Gln Val Ser Glu His Leu Ser Trp Ser Glu Thr His Asp Leu
            35                  40                  45
   tat cat gat gca caa cag gca caa aag gat aat cgc ctg tat gaa gcg   192
   Tyr His Asp Ala Gln Gln Ala Gln Lys Asp Asn Arg Leu Tyr Glu Ala
        50                  55                  60
   cgt att ctc aaa cgc gcc aat ccc caa tta caa aat gcg gtg cat ctt   240
   Arg Ile Leu Lys Arg Ala Asn Pro Gln Leu Gln Asn Ala Val His Leu
    65                  70                  75                  80
   gcc att ctc gct ccc aat gct gaa ctg ata ggc tat aac aat caa ttt   288
   Ala Ile Leu Ala Pro Asn Ala Glu Leu Ile Gly Tyr Asn Asn Gln Phe
                    85                  90                  95
   agc ggt aga gcc agt caa tat gtt gcg ccg ggt acc gtt tct tcc atg   336
   Ser Gly Arg Ala Ser Gln Tyr Val Ala Pro Gly Thr Val Ser Ser Met
               100                 105                 110
   ttc tcc ccc gcc gct tat ttg act gaa ctt tat cgt gaa gca cgc aat     384
   Phe Ser Pro Ala Ala Tyr Leu Thr Glu Leu Tyr Arg Glu Ala Arg Asn
           115                 120                 125
   tta cac gca agt gac tcc gtt tat tat ctg gat acc cgc cgc cca gat     432
   Leu His Ala Ser Asp Ser Val Tyr Tyr Leu Asp Thr Arg Arg Pro Asp
       130                 135                 140
   ctc aaa tca atg gcg ctc agt cag caa aat atg gat ata gaa tta tcc     480
   Leu Lys Ser Met Ala Leu Ser Gln Gln Asn Met Asp Ile Glu Leu Ser
   145                 150                 155                 160
   aca ctc tct ttg tcc aat gag ctg tta ttg gaa agc att aaa act gaa     528
   Thr Leu Ser Leu Ser Asn Glu Leu Leu Leu Glu Ser Ile Lys Thr Glu
                   165                 170                 175
   tct aaa ctg gaa aac tat act aaa gtg atg gaa atg ctc tcc act ttc     576
   Ser Lys Leu Glu Asn Tyr Thr Lys Val Met Glu Met Leu Ser Thr Phe
               180                 185                 190
   cgt cct tcc ggc gca acg cct tat cat gat gct tat gaa aat gtg cgt     624
   Arg Pro Ser Gly Ala Thr Pro Tyr His Asp Ala Tyr Glu Asn Val Arg
           195                 200                 205
   gaa gtt atc cag cta caa gat cct gga ctt gag caa ctc aat gca tca     672
   Glu Val Ile Gln Leu Gln Asp Pro Gly Leu Glu Gln Leu Asn Ala Ser
       210                 215                 220
   ccg gca att gcc ggg ttg atg cat caa gcc tcc cta ttg ggt att aac     720
   Pro Ala Ile Ala Gly Leu Met His Gln Ala Ser Leu Leu Gly Ile Asn
   225                 230                 235                 240
   gct tca atc tcg cct gag cta ttt aat att ctg acg gag gag att acc     768
   Ala Ser Ile Ser Pro Glu Leu Phe Asn Ile Leu Thr Glu Glu Ile Thr
                   245                 250                 255
   gaa ggt aat gct gag gaa ctt tat aag aaa aat ttt ggt aat atc gaa     816
   Glu Gly Asn Ala Glu Glu Leu Tyr Lys Lys Asn Phe Gly Asn Ile Glu
               260                 265                 270
   ccg gcc tca ttg gct atg ccg gaa tac ctt aaa cgt tat tat aat tta     864
   Pro Ala Ser Leu Ala Met Pro Glu Tyr Leu Lys Arg Tyr Tyr Asn Leu
           275                 280                 285
   agc gat gaa gaa ctt agt cag ttt att ggt aaa gcc agc aat ttt ggt     912
   Ser Asp Glu Glu Leu Ser Gln Phe Ile Gly Lys Ala Ser Asn Phe Gly
       290                 295                 300
   caa cag gaa tat agt aat aac caa ctt att act ccg gta gtc aac agc     960
   Gln Gln Glu Tyr Ser Asn Asn Gln Leu Ile Thr Pro Val Val Asn Ser
   305                 310                 315                 320
   agt gat ggc acg gtt aag gta tat cgg atc acc cgc gaa tat aca acc     1008
   Ser Asp Gly Thr Val Lys Val Tyr Arg Ile Thr Arg Glu Tyr Thr Thr
                   325                 330                 335
   aat gct tat caa atg gat gtg gag cta ttt ccc ttc ggt ggt gag aat     1056
   Asn Ala Tyr Gln Met Asp Val Glu Leu Phe Pro Phe Gly Gly Glu Asn
               340                 345                 350
   tat cgg tta gat tat aaa ttc aaa aat ttt tat aat gcc tct tat tta   1104
   Tyr Arg Leu Asp Tyr Lys Phe Lys Asn Phe Tyr Asn Ala Ser Tyr Leu
           355                 360                 365
   tcc atc aag tta aat gat aaa aga gaa ctt gtt cga act gaa ggc gct   1152
   Ser Ile Lys Leu Asn Asp Lys Arg Glu Leu Val Arg Thr Glu Gly Ala
       370                 375                 380
   cct caa gtc aat ata gaa tac tcc gca aat atc aca tta aat acc gct   1200
   Pro Gln Val Asn Ile Glu Tyr Ser Ala Asn Ile Thr Leu Asn Thr Ala
   385                 390                 395                 400
   gat atc agt caa cct ttt gaa att ggc ctg aca cga gta ctt cct tcc   1248
   Asp Ile Ser Gln Pro Phe Glu Ile Gly Leu Thr Arg Val Leu Pro Ser
                   405                 410                 415
   ggt tct tgg gca tat gcc gcc gca aaa ttt acc gtt gaa gag tat aac   1296
   Gly Ser Trp Ala Tyr Ala Ala Ala Lys Phe Thr Val Glu Glu Tyr Asn
               420                 425                 430
   caa tac tct ttt ctg cta aaa ctt aac aag gct att cgt cta tca cgt   1344
   Gln Tyr Ser Phe Leu Leu Lys Leu Asn Lys Ala Ile Arg Leu Ser Arg
           435                 440                 445
   gcg aca gaa ttg tca ccc acg att ctg gaa ggc att gtg cgc agt gtt   1392
   Ala Thr Glu Leu Ser Pro Thr Ile Leu Glu Gly Ile Val Arg Ser Val
       450                 455                 460
   aat cta caa ctg gat atc aac aca gac gta tta ggt aaa gtt ttt ctg   1440
   Asn Leu Gln Leu Asp Ile Asn Thr Asp Val Leu Gly Lys Val Phe Leu
   465                 470                 475                 480
   act aaa tat tat atg cag cgt tat gct att cat gct gaa act gcc ctg   1488
   Thr Lys Tyr Tyr Met Gln Arg Tyr Ala Ile His Ala Glu Thr Ala Leu
                   485                 490                 495
   ata cta tgc aac gcg cct att tca caa cgt tca tat gat aat caa cct   1536
   Ile Leu Cys Asn Ala Pro Ile Ser Gln Arg Ser Tyr Asp Asn Gln Pro
               500                 505                 510
   agc caa ttt gat cgc ctg ttt aat acg cca tta ctg aac gga caa tat   1584
   Ser Gln Phe Asp Arg Leu Phe Asn Thr Pro Leu Leu Asn Gly Gln Tyr
           515                 520                 525
   ttt tct acc ggc gat gag gag att gat tta aat tca ggt agc acc ggc   1632
   Phe Ser Thr Gly Asp Glu Glu Ile Asp Leu Asn Ser Gly Ser Thr Gly
       530                 535                 540
   gat tgg cga aaa acc ata ctt aag cgt gca ttt aat att gat gat gtc   1680
   Asp Trp Arg Lys Thr Ile Leu Lys Arg Ala Phe Asn Ile Asp Asp Val
   545                 550                 555                 560
   tcg ctc ttc cgc ctg ctt aaa att acc gac cat gat aat aaa gat gga   1728
   Ser Leu Phe Arg Leu Leu Lys Ile Thr Asp His Asp Asn Lys Asp Gly
                   565                 570                 575
   aaa att aaa aat aac cta aag aat ctt tcc aat tta tat att gga aaa   1776
   Lys Ile Lys Asn Asn Leu Lys Asn Leu Ser Asn Leu Tyr Ile Gly Lys
               580                 585                 590
   tta ctg gca gat att cat caa tta acc att gat gaa ctg gat tta tta     1824
   Leu Leu Ala Asp Ile His Gln Leu Thr Ile Asp Glu Leu Asp Leu Leu
           595                 600                 605
   ctg att gcc gta ggt gaa gga aaa act aat tta tcc gct atc agt gat     1872
   Leu Ile Ala Val Gly Glu Gly Lys Thr Asn Leu Ser Ala Ile Ser Asp
       610                 615                 620
   aag caa ttg gct acc ctg atc aga aaa ctc aat act att acc agc tgg     1920
   Lys Gln Leu Ala Thr Leu Ile Arg Lys Leu Asn Thr Ile Thr Ser Trp
   625                 630                 635                 640
   cta cat aca cag aag tgg agt gta ttc cag cta ttt atc atg acc tcc     1968
   Leu His Thr Gln Lys Trp Ser Val Phe Gln Leu Phe Ile Met Thr Ser
                   645                 650                 655
   acc agc tat aac aaa acg cta acg cct gaa att aag aat ttg ctg gat     2016
   Thr Ser Tyr Asn Lys Thr Leu Thr Pro Glu Ile Lys Asn Leu Leu Asp
               660                 665                 670
   acc gtc tac cac ggt tta caa ggt ttt gat aaa gac aaa gca gat ttg     2064
   Thr Val Tyr His Gly Leu Gln Gly Phe Asp Lys Asp Lys Ala Asp Leu
           675                 680                 685
   cta cat gtc atg gcg ccc tat att gcg gcc acc ttg caa tta tca tcg     2112
   Leu His Val Met Ala Pro Tyr Ile Ala Ala Thr Leu Gln Leu Ser Ser
       690                 695                 700
   gaa aat gtc gcc cac tcg gta ctc ctt tgg gca gat aag tta cag ccc     2160
   Glu Asn Val Ala His Ser Val Leu Leu Trp Ala Asp Lys Leu Gln Pro
   705                 710                 715                 720
   ggc gac ggc gca atg aca gca gaa aaa ttc tgg gac tgg ttg aat act     2208
   Gly Asp Gly Ala Met Thr Ala Glu Lys Phe Trp Asp Trp Leu Asn Thr
                   725                 730                 735
   aag tat acg ccg ggt tca tcg gaa gcc gta gaa acg cag gaa cat atc     2256
   Lys Tyr Thr Pro Gly Ser Ser Glu Ala Val Glu Thr Gln Glu His Ile
               740                 745                 750
   gtt cag tat tgt cag gct ctg gca caa ttg gaa atg gtt tac cat tcc     2304
   Val Gln Tyr Cys Gln Ala Leu Ala Gln Leu Glu Met Val Tyr His Ser
           755                 760                 765
   acc ggc atc aac gaa aac gcc ttc cgt cta ttt gtg aca aaa cca gag     2352
   Thr Gly Ile Asn Glu Asn Ala Phe Arg Leu Phe Val Thr Lys Pro Glu
       770                 775                 780
   atg ttt ggc gct gca act gga gca gcg ccc gcg cat gat gcc ctt tca     2400
   Met Phe Gly Ala Ala Thr Gly Ala Ala Pro Ala His Asp Ala Leu Ser
   785                 790                 795                 800
   ctg att atg ctg aca cgt ttt gcg gat tgg gtg aac gca cta ggc gaa     2448
   Leu Ile Met Leu Thr Arg Phe Ala Asp Trp Val Asn Ala Leu Gly Glu
                   805                 810                 815
   aaa gcg tcc tcg gtg cta gcg gca ttt gaa gct aac tcg tta acg gca     2496
   Lys Ala Ser Ser Val Leu Ala Ala Phe Glu Ala Asn Ser Leu Thr Ala
               820                 825                 830
   gaa caa ctg gct gat gcc atg aat ctt gat gct aat ttg ctg ttg caa     2544
   Glu Gln Leu Ala Asp Ala Met Asn Leu Asp Ala Asn Leu Leu Leu Gln
   835 840 845
   gcc agt att caa gca caa aat cat caa cat ctt ccc cca gta act cca 2592
   Ala Ser Ile Gln Ala Gln Asn His Gln His Leu Pro Pro Val Thr Pro
   850 855 860
   gaa aat gcg ttc tcc tgt tgg aca tct atc aat act atc ctg caa tgg 2640
   Glu Asn Ala Phe Ser Cys Trp Thr Ser Ile Asn Thr Ile Leu Gln Trp
   865 870 875 880
   gtt aat gtc gca caa caa ttg aat gtc gcc cca cag ggc gtt tcc gct 2688
   Val Asn Val Ala Gln Gln Leu Asn Val Ala Pro Gln Gly Val Ser Ala
   885 890 895
   ttg gtc ggg ctg gat tat att caa tca atg aaa gag aca ccg acc tat 2736
   Leu Val Gly Leu Asp Tyr Ile Gln Ser Met Lys Glu Thr Pro Thr Tyr
   900 905 910
   gcc cag tgg gaa aac gcg gca ggc gta tta acc gcc ggg ttg aat tca 2784
   Ala Gln Trp Glu Asn Ala Ala Gly Val Leu Thr Ala Gly Leu Asn Ser
   915 920 925
   caa cag gct aat aca tta cac gct ttt ctg gat gaa tct cgc agt gcc 2832
   Gln Gln Ala Asn Thr Leu His Ala Phe Leu Asp Glu Ser Arg Ser Ala
   930 935 940
   gca tta agc acc tac tat atc cgt caa gtc gcc aag gca gcg gcg gct 2880
   Ala Leu Ser Thr Tyr Tyr Ile Arg Gln Val Ala Lys Ala Ala Ala Ala
   945 950 955 960
   att aaa agc cgt gat gac ttg tat caa tac tta ctg att gat aat cag 2928
   Ile Lys Ser Arg Asp Asp Leu Tyr Gln Tyr Leu Leu Ile Asp Asn Gln
   965 970 975
   gtt tct gcg gca ata aaa acc acc cgg atc gcc gaa gcc att gcc agt 2976
   Val Ser Ala Ala Ile Lys Thr Thr Arg Ile Ala Glu Ala Ile Ala Ser
   980 985 990
   att caa ctg tac gtc aac cgg gca ttg gaa aat gtg gaa gaa aat gcc 3024
   Ile Gln Leu Tyr Val Asn Arg Ala Leu Glu Asn Val Glu Glu Asn Ala
   995 1000 1005
   aat tcg ggg gtt atc agc cgc caa ttc ttt atc gac tgg gac aaa tac 3072
   Asn Ser Gly Val Ile Ser Arg Gln Phe Phe Ile Asp Trp Asp Lys Tyr
   1010 1015 1020
   aat aaa cgc tac agc act tgg gcg ggt gtt tct caa tta gtt tac tac 3120
   Asn Lys Arg Tyr Ser Thr Trp Ala Gly Val Ser Gln Leu Val Tyr Tyr
   1025 1030 1035 1040
   ccg gaa aac tat att gat ccg acc atg cgt atc gga caa acc aaa atg 3168
   Pro Glu Asn Tyr Ile Asp Pro Thr Met Arg Ile Gly Gln Thr Lys Met
   1045 1050 1055
   atg gac gca tta ctg caa tcc gtc agc caa agc caa tta aac gcc gat 3216
   Met Asp Ala Leu Leu Gln Ser Val Ser Gln Ser Gln Leu Asn Ala Asp
   1060 1065 1070
   acc gtc gaa gat gcc ttt atg tct tat ctg aca tcg ttt gaa caa gtg 3264
   Thr Val Glu Asp Ala Phe Met Ser Tyr Leu Thr Ser Phe Glu Gln Val
           1075                1080                1085
   gct aat ctt aaa gtt att agc gca tat cac gat aat att aat aac gat    3312
   Ala Asn Leu Lys Val Ile Ser Ala Tyr His Asp Asn Ile Asn Asn Asp
       1090                1095                1100
   caa ggg ctg acc tat ttt atc gga ctc agt gaa act gat gcc ggt gaa    3360
   Gln Gly Leu Thr Tyr Phe Ile Gly Leu Ser Glu Thr Asp Ala Gly Glu
   1105                1110                1115                1120
   tat tat tgg cgc agt gtc gat cac agt aaa ttc aac gac ggt aaa ttc    3408
   Tyr Tyr Trp Arg Ser Val Asp His Ser Lys Phe Asn Asp Gly Lys Phe
                   1125                1130                1135
   gcg gct aat gcc tgg agt gaa tgg cat aaa att gat tgt cca att aac    3456
   Ala Ala Asn Ala Trp Ser Glu Trp His Lys Ile Asp Cys Pro Ile Asn
               1140                1145                1150
   cct tat aaa agc act atc cgt cca gtg ata tat aaa tcc cgc ctg tat    3504
   Pro Tyr Lys Ser Thr Ile Arg Pro Val Ile Tyr Lys Ser Arg Leu Tyr
           1155                1160                1165
   ctg ctc tgg ttg gaa caa aag gag atc acc aaa cag aca gga aat agt    3552
   Leu Leu Trp Leu Glu Gln Lys Glu Ile Thr Lys Gln Thr Gly Asn Ser
       1170                1175                1180
   aaa gat ggc tat caa act gaa acg gat tat cgt tat gaa cta aaa ttg    3600
   Lys Asp Gly Tyr Gln Thr Glu Thr Asp Tyr Arg Tyr Glu Leu Lys Leu
   1185                1190                1195                1200
   gcg cat atc cgc tat gat ggc act tgg aat acg cca atc acc ttt gat    3648
   Ala His Ile Arg Tyr Asp Gly Thr Trp Asn Thr Pro Ile Thr Phe Asp
                   1205                1210                1215
   gtc aat aaa aaa ata tcc gag cta aaa ctg gaa aaa aat aga gcg ccc    3696
   Val Asn Lys Lys Ile Ser Glu Leu Lys Leu Glu Lys Asn Arg Ala Pro
               1220                1225                1230
   gga ctc tat tgt gcc ggt tat caa ggt gaa gat acg ttg ctg gtg atg    3744
   Gly Leu Tyr Cys Ala Gly Tyr Gln Gly Glu Asp Thr Leu Leu Val Met
           1235                1240                1245
   ttt tat aac caa caa gac aca cta gat agt tat aaa aac gct tca atg    3792
   Phe Tyr Asn Gln Gln Asp Thr Leu Asp Ser Tyr Lys Asn Ala Ser Met
       1250                1255                1260
   caa gga cta tat atc ttt gct gat atg gca tcc aaa gat atg acc cca    3840
   Gln Gly Leu Tyr Ile Phe Ala Asp Met Ala Ser Lys Asp Met Thr Pro
   1265                1270                1275                1280
   gaa cag agc aat gtt tat cgg gat aat agc tat caa caa ttt gat acc    3888
   Glu Gln Ser Asn Val Tyr Arg Asp Asn Ser Tyr Gln Gln Phe Asp Thr
                   1285                1290                1295
   aat aat gtc aga aga gtg aat aac cgc tat gca gag gat tat gag att    3936
   Asn Asn Val Arg Arg Val Asn Asn Arg Tyr Ala Glu Asp Tyr Glu Ile
               1300                1305                1310
   cct tcc tcg gta agt agc cgt aaa gac tat ggt tgg gga gat tat tac    3984
   Pro Ser Ser Val Ser Ser Arg Lys Asp Tyr Gly Trp Gly Asp Tyr Tyr
           1315                1320                1325
   ctc agc atg gta tat aac gga gat att cca act atc aat tac aaa gcc      4032
   Leu Ser Met Val Tyr Asn Gly Asp Ile Pro Thr Ile Asn Tyr Lys Ala
       1330                1335                1340
   gca tca agt gat tta aaa atc tat atc tca cca aaa tta aga att att      4080
   Ala Ser Ser Asp Leu Lys Ile Tyr Ile Ser Pro Lys Leu Arg Ile Ile
   1345                1350                1355                1360
   cat aat gga tat gaa gga cag aag cgc aat caa tgc aat ctg atg aat      4128
   His Asn Gly Tyr Glu Gly Gln Lys Arg Asn Gln Cys Asn Leu Met Asn
                   1365                1370                1375
   aaa tat ggc aaa cta ggt gat aaa ttt att gtt tat act agc ttg ggg      4176
   Lys Tyr Gly Lys Leu Gly Asp Lys Phe Ile Val Tyr Thr Ser Leu Gly
               1380                1385                1390
   gtc aat cca aat aac tcg tca aat aag ctc atg ttt tac ccc gtc tat      4224
   Val Asn Pro Asn Asn Ser Ser Asn Lys Leu Met Phe Tyr Pro Val Tyr
           1395                1400                1405
   caa tat agc gga aac acc agt gga ctc aat caa ggg aga cta cta ttc      4272
   Gln Tyr Ser Gly Asn Thr Ser Gly Leu Asn Gln Gly Arg Leu Leu Phe
       1410                1415                1420
   cac cgt gac acc act tat cca tct aaa gta gaa gct tgg att cct gga      4320
   His Arg Asp Thr Thr Tyr Pro Ser Lys Val Glu Ala Trp Ile Pro Gly
   1425                1430                1435                1440
   gca aaa cgt tct cta acc aac caa aat gcc gcc att ggt gat gat tat      4368
   Ala Lys Arg Ser Leu Thr Asn Gln Asn Ala Ala Ile Gly Asp Asp Tyr
                   1445                1450                1455
   gct aca gac tct ctg aat aaa ccg gat gat ctt aag caa tat atc ttt      4416
   Ala Thr Asp Ser Leu Asn Lys Pro Asp Asp Leu Lys Gln Tyr Ile Phe
               1460                1465                1470
   atg act gac agt aaa ggg act gct act gat gtc tca ggc cca gta gag      4464
   Met Thr Asp Ser Lys Gly Thr Ala Thr Asp Val Ser Gly Pro Val Glu
           1475                1480                1485
   att aat act gca att tct cca gca aaa gtt cag ata ata gtc aaa gcg      4512
   Ile Asn Thr Ala Ile Ser Pro Ala Lys Val Gln Ile Ile Val Lys Ala
       1490                1495                1500
   ggt ggc aag gag caa act ttt acc gca gat aaa gat gtc tcc att cag      4560
   Gly Gly Lys Glu Gln Thr Phe Thr Ala Asp Lys Asp Val Ser Ile Gln
   1505                1510                1515                1520
   cca tca cct agc ttt gat gaa atg aat tat caa ttt aat gcc ctt gaa      4608
   Pro Ser Pro Ser Phe Asp Glu Met Asn Tyr Gln Phe Asn Ala Leu Glu
                   1525                1530                1535
   ata gac ggt tct ggt ctg aat ttt att aac aac tca gcc agt att gat      4656
   Ile Asp Gly Ser Gly Leu Asn Phe Ile Asn Asn Ser Ala Ser Ile Asp
               1540                1545                1550
   gtt act ttt acc gca ttt gcg gag gat ggc cgc aaa ctg ggt tat gaa      4704
   Val Thr Phe Thr Ala Phe Ala Glu Asp Gly Arg Lys Leu Gly Tyr Glu
           1555                1560                1565
   agt ttc agt att cct gtt acc ctc aag gta agt acc gat aat gcc ctg      4752
   Ser Phe Ser Ile Pro Val Thr Leu Lys Val Ser Thr Asp Asn Ala Leu
      1570                1575                1580
   acc ctg cac cat aat gaa aat ggt gcg caa tat atg caa tgg caa tcc      4800
   Thr Leu His His Asn Glu Asn Gly Ala Gln Tyr Met Gln Trp Gln Ser
   1585                1590                1595                1600
   tat cgt acc cgc ctg aat act cta ttt gcc cgc cag ttg gtt gca cgc      4848
   Tyr Arg Thr Arg Leu Asn Thr Leu Phe Ala Arg Gln Leu Val Ala Arg
                   1605                1610                1615
   gcc acc acc gga atc gat aca att ctg agt atg gaa act cag aat att      4896
   Ala Thr Thr Gly Ile Asp Thr Ile Leu Ser Met Glu Thr Gln Asn Ile
               1620                1625                1630
   cag gaa ccg cag tta ggc aaa ggt ttc tat gct acg ttc gtg ata cct      4944
   Gln Glu Pro Gln Leu Gly Lys Gly Phe Tyr Ala Thr Phe Val Ile Pro
           1635                1640                1645
   ccc tat aac cta tca act cat ggt gat gaa cgt tgg ttt aag ctt tat      4992
   Pro Tyr Asn Leu Ser Thr His Gly Asp Glu Arg Trp Phe Lys Leu Tyr
       1650                1655                1660
   atc aaa cat gtt gtt gat aat aat tca cat att atc tat tca ggc cag      5040
   Ile Lys His Val Val Asp Asn Asn Ser His Ile Ile Tyr Ser Gly Gln
   1665                1670                1675                1680
   cta aca gat aca aat ata aac atc aca tta ttt att cct ctt gat gat      5088
   Leu Thr Asp Thr Asn Ile Asn Ile Thr Leu Phe Ile Pro Leu Asp Asp
                   1685                1690                1695
   gtc cca ttg aat caa gat tat cac gcc aag gtt tat atg acc ttc aag      5136
   Val Pro Leu Asn Gln Asp Tyr His Ala Lys Val Tyr Met Thr Phe Lys
               1700                1705                1710
   aaa tca cca tca gat ggt acc tgg tgg ggc cct cac ttt gtt aga gat      5184
   Lys Ser Pro Ser Asp Gly Thr Trp Trp Gly Pro His Phe Val Arg Asp
           1715                1720                1725
   gat aaa gga ata gta aca ata aac cct aaa tcc att ttg acc cat ttt      5232
   Asp Lys Gly Ile Val Thr Ile Asn Pro Lys Ser Ile Leu Thr His Phe
       1730                1735                1740
   gag agc gtc aat gtc ctg aat aat att agt agc gaa cca atg gat ttc      5280
   Glu Ser Val Asn Val Leu Asn Asn Ile Ser Ser Glu Pro Met Asp Phe
   1745                1750                1755                1760
   agc ggc gct aac agc ctc tat ttc tgg gaa ctg ttc tac tat acc ccg      5328
   Ser Gly Ala Asn Ser Leu Tyr Phe Trp Glu Leu Phe Tyr Tyr Thr Pro
                   1765                1770                1775
   atg ctg gtt gct caa cgt ttg ctg cat gaa cag aac ttc gat gaa gcc      5376
   Met Leu Val Ala Gln Arg Leu Leu His Glu Gln Asn Phe Asp Glu Ala
               1780                1785                1790
   aac cgt tgg ctg aaa tat gtc tgg agt cca tcc ggt tat att gtc cac      5424
   Asn Arg Trp Leu Lys Tyr Val Trp Ser Pro Ser Gly Tyr Ile Val His
           1795                1800                1805
   ggc cag att cag aac tac cag tgg aac gtc cgc ccg tta ctg gaa gac      5472
   Gly Gln Ile Gln Asn Tyr Gln Trp Asn Val Arg Pro Leu Leu Glu Asp
      1810                1815                1820
   acc agt tgg aac agt gat cct ttg gat tcc gtc gat cct gac gcg gta    5520
   Thr Ser Trp Asn Ser Asp Pro Leu Asp Ser Val Asp Pro Asp Ala Val
   1825                1830                1835                1840
   gca cag cac gat cca atg cac tac aaa gtt tca act ttt atg cgt acc    5568
   Ala Gln His Asp Pro Met His Tyr Lys Val Ser Thr Phe Met Arg Thr
                   1845                1850                1855
   ttg gat cta ttg ata gca cgc ggc gac cat gct tat cgc caa ctg gaa    5616
   Leu Asp Leu Leu Ile Ala Arg Gly Asp His Ala Tyr Arg Gln Leu Glu
               1860                1865                1870
   cga gat aca ctc aac gaa gcg aag atg tgg tat atg caa gcg ctg cat    5664
   Arg Asp Thr Leu Asn Glu Ala Lys Met Trp Tyr Met Gln Ala Leu His
           1875                1880                1885
   cta tta ggt gac aaa cct tat cta ccg ctg agt acg aca tgg agt gat    5712
   Leu Leu Gly Asp Lys Pro Tyr Leu Pro Leu Ser Thr Thr Trp Ser Asp
      1890                1895                1900
   cca cga cta gac aga gcc gcg gat atc act acc caa aat gct cac gac    5760
   Pro Arg Leu Asp Arg Ala Ala Asp Ile Thr Thr Gln Asn Ala His Asp
   1905                1910                1915                1920
   agc gca ata gtc gct ctg cgg cag aat ata cct aca ccg gca cct tta    5808
   Ser Ala Ile Val Ala Leu Arg Gln Asn Ile Pro Thr Pro Ala Pro Leu
                   1925                1930                1935
   tca ttg cgc agc gct aat acc ctg act gat ctc ttc ctg ccg caa atc    5856
   Ser Leu Arg Ser Ala Asn Thr Leu Thr Asp Leu Phe Leu Pro Gln Ile
               1940                1945                1950
   aat gaa gtg atg atg aat tac tgg cag aca tta gct cag aga gta tac    5904
   Asn Glu Val Met Met Asn Tyr Trp Gln Thr Leu Ala Gln Arg Val Tyr
           1955                1960                1965
   aat ctg cgt cat aac ctc tct atc gac ggc cag ccg tta tat ctg cca    5952
   Asn Leu Arg His Asn Leu Ser Ile Asp Gly Gln Pro Leu Tyr Leu Pro
      1970                1975                1980
   atc tat gcc aca ccg gcc gat ccg aaa gcg tta ctc agc gcc gcc gtt    6000
   Ile Tyr Ala Thr Pro Ala Asp Pro Lys Ala Leu Leu Ser Ala Ala Val
   1985                1990                1995                2000
   gcc act tct caa ggt gga ggc aag cta ccg gaa tca ttt atg tcc ctg    6048
   Ala Thr Ser Gln Gly Gly Gly Lys Leu Pro Glu Ser Phe Met Ser Leu
                   2005                2010                2015
   tgg cgt ttc ccg cac atg ctg gaa aat gcg cgc ggc atg gtt agc cag    6096
   Trp Arg Phe Pro His Met Leu Glu Asn Ala Arg Gly Met Val Ser Gln
               2020                2025                2030
   ctc acc cag ttc ggc tcc acg tta caa aat att atc gaa cgt cag gac    6144
   Leu Thr Gln Phe Gly Ser Thr Leu Gln Asn Ile Ile Glu Arg Gln Asp
           2035                2040                2045
   gcg gaa gcg ctc aat gcg tta tta caa aat cag gcc gcc gag ctg ata    6192
   Ala Glu Ala Leu Asn Ala Leu Leu Gln Asn Gln Ala Ala Glu Leu Ile
    2050                2055                2060
   ttg act aac ctg agc att cag gac aaa acc att gaa gaa ttg gat gcc    6240
   Leu Thr Asn Leu Ser Ile Gln Asp Lys Thr Ile Glu Glu Leu Asp Ala
   2065                2070                2075                2080
   gag aaa acg gtg ttg gaa aaa tcc aaa gcg gga gca caa tcg cgc ttt    6288
   Glu Lys Thr Val Leu Glu Lys Ser Lys Ala Gly Ala Gln Ser Arg Phe
                   2085                2090                2095
   gat agc tac ggc aaa ctg tac gat gag aat atc aac gcc ggt gaa aac    6336
   Asp Ser Tyr Gly Lys Leu Tyr Asp Glu Asn Ile Asn Ala Gly Glu Asn
               2100                2105                2110
   caa gcc atg acg cta cga gcg tcc gcc gcc ggg ctt acc acg gca gtt    6384
   Gln Ala Met Thr Leu Arg Ala Ser Ala Ala Gly Leu Thr Thr Ala Val
           2115                2120                2125
   cag gca tcc cgt ctg gcc ggt gcg gcg gct gat ctg gtg cct aac atc    6432
   Gln Ala Ser Arg Leu Ala Gly Ala Ala Ala Asp Leu Val Pro Asn Ile
       2130                2135                2140
   ttc ggc ttt gcc ggt ggc ggc agc cgt tgg ggg gct atc gct gag gcg    6480
   Phe Gly Phe Ala Gly Gly Gly Ser Arg Trp Gly Ala Ile Ala Glu Ala
   2145                2150                2155                2160
   aca ggt tat gtg atg gaa ttc tcc gcg aat gtt atg aac acc gaa gcg    6528
   Thr Gly Tyr Val Met Glu Phe Ser Ala Asn Val Met Asn Thr Glu Ala
                   2165                2170                2175
   gat aaa att agc caa tct gaa acc tac cgt cgt cgc cgt cag gag tgg    6576
   Asp Lys Ile Ser Gln Ser Glu Thr Tyr Arg Arg Arg Arg Gln Glu Trp
               2180                2185                2190
   gag atc cag cgg aat aat gcc gaa gcg gaa ttg aag caa atc gat gct    6624
   Glu Ile Gln Arg Asn Asn Ala Glu Ala Glu Leu Lys Gln Ile Asp Ala
           2195                2200                2205
   cag ctc aaa tca ctc gct gta cgc cgc gaa gcc gcc gta ttg cag aaa    6672
   Gln Leu Lys Ser Leu Ala Val Arg Arg Glu Ala Ala Val Leu Gln Lys
       2210                2215                2220
   acc agt ctg aaa acc caa caa gaa cag acc caa tct caa ttg gcc ttc    6720
   Thr Ser Leu Lys Thr Gln Gln Glu Gln Thr Gln Ser Gln Leu Ala Phe
   2225                2230                2235                2240
   ctg caa cgt aag ttc agc aat cag gcg tta tac aac tgg ctg cgt ggt    6768
   Leu Gln Arg Lys Phe Ser Asn Gln Ala Leu Tyr Asn Trp Leu Arg Gly
                   2245                2250                2255
   cga ctg gcg gcg att tac ttc cag ttc tac gat ttg gcc gtc gcg cgt    6816
   Arg Leu Ala Ala Ile Tyr Phe Gln Phe Tyr Asp Leu Ala Val Ala Arg
               2260                2265                2270
   tgc ctg atg gca gaa caa gct tac cgt tgg gaa ctc aat gat gac tct    6864
   Cys Leu Met Ala Glu Gln Ala Tyr Arg Trp Glu Leu Asn Asp Asp Ser
           2275                2280                2285
   gcc cgc ttc att aaa ccg ggc gcc tgg cag gga acc tat gcc ggt ctg    6912
   Ala Arg Phe Ile Lys Pro Gly Ala Trp Gln Gly Thr Tyr Ala Gly Leu
       2290                2295                2300
   ctt gca ggt gaa acc ttg atg ctg agt ctg gca caa atg gaa gac gct      6960
   Leu Ala Gly Glu Thr Leu Met Leu Ser Leu Ala Gln Met Glu Asp Ala
   2305                2310                2315                2320
   cat ctg aaa cgc gat aaa cgc gca tta gag gtt gaa cgc aca gta tcg      7008
   His Leu Lys Arg Asp Lys Arg Ala Leu Glu Val Glu Arg Thr Val Ser
                   2325                2330                2335
   ctg gcc gaa gtt tat gca gga tta cca aaa gat aac ggt cca ttt tcc      7056
   Leu Ala Glu Val Tyr Ala Gly Leu Pro Lys Asp Asn Gly Pro Phe Ser
               2340                2345                2350
   ctg gct cag gaa att gac aag ctg gtg agt caa ggt tca ggc agt gcc      7104
   Leu Ala Gln Glu Ile Asp Lys Leu Val Ser Gln Gly Ser Gly Ser Ala
           2355                2360                2365
   ggc agt ggt aat aat aat ttg gcg ttc ggc gcc ggc acg gac act aaa      7152
   Gly Ser Gly Asn Asn Asn Leu Ala Phe Gly Ala Gly Thr Asp Thr Lys
       2370                2375                2380
   acc tct ttg cag gca tca gtt tca ttc gct gat ttg aaa att cgt gaa      7200
   Thr Ser Leu Gln Ala Ser Val Ser Phe Ala Asp Leu Lys Ile Arg Glu
   2385                2390                2395                2400
   gat tac ccg gca tcg ctt ggc aaa att cga cgt atc aaa cag atc agc      7248
   Asp Tyr Pro Ala Ser Leu Gly Lys Ile Arg Arg Ile Lys Gln Ile Ser
                   2405                2410                2415
   gtc act ttg ccc gcg cta ctg gga ccg tat cag gat gta cag gca ata      7296
   Val Thr Leu Pro Ala Leu Leu Gly Pro Tyr Gln Asp Val Gln Ala Ile
               2420                2425                2430
   ttg tct tac ggc gat aaa gcc gga tta gct aac ggc tgt gaa gcg ctg      7344
   Leu Ser Tyr Gly Asp Lys Ala Gly Leu Ala Asn Gly Cys Glu Ala Leu
           2435                2440                2445
   gca gtt tct cac ggt atg aat gac agc ggc caa ttc cag ctc gat ttc      7392
   Ala Val Ser His Gly Met Asn Asp Ser Gly Gln Phe Gln Leu Asp Phe
       2450                2455                2460
   aac gat ggc aaa ttc ctg cca ttc gaa ggc atc gcc att gat caa ggc      7440
   Asn Asp Gly Lys Phe Leu Pro Phe Glu Gly Ile Ala Ile Asp Gln Gly
   2465                2470                2475                2480
   acg ctg aca ctg agc ttc cca aat gca tct atg ccg gag aaa ggt aaa      7488
   Thr Leu Thr Leu Ser Phe Pro Asn Ala Ser Met Pro Glu Lys Gly Lys
                   2485                2490                2495
   caa gcc act atg tta aaa acc ctg aac gat atc att ttg cat att cgc      7536
   Gln Ala Thr Met Leu Lys Thr Leu Asn Asp Ile Ile Leu His Ile Arg
               2500                2505                2510
   tac acc att aaa taa                                                  7551
   Tyr Thr Ile Lys
           2515
<210> 2
   <211> 7515
   <212> DNA
   <213> Photorhabdus luminescens
<220>
   <221> CDS
   <222> (1)..(7512)
<400> 2
   atg caa aac tca tta tca agc act atc gat act att tgt cag aaa ctg      48
   Met Gln Asn Ser Leu Ser Ser Thr Ile Asp Thr Ile Cys Gln Lys Leu
    1               5                  10                  15
   caa tta act tgt ccg gcg gaa att gct ttg tat ccc ttt gat act ttc      96
   Gln Leu Thr Cys Pro Ala Glu Ile Ala Leu Tyr Pro Phe Asp Thr Phe
               20                  25                  30
   cgg gaa aaa act cgg gga atg gtt aat tgg ggg gaa gca aaa cgg att     144
   Arg Glu Lys Thr Arg Gly Met Val Asn Trp Gly Glu Ala Lys Arg Ile
           35                  40                  45
   tat gaa att gca caa gcg gaa cag gat aga aac cta ctt cat gaa aaa     192
   Tyr Glu Ile Ala Gln Ala Glu Gln Asp Arg Asn Leu Leu His Glu Lys
       50                  55                  60
   cgt att ttt gcc tat gct aat ccg ctg ctg aaa aac gct gtt cgg ttg     240
   Arg Ile Phe Ala Tyr Ala Asn Pro Leu Leu Lys Asn Ala Val Arg Leu
   65                  70                  75                  80
   ggt acc cgg caa atg ttg ggt ttt ata caa ggt tat agt gat ctg ttt     288
   Gly Thr Arg Gln Met Leu Gly Phe Ile Gln Gly Tyr Ser Asp Leu Phe
                   85                  90                  95
   ggt aat cgt gct gat aac tat gcc gcg ccg ggc tcg gtt gca tcg atg     336
   Gly Asn Arg Ala Asp Asn Tyr Ala Ala Pro Gly Ser Val Ala Ser Met
               100                 105                 110
   ttc tca ccg gcg gct tat ttg acg gaa ttg tac cgt gaa gcc aaa aac     384
   Phe Ser Pro Ala Ala Tyr Leu Thr Glu Leu Tyr Arg Glu Ala Lys Asn
           115                 120                 125
   ttg cat gac agc agc tca att tat tac cta gat aaa cgt cgc ccg gat     432
   Leu His Asp Ser Ser Ser Ile Tyr Tyr Leu Asp Lys Arg Arg Pro Asp
       130                 135                 140
   tta gca agc tta atg ctc agc cag aaa aat atg gat gag gaa att tca     480
   Leu Ala Ser Leu Met Leu Ser Gln Lys Asn Met Asp Glu Glu Ile Ser
   145                 150                 155                 160
   acg ctg gct ctc tct aat gaa ttg tgc ctt gcc ggg atc gaa aca aaa     528
   Thr Leu Ala Leu Ser Asn Glu Leu Cys Leu Ala Gly Ile Glu Thr Lys
                   165                 170                 175
   aca gga aaa tca caa gat gaa gtg atg gat atg ttg tca act tat cgt     576
   Thr Gly Lys Ser Gln Asp Glu Val Met Asp Met Leu Ser Thr Tyr Arg
               180                 185                 190
   tta agt gga gag aca cct tat cat cac gct tat gaa act gtt cgt gaa     624
   Leu Ser Gly Glu Thr Pro Tyr His His Ala Tyr Glu Thr Val Arg Glu
           195                 200                 205
   atc gtt cat gaa cgt gat cca gga ttt cgt cat ttg tca cag gca ccc    672
   Ile Val His Glu Arg Asp Pro Gly Phe Arg His Leu Ser Gln Ala Pro
       210                 215                 220
   att gtt gct gct aag ctc gat cct gtg act ttg ttg ggt att agc tcc    720
   Ile Val Ala Ala Lys Leu Asp Pro Val Thr Leu Leu Gly Ile Ser Ser
   225                 230                 235                 240
   cat att tcg cca gaa ctg tat aac ttg ctg att gag gag atc ccg gaa    768
   His Ile Ser Pro Glu Leu Tyr Asn Leu Leu Ile Glu Glu Ile Pro Glu
                   245                 250                 255
   aaa gat gaa gcc gcg ctt gat acg ctt tat aaa aca aac ttt ggc gat    816
   Lys Asp Glu Ala Ala Leu Asp Thr Leu Tyr Lys Thr Asn Phe Gly Asp
               260                 265                 270
   att act act gct cag tta atg tcc cca agt tat ctg gcc cgg tat tat    864
   Ile Thr Thr Ala Gln Leu Met Ser Pro Ser Tyr Leu Ala Arg Tyr Tyr
           275                 280                 285
   ggc gtc tca ccg gaa gat att gcc tac gtg acg act tca tta tca cat    912
   Gly Val Ser Pro Glu Asp Ile Ala Tyr Val Thr Thr Ser Leu Ser His
       290                 295                 300
   gtt gga tat agc agt gat att ctg gtt att ccg ttg gtc gat ggt gtg    960
   Val Gly Tyr Ser Ser Asp Ile Leu Val Ile Pro Leu Val Asp Gly Val
   305                 310                 315                 320
   ggt aag atg gaa gta gtt cgt gtt acc cga aca cca tcg gat aat tat    1008
   Gly Lys Met Glu Val Val Arg Val Thr Arg Thr Pro Ser Asp Asn Tyr
                   325                 330                 335
   acc agt cag acg aat tat att gag ctg tat cca cag ggt ggc gac aat    1056
   Thr Ser Gln Thr Asn Tyr Ile Glu Leu Tyr Pro Gln Gly Gly Asp Asn
               340                 345                 350
   tat ttg atc aaa tac aat cta agc aat agt ttt ggt ttg gat gat ttt    1104
   Tyr Leu Ile Lys Tyr Asn Leu Ser Asn Ser Phe Gly Leu Asp Asp Phe
           355                 360                 365
   tat ctg caa tat aaa gat ggt tcc gct gat tgg act gag att gcc cat    1152
   Tyr Leu Gln Tyr Lys Asp Gly Ser Ala Asp Trp Thr Glu Ile Ala His
       370                 375                 380
   aat ccc tat cct gat atg gtc ata aat caa aag tat gaa tca cag gcg    1200
   Asn Pro Tyr Pro Asp Met Val Ile Asn Gln Lys Tyr Glu Ser Gln Ala
   385                 390                 395                 400
   aca atc aaa cgt agt gac tct gac aat ata ctc agt ata ggg tta caa    1248
   Thr Ile Lys Arg Ser Asp Ser Asp Asn Ile Leu Ser Ile Gly Leu Gln
                   405                 410                 415
   aga tgg cat agc ggt agt tat aat ttt gcc gcc gcc aat ttt aaa att    1296
   Arg Trp His Ser Gly Ser Tyr Asn Phe Ala Ala Ala Asn Phe Lys Ile
               420                 425                 430
   gac caa tac tcc ccg aaa gct ttc ctg ctt aaa atg aat aag gct att    1344
   Asp Gln Tyr Ser Pro Lys Ala Phe Leu Leu Lys Met Asn Lys Ala Ile
           435                 440                 445
   cgg ttg ctc aaa gct acc ggc ctc tct ttt gct acg ttg gag cgt att    1392
   Arg Leu Leu Lys Ala Thr Gly Leu Ser Phe Ala Thr Leu Glu Arg Ile
       450                 455                 460
   gtt gat agt gtt aat agc acc aaa tcc atc acg gtt gag gta tta aac      1440
   Val Asp Ser Val Asn Ser Thr Lys Ser Ile Thr Val Glu Val Leu Asn
   465                 470                 475                 480
   aag gtt tat cgg gta aaa ttc tat att gat cgt tat ggc atc agt gaa      1488
   Lys Val Tyr Arg Val Lys Phe Tyr Ile Asp Arg Tyr Gly Ile Ser Glu
                   485                 490                 495
   gag aca gcc gct att ttg gct aat att aat atc tct cag caa gct gtt      1536
   Glu Thr Ala Ala Ile Leu Ala Asn Ile Asn Ile Ser Gln Gln Ala Val
               500                 505                 510
   ggc aat cag ctt agc cag ttt gag caa cta ttt aat cac ccg ccg ctc      1584
   Gly Asn Gln Leu Ser Gln Phe Glu Gln Leu Phe Asn His Pro Pro Leu
           515                 520                 525
   aat ggt att cgc tat gaa atc agt gag gac aac tcc aaa cat ctt cct      1632
   Asn Gly Ile Arg Tyr Glu Ile Ser Glu Asp Asn Ser Lys His Leu Pro
       530                 535                 540
   aat cct gat ctg aac ctt aaa cca gac agt acc ggt gat gat caa cgc      1680
   Asn Pro Asp Leu Asn Leu Lys Pro Asp Ser Thr Gly Asp Asp Gln Arg
   545                 550                 555                 560
   aag gcg gtt tta aaa cgc gcg ttt cag gtt aac gcc agt gag ttg tat      1728
   Lys Ala Val Leu Lys Arg Ala Phe Gln Val Asn Ala Ser Glu Leu Tyr
                   565                 570                 575
   cag atg tta ttg atc act gat cgt aaa gaa gac ggt gtt atc aaa aat      1776
   Gln Met Leu Leu Ile Thr Asp Arg Lys Glu Asp Gly Val Ile Lys Asn
               580                 585                 590
   aac tta gag aat ttg tct gat ctg tat ttg gtt agt ttg ctg gcc cag      1824
   Asn Leu Glu Asn Leu Ser Asp Leu Tyr Leu Val Ser Leu Leu Ala Gln
           595                 600                 605
   att cat aac ctg act att gct gaa ttg aac att ttg ttg gtg att tgt      1872
   Ile His Asn Leu Thr Ile Ala Glu Leu Asn Ile Leu Leu Val Ile Cys
       610                 615                 620
   ggc tat ggc gac acc aac att tat cag att acc gac gat aat tta gcc      1920
   Gly Tyr Gly Asp Thr Asn Ile Tyr Gln Ile Thr Asp Asp Asn Leu Ala
   625                 630                 635                 640
   aaa ata gtg gaa aca ttg ttg tgg atc act caa tgg ttg aag acc caa      1968
   Lys Ile Val Glu Thr Leu Leu Trp Ile Thr Gln Trp Leu Lys Thr Gln
                   645                 650                 655
   aaa tgg aca gtt acc gac ctg ttt ctg atg acc acg gcc act tac agc      2016
   Lys Trp Thr Val Thr Asp Leu Phe Leu Met Thr Thr Ala Thr Tyr Ser
               660                 665                 670
   acc act tta acg cca gaa att agc aat ctg acg gct acg ttg tct tca      2064
   Thr Thr Leu Thr Pro Glu Ile Ser Asn Leu Thr Ala Thr Leu Ser Ser
           675                 680                 685
   act ttg cat ggc aaa gag agt ctg att ggg gaa gat ctg aaa aga gca      2112
   Thr Leu His Gly Lys Glu Ser Leu Ile Gly Glu Asp Leu Lys Arg Ala
       690                 695                 700
   atg gcg cct tgc ttc act tcg gct ttg cat ttg act tct caa gaa gtt      2160
   Met Ala Pro Cys Phe Thr Ser Ala Leu His Leu Thr Ser Gln Glu Val
   705                 710                 715                 720
   gcg tat gac ctg ctg ttg tgg ata gac cag att caa ccg gca caa ata      2208
   Ala Tyr Asp Leu Leu Leu Trp Ile Asp Gln Ile Gln Pro Ala Gln Ile
                   725                 730                 735
   act gtt gat ggg ttt tgg gaa gaa gtg caa aca aca cca acc agc ttg      2256
   Thr Val Asp Gly Phe Trp Glu Glu Val Gln Thr Thr Pro Thr Ser Leu
               740                 745                 750
   aag gtg att acc ttt gct cag gtg ctg gca caa ttg agc ctg atc tat      2304
   Lys Val Ile Thr Phe Ala Gln Val Leu Ala Gln Leu Ser Leu Ile Tyr
           755                 760                 765
   cgt cgt att ggg tta agt gaa acg gaa ctg tca ctg atc gtg act caa      2352
   Arg Arg Ile Gly Leu Ser Glu Thr Glu Leu Ser Leu Ile Val Thr Gln
       770                 775                 780
   tct tct ctg cta gtg gca ggc aaa agc ata ctg gat cac ggt ctg tta      2400
   Ser Ser Leu Leu Val Ala Gly Lys Ser Ile Leu Asp His Gly Leu Leu
   785                 790                 795                 800
   acc ctg atg gcc ttg gaa ggt ttt cat acc tgg gtt aat ggc ttg ggg      2448
   Thr Leu Met Ala Leu Glu Gly Phe His Thr Trp Val Asn Gly Leu Gly
                   805                 810                 815
   caa cat gcc tcc ttg ata ttg gcg gcg ttg aaa gac gga gcc ttg aca      2496
   Gln His Ala Ser Leu Ile Leu Ala Ala Leu Lys Asp Gly Ala Leu Thr
               820                 825                 830
   gtt acc gat gta gca caa gct atg aat aag gag gaa tct ctc cta caa      2544
   Val Thr Asp Val Ala Gln Ala Met Asn Lys Glu Glu Ser Leu Leu Gln
           835                 840                 845
   atg gca gct aat cag gtg gag aag gat cta aca aaa ctg acc agt tgg      2592
   Met Ala Ala Asn Gln Val Glu Lys Asp Leu Thr Lys Leu Thr Ser Trp
       850                 855                 860
   aca cag att gac gct att ctg caa tgg tta cag atg tct tcg gcc ttg      2640
   Thr Gln Ile Asp Ala Ile Leu Gln Trp Leu Gln Met Ser Ser Ala Leu
   865                 870                 875                 880
   gcg gtt tct cca ctg gat ctg gca ggg atg atg gcc ctg aaa tat ggg      2688
   Ala Val Ser Pro Leu Asp Leu Ala Gly Met Met Ala Leu Lys Tyr Gly
                   885                 890                 895
   ata gat cat aac tat gct gcc tgg caa gct gcg gcg gct gcg ctg atg      2736
   Ile Asp His Asn Tyr Ala Ala Trp Gln Ala Ala Ala Ala Ala Leu Met
               900                 905                 910
   gct gat cat gct aat cag gca cag aaa aaa ctg gat gag acg ttc agt      2784
   Ala Asp His Ala Asn Gln Ala Gln Lys Lys Leu Asp Glu Thr Phe Ser
           915                 920                 925
   aag gca tta tgt aac tat tat att aat gct gtt gtc gat agt gct gct      2832
   Lys Ala Leu Cys Asn Tyr Tyr Ile Asn Ala Val Val Asp Ser Ala Ala
       930                 935                 940
   gga gta cgt gat cgt aac ggt tta tat acc tat ttg ctg att gat aat      2880
   Gly Val Arg Asp Arg Asn Gly Leu Tyr Thr Tyr Leu Leu Ile Asp Asn
   945                 950                 955                 960
   cag gtt tct gcc gat gtg atc act tca cgt att gca gaa gct atc gcc      2928
   Gln Val Ser Ala Asp Val Ile Thr Ser Arg Ile Ala Glu Ala Ile Ala
                   965                 970                 975
   ggt att caa ctg tac gtt aac cgg gct tta aac cga gat gaa ggt cag      2976
   Gly Ile Gln Leu Tyr Val Asn Arg Ala Leu Asn Arg Asp Glu Gly Gln
               980                 985                 990
   ctt gca tcg gac gtt agt acc cgt cag ttc ttc act gac tgg gaa cgt      3024
   Leu Ala Ser Asp Val Ser Thr Arg Gln Phe Phe Thr Asp Trp Glu Arg
           995                 1000                1005
   tac aat aaa cgt tac agt act tgg gct ggt gtc tct gaa ctg gtc tat      3072
   Tyr Asn Lys Arg Tyr Ser Thr Trp Ala Gly Val Ser Glu Leu Val Tyr
       1010                1015                1020
   tat cca gaa aac tat gtt gat ccc act cag cgc att ggg caa acc aaa      3120
   Tyr Pro Glu Asn Tyr Val Asp Pro Thr Gln Arg Ile Gly Gln Thr Lys
   1025                1030                1035                1040
   atg atg gat gcg ctg ttg caa tcc atc aac cag agc cag cta aat gcg      3168
   Met Met Asp Ala Leu Leu Gln Ser Ile Asn Gln Ser Gln Leu Asn Ala
                   1045                1050                1055
   gat acg gtg gaa gat gct ttc aaa act tat ttg acc agc ttt gag cag      3216
   Asp Thr Val Glu Asp Ala Phe Lys Thr Tyr Leu Thr Ser Phe Glu Gln
               1060                1065                1070
   gta gca aat ctg aaa gta att agt gct tac cac gat aat gtg aat gtg      3264
   Val Ala Asn Leu Lys Val Ile Ser Ala Tyr His Asp Asn Val Asn Val
           1075                1080                1085
   gat caa gga tta act tat ttt atc ggt atc gac caa gca gct ccg ggt      3312
   Asp Gln Gly Leu Thr Tyr Phe Ile Gly Ile Asp Gln Ala Ala Pro Gly
       1090                1095                1100
   acg tat tac tgg cgt agt gtt gat cac agc aaa tgt gaa aat ggc aag      3360
   Thr Tyr Tyr Trp Arg Ser Val Asp His Ser Lys Cys Glu Asn Gly Lys
   1105                1110                1115                1120
   ttt gcc gct aat gct tgg ggt gag tgg aat aaa att acc tgt gct gtc      3408
   Phe Ala Ala Asn Ala Trp Gly Glu Trp Asn Lys Ile Thr Cys Ala Val
                   1125                1130                1135
   aat cct tgg aaa aat atc atc cgt ccg gtt gtt tat atg tcc cgc tta      3456
   Asn Pro Trp Lys Asn Ile Ile Arg Pro Val Val Tyr Met Ser Arg Leu
               1140                1145                1150
   tat ctg cta tgg ctg gag cag caa tca aag aaa agt gat gat ggt aaa      3504
   Tyr Leu Leu Trp Leu Glu Gln Gln Ser Lys Lys Ser Asp Asp Gly Lys
           1155                1160                1165
   acc acg att tat caa tat aac tta aaa ctg gct cat att cgt tac gac      3552
   Thr Thr Ile Tyr Gln Tyr Asn Leu Lys Leu Ala His Ile Arg Tyr Asp
       1170                1175                1180
   ggt agt tgg aat aca cca ttt act ttt gat gtg aca gaa aag gta aaa    3600
   Gly Ser Trp Asn Thr Pro Phe Thr Phe Asp Val Thr Glu Lys Val Lys
   1185                1190                1195                1200
   aat tac acg tcg agt act gat gct gct gaa tct tta ggg ttg tat tgt    3648
   Asn Tyr Thr Ser Ser Thr Asp Ala Ala Glu Ser Leu Gly Leu Tyr Cys
                   1205                1210                1215
   act ggt tat caa ggg gaa gac act cta tta gtt atg ttc tat tcg atg    3696
   Thr Gly Tyr Gln Gly Glu Asp Thr Leu Leu Val Met Phe Tyr Ser Met
               1220                1225                1230
   cag agt agt tat agc tcc tat acc gat aat aat gcg ccg gtc act ggg    3744
   Gln Ser Ser Tyr Ser Ser Tyr Thr Asp Asn Asn Ala Pro Val Thr Gly
           1235                1240                1245
   cta tat att ttc gct gat atg tca tca gac aat atg acg aat gca caa    3792
   Leu Tyr Ile Phe Ala Asp Met Ser Ser Asp Asn Met Thr Asn Ala Gln
       1250                1255                1260
   gca act aac tat tgg aat aac agt tat ccg caa ttt gat act gtg atg    3840
   Ala Thr Asn Tyr Trp Asn Asn Ser Tyr Pro Gln Phe Asp Thr Val Met
   1265                1270                1275                1280
   gca gat ccg gat agc gac aat aaa aaa gtc ata acc aga aga gtt aat    3888
   Ala Asp Pro Asp Ser Asp Asn Lys Lys Val Ile Thr Arg Arg Val Asn
                   1285                1290                1295
   aac cgt tat gcg gag gat tat gaa att cct tcc tct gtg aca agt aac    3936
   Asn Arg Tyr Ala Glu Asp Tyr Glu Ile Pro Ser Ser Val Thr Ser Asn
               1300                1305                1310
   agt aat tat tct tgg ggt gat cac agt tta acc atg ctt tat ggt ggt    3984
   Ser Asn Tyr Ser Trp Gly Asp His Ser Leu Thr Met Leu Tyr Gly Gly
           1315                1320                1325
   agt gtt cct aat att act ttt gaa tcg gcg gca gaa gat tta agg cta    4032
   Ser Val Pro Asn Ile Thr Phe Glu Ser Ala Ala Glu Asp Leu Arg Leu
       1330                1335                1340
   tct acc aat atg gca ttg agt att att cat aat gga tat gcg gga acc    4080
   Ser Thr Asn Met Ala Leu Ser Ile Ile His Asn Gly Tyr Ala Gly Thr
   1345                1350                1355                1360
   cgc cgt ata caa tgt aat ctt atg aaa caa tac gct tca tta ggt gat    4128
   Arg Arg Ile Gln Cys Asn Leu Met Lys Gln Tyr Ala Ser Leu Gly Asp
                   1365                1370                1375
   aaa ttt ata att tat gat tca tca ttt gat gat gca aac cgt ttt aat    4176
   Lys Phe Ile Ile Tyr Asp Ser Ser Phe Asp Asp Ala Asn Arg Phe Asn
               1380                1385                1390
   ctg gtg cca ttg ttt aaa ttc gga aaa gac gag aac tca gat gat agt    4224
   Leu Val Pro Leu Phe Lys Phe Gly Lys Asp Glu Asn Ser Asp Asp Ser
           1395                1400                1405
   att tgt ata tat aat gaa aac cct tcc tct gaa gat aag aag tgg tat    4272
   Ile Cys Ile Tyr Asn Glu Asn Pro Ser Ser Glu Asp Lys Lys Trp Tyr
       1410                1415                1420
   ttt tct tcg aaa gat gac aat aaa aca gcg gat tat aat ggt gga act    4320
   Phe Ser Ser Lys Asp Asp Asn Lys Thr Ala Asp Tyr Asn Gly Gly Thr
   1425                1430                1435                1440
   caa tgt ata gat gct gga acc agt aac aaa gat ttt tat tat aat ctc    4368
   Gln Cys Ile Asp Ala Gly Thr Ser Asn Lys Asp Phe Tyr Tyr Asn Leu
                   1445                1450                1455
   cag gag att gaa gta att agt gtt act ggt ggg tat tgg tcg agt tat    4416
   Gln Glu Ile Glu Val Ile Ser Val Thr Gly Gly Tyr Trp Ser Ser Tyr
               1460                1465                1470
   aaa ata tcc aac ccg att aat atc aat acg ggc att gat agt gct aaa    4464
   Lys Ile Ser Asn Pro Ile Asn Ile Asn Thr Gly Ile Asp Ser Ala Lys
           1475                1480                1485
   gta aaa gtc acc gta aaa gcg ggt ggt gac gat caa atc ttt act gct    4512
   Val Lys Val Thr Val Lys Ala Gly Gly Asp Asp Gln Ile Phe Thr Ala
       1490                1495                1500
   gat aat agt acc tat gtt cct cag caa ccg gca ccc agt ttt gag gag    4560
   Asp Asn Ser Thr Tyr Val Pro Gln Gln Pro Ala Pro Ser Phe Glu Glu
   1505                1510                1515                1520
   atg att tat cag ttc aat aac ctg aca ata gat tgt aag aat tta aat    4608
   Met Ile Tyr Gln Phe Asn Asn Leu Thr Ile Asp Cys Lys Asn Leu Asn
                   1525                1530                1535
   ttc atc gac aat cag gca cat att gag att gat ttc acc gct acg gca    4656
   Phe Ile Asp Asn Gln Ala His Ile Glu Ile Asp Phe Thr Ala Thr Ala
               1540                1545                1550
   caa gat ggc cga ttc ttg ggt gca gaa act ttt att atc ccg gta act    4704
   Gln Asp Gly Arg Phe Leu Gly Ala Glu Thr Phe Ile Ile Pro Val Thr
           1555                1560                1565
   aaa aaa gtt ctc ggt act gag aac gtg att gcg tta tat agc gaa aat    4752
   Lys Lys Val Leu Gly Thr Glu Asn Val Ile Ala Leu Tyr Ser Glu Asn
       1570                1575                1580
   aac ggt gtt caa tat atg caa att ggc gca tat cgt acc cgt ttg aat    4800
   Asn Gly Val Gln Tyr Met Gln Ile Gly Ala Tyr Arg Thr Arg Leu Asn
   1585                1590                1595                1600
   acg tta ttc gct caa cag ttg gtt agc cgt gct aat cgt ggc att gat    4848
   Thr Leu Phe Ala Gln Gln Leu Val Ser Arg Ala Asn Arg Gly Ile Asp
                   1605                1610                1615
   gca gtg ctc agt atg gaa act cag aat att cag gaa ccg caa tta gga    4896
   Ala Val Leu Ser Met Glu Thr Gln Asn Ile Gln Glu Pro Gln Leu Gly
               1620                1625                1630
   gcg ggc aca tat gtg cag ctt gtg ttg gat aaa tat gat gag tct att    4944
   Ala Gly Thr Tyr Val Gln Leu Val Leu Asp Lys Tyr Asp Glu Ser Ile
           1635                1640                1645
   cat ggc act aat aaa agc ttt gct att gaa tat gtt gat ata ttt aaa    4992
   His Gly Thr Asn Lys Ser Phe Ala Ile Glu Tyr Val Asp Ile Phe Lys
       1650                1655                1660
   gag aac gat agt ttt gtg att tat caa gga gaa ctt agc gaa aca agt    5040
   Glu Asn Asp Ser Phe Val Ile Tyr Gln Gly Glu Leu Ser Glu Thr Ser
   1665                1670                1675                1680
   caa act gtt gtg aaa gtt ttc tta tcc tat ttt ata gag gcg act gga    5088
   Gln Thr Val Val Lys Val Phe Leu Ser Tyr Phe Ile Glu Ala Thr Gly
                   1685                1690                1695
   aat aag aac cac tta tgg gta cgt gct aaa tac caa aag gaa acg act    5136
   Asn Lys Asn His Leu Trp Val Arg Ala Lys Tyr Gln Lys Glu Thr Thr
               1700                1705                1710
   gat aag atc ttg ttc gac cgt act gat gag aaa gat ccg cac ggt tgg    5184
   Asp Lys Ile Leu Phe Asp Arg Thr Asp Glu Lys Asp Pro His Gly Trp
           1715                1720                1725
   ttt ctc agc gac gat cac aag acc ttt agt ggt ctc tct tcc gca cag    5232
   Phe Leu Ser Asp Asp His Lys Thr Phe Ser Gly Leu Ser Ser Ala Gln
       1730                1735                1740
   gca tta aag aac gac agt gaa ccg atg gat ttc tct ggc gcc aat gct    5280
   Ala Leu Lys Asn Asp Ser Glu Pro Met Asp Phe Ser Gly Ala Asn Ala
   1745                1750                1755                1760
   ctc tat ttc tgg gaa ctg ttc tat tac acg ccg atg atg atg gct cat    5328
   Leu Tyr Phe Trp Glu Leu Phe Tyr Tyr Thr Pro Met Met Met Ala His
                   1765                1770                1775
   cgt ttg ttg cag gaa cag aat ttt gat gcg gcg aac cat tgg ttc cgt    5376
   Arg Leu Leu Gln Glu Gln Asn Phe Asp Ala Ala Asn His Trp Phe Arg
               1780                1785                1790
   tat gtc tgg agt cca tcc ggt tat atc gtt gat ggt aaa att gct atc    5424
   Tyr Val Trp Ser Pro Ser Gly Tyr Ile Val Asp Gly Lys Ile Ala Ile
           1795                1800                1805
   tac cac tgg aac gtg cga ccg ctg gaa gaa gac acc agt tgg aat gca    5472
   Tyr His Trp Asn Val Arg Pro Leu Glu Glu Asp Thr Ser Trp Asn Ala
       1810                1815                1820
   caa caa ctg gac tcc acc gat cca gat gct gta gcc caa gat gat ccg    5520
   Gln Gln Leu Asp Ser Thr Asp Pro Asp Ala Val Ala Gln Asp Asp Pro
   1825                1830                1835                1840
   atg cac tac aag gtg gct acc ttt atg gcg acg ttg gat ctg cta atg    5568
   Met His Tyr Lys Val Ala Thr Phe Met Ala Thr Leu Asp Leu Leu Met
                   1845                1850                1855
   gcc cgt ggt gat gct gct tac cgc cag tta gag cgt gat acg ttg gct    5616
   Ala Arg Gly Asp Ala Ala Tyr Arg Gln Leu Glu Arg Asp Thr Leu Ala
               1860                1865                1870
   gaa gct aaa atg tgg tat aca cag gcg ctt aat ctg ttg ggt gat gag    5664
   Glu Ala Lys Met Trp Tyr Thr Gln Ala Leu Asn Leu Leu Gly Asp Glu
           1875                1880                1885
   cca caa gtg atg ctg agt acg act tgg gct aat cca aca ttg ggt aat    5712
   Pro Gln Val Met Leu Ser Thr Thr Trp Ala Asn Pro Thr Leu Gly Asn
       1890                1895                1900
   gct gct tca aaa acc aca cag cag gtt cgt cag caa gtg ctt acc cag    5760
   Ala Ala Ser Lys Thr Thr Gln Gln Val Arg Gln Gln Val Leu Thr Gln
   1905                1910                1915                1920
   ttg cgt ctc aat agc agg gta aaa acc ccg ttg cta gga aca gcc aat   5808
   Leu Arg Leu Asn Ser Arg Val Lys Thr Pro Leu Leu Gly Thr Ala Asn
                   1925                1930                1935
   tcc ctg acc gct tta ttc ctg ccg cag gaa aat agc aag ctc aaa ggc   5856
   Ser Leu Thr Ala Leu Phe Leu Pro Gln Glu Asn Ser Lys Leu Lys Gly
               1940                1945                1950
   tac tgg cgg aca ctg gcg cag cgt atg ttt aat tta cgt cat aat ctg   5904
   Tyr Trp Arg Thr Leu Ala Gln Arg Met Phe Asn Leu Arg His Asn Leu
           1955                1960                1965
   tcg att gac ggc cag ccg ctc tcc ttg ccg ctg tat gct aaa ccg gct   5952
   Ser Ile Asp Gly Gln Pro Leu Ser Leu Pro Leu Tyr Ala Lys Pro Ala
       1970                1975                1980
   gat cca aaa gct tta ctg agt gcg gcg gtt tca gct tct caa ggg gga   6000
   Asp Pro Lys Ala Leu Leu Ser Ala Ala Val Ser Ala Ser Gln Gly Gly
   1985                1990                1995                2000
   gcc gac ttg ccg aag gcg ccg ctg act att cac cgc ttc cct caa atg   6048
   Ala Asp Leu Pro Lys Ala Pro Leu Thr Ile His Arg Phe Pro Gln Met
                   2005                2010                2015
   cta gaa ggg gca cgg ggc ttg gtt aac cag ctt ata cag ttc ggt agt   6096
   Leu Glu Gly Ala Arg Gly Leu Val Asn Gln Leu Ile Gln Phe Gly Ser
               2020                2025                2030
   tca cta ttg ggg tac agt gag cgt cag gat gcg gaa gct atg agt caa   6144
   Ser Leu Leu Gly Tyr Ser Glu Arg Gln Asp Ala Glu Ala Met Ser Gln
           2035                2040                2045
   cta ctg caa acc caa gcc agc gag tta ata ctg acc agt att cgt atg   6192
   Leu Leu Gln Thr Gln Ala Ser Glu Leu Ile Leu Thr Ser Ile Arg Met
       2050                2055                2060
   cag gat aac caa ttg gca gag ctg gat tcg gaa aaa acc gcc ttg caa   6240
   Gln Asp Asn Gln Leu Ala Glu Leu Asp Ser Glu Lys Thr Ala Leu Gln
   2065                2070                2075                2080
   gtc tct tta gct gga gtg caa caa cgg ttt gac agc tat agc caa ctg   6288
   Val Ser Leu Ala Gly Val Gln Gln Arg Phe Asp Ser Tyr Ser Gln Leu
                   2085                2090                2095
   tat gag gag aac atc aac gca ggt gag cag cga gcg ctg gcg tta cgc   6336
   Tyr Glu Glu Asn Ile Asn Ala Gly Glu Gln Arg Ala Leu Ala Leu Arg
               2100                2105                2110
   tca gaa tct gct att gag tct cag gga gcg cag att tcc cgt atg gca   6384
   Ser Glu Ser Ala Ile Glu Ser Gln Gly Ala Gln Ile Ser Arg Met Ala
           2115                2120                2125
   ggc gcg ggt gtt gat atg gca cca aat atc ttc ggc ctg gct gat ggc   6432
   Gly Ala Gly Val Asp Met Ala Pro Asn Ile Phe Gly Leu Ala Asp Gly
       2130                2135                2140
   ggc atg cat tat ggt gct att gcc tat gcc atc gct gac ggt att gag   6480
   Gly Met His Tyr Gly Ala Ile Ala Tyr Ala Ile Ala Asp Gly Ile Glu
   2145                2150                2155                2160
   ttg agt gct tct gcc aag atg gtt gat gcg gag aaa gtt gct cag tcg    6528
   Leu Ser Ala Ser Ala Lys Met Val Asp Ala Glu Lys Val Ala Gln Ser
                   2165                2170                2175
   gaa ata tat cgc cgt cgc cgt caa gaa tgg aaa att cag cgt gac aac    6576
   Glu Ile Tyr Arg Arg Arg Arg Gln Glu Trp Lys Ile Gln Arg Asp Asn
               2180                2185                2190
   gca caa gcg gag att aac cag tta aac gcg caa ctg gaa tca ctg tct    6624
   Ala Gln Ala Glu Ile Asn Gln Leu Asn Ala Gln Leu Glu Ser Leu Ser
           2195                2200                2205
   att cgc cgt gaa gcc gct gaa atg caa aaa gag tac ctg aaa acc cag    6672
   Ile Arg Arg Glu Ala Ala Glu Met Gln Lys Glu Tyr Leu Lys Thr Gln
       2210                2215                2220
   caa gct cag gcg cag gca caa ctt act ttc tta aga agc aaa ttc agt    6720
   Gln Ala Gln Ala Gln Ala Gln Leu Thr Phe Leu Arg Ser Lys Phe Ser
   2225                2230                2235                2240
   aat caa gcg tta tat agt tgg tta cga ggg cgt ttg tca ggt att tat    6768
   Asn Gln Ala Leu Tyr Ser Trp Leu Arg Gly Arg Leu Ser Gly Ile Tyr
                   2245                2250                2255
   ttc cag ttc tat gac ttg gcc gta tca cgt tgc ctg atg gca gag caa    6816
   Phe Gln Phe Tyr Asp Leu Ala Val Ser Arg Cys Leu Met Ala Glu Gln
               2260                2265                2270
   tcc tat caa tgg gaa gct aat gat aat tcc att agc ttt gtc aaa ccg    6864
   Ser Tyr Gln Trp Glu Ala Asn Asp Asn Ser Ile Ser Phe Val Lys Pro
           2275                2280                2285
   ggt gca tgg caa gga act tac gcc ggc tta ttg tgt gga gaa gct ttg    6912
   Gly Ala Trp Gln Gly Thr Tyr Ala Gly Leu Leu Cys Gly Glu Ala Leu
       2290                2295                2300
   ata caa aat ctg gca caa atg gaa gag gca tat ctg aaa tgg gaa tct    6960
   Ile Gln Asn Leu Ala Gln Met Glu Glu Ala Tyr Leu Lys Trp Glu Ser
   2305                2310                2315                2320
   cgc gct ttg gaa gta gaa cgc acg gtt tca ttg gca gtg gtt tat gat    7008
   Arg Ala Leu Glu Val Glu Arg Thr Val Ser Leu Ala Val Val Tyr Asp
                   2325                2330                2335
   tca ctg gaa ggt aat gat cgt ttt aat tta gcg gaa caa ata cct gca    7056
   Ser Leu Glu Gly Asn Asp Arg Phe Asn Leu Ala Glu Gln Ile Pro Ala
               2340                2345                2350
   tta ttg gat aag ggg gag gga aca gca gga act aaa gaa aat ggg tta    7104
   Leu Leu Asp Lys Gly Glu Gly Thr Ala Gly Thr Lys Glu Asn Gly Leu
           2355                2360                2365
   tca ttg gct aat gct atc ctg tca gct tcg gtc aaa ttg tcc gac ttg    7152
   Ser Leu Ala Asn Ala Ile Leu Ser Ala Ser Val Lys Leu Ser Asp Leu
       2370                2375                2380
   aaa ctg gga acg gat tat cca gac agt atc gtt ggt agc aac aag gtt    7200
   Lys Leu Gly Thr Asp Tyr Pro Asp Ser Ile Val Gly Ser Asn Lys Val
   2385                2390                2395                2400
   cgt cgt att aag caa atc agt gtt tcg cta cct gca ttg gtt ggg cct    7248
   Arg Arg Ile Lys Gln Ile Ser Val Ser Leu Pro Ala Leu Val Gly Pro
                   2405                2410                2415
   tat cag gat gtt cag gct atg ctc agc tat ggt ggc agt act caa ttg     7296
   Tyr Gln Asp Val Gln Ala Met Leu Ser Tyr Gly Gly Ser Thr Gln Leu
               2420                2425                2430
   ccg aaa ggt tgt tca gcg ttg gct gtg tct cat ggt acc aat gat agt     7344
   Pro Lys Gly Cys Ser Ala Leu Ala Val Ser His Gly Thr Asn Asp Ser
           2435                2440                2445
   ggt cag ttc cag ttg gat ttc aat gac ggc aaa tac ctg cca ttt gaa     7392
   Gly Gln Phe Gln Leu Asp Phe Asn Asp Gly Lys Tyr Leu Pro Phe Glu
       2450                2455                2460
   ggt att gct ctt gat gat cag ggt aca ctg aat ctt caa ttt ccg aat     7440
   Gly Ile Ala Leu Asp Asp Gln Gly Thr Leu Asn Leu Gln Phe Pro Asn
   2465                2470                2475                2480
   gct acc gac aag cag aaa gca ata ttg caa act atg agc gat att att     7488
   Ala Thr Asp Lys Gln Lys Ala Ile Leu Gln Thr Met Ser Asp Ile Ile
                   2485                2490                2495
   ttg cat att cgt tat acc atc cgt taa                                 7515
   Leu His Ile Arg Tyr Thr Ile Arg
               2500
<210> 3
   <211> 7577
   <212> DNA
   <213> Artificial Sequence
<220>
   <221> CDS
   <222> (3)..(7553)
<220>
   <223> Description of Artificial Sequence:hemicot tcdA
<400> 3
   cc atg gct aac gag tcc gtc aag gag atc cca gac gtc ctc aag tcc     47
      Met Ala Asn Glu Ser Val Lys Glu Ile Pro Asp Val Leu Lys Ser
        1               5                  10                  15
   caa tgc ggt ttc aac tgc ctc act gac atc tcc cac agc tcc ttc aac    95
   Gln Cys Gly Phe Asn Cys Leu Thr Asp Ile Ser His Ser Ser Phe Asn
                    20                  25                  30
   gag ttc aga caa caa gtc tct gag cac ctc tcc tgg tcc gag acc cat    143
   Glu Phe Arg Gln Gln Val Ser Glu His Leu Ser Trp Ser Glu Thr His
                35                  40                  45
   gac ctc tac cat gac gct cag caa gct cag aag gac aac agg ctc tac    191
   Asp Leu Tyr His Asp Ala Gln Gln Ala Gln Lys Asp Asn Arg Leu Tyr
            50                  55                  60
   gag gct agg atc ctc aag agg gct aac cca caa ctc cag aac gct gtc    239
   Glu Ala Arg Ile Leu Lys Arg Ala Asn Pro Gln Leu Gln Asn Ala Val
        65                  70                  75
   cac ctc gcc atc ttg gct cca aac gct gag ttg att ggt tac aac aac     287
   His Leu Ala Ile Leu Ala Pro Asn Ala Glu Leu Ile Gly Tyr Asn Asn
    80                 85                  90                  95
   cag ttc tct ggc aga gct agc cag tac gtg gct cct ggt aca gtc tcc     335
   Gln Phe Ser Gly Arg Ala Ser Gln Tyr Val Ala Pro Gly Thr Val Ser
                   100                 105                 110
   tcc atg ttc agc cca gcc gct tac ctc act gag ttg tac cgc gag gct     383
   Ser Met Phe Ser Pro Ala Ala Tyr Leu Thr Glu Leu Tyr Arg Glu Ala
               115                 120                 125
   agg aac ctt cat gct tct gac tcc gtc tac tac ttg gac aca cgc aga     431
   Arg Asn Leu His Ala Ser Asp Ser Val Tyr Tyr Leu Asp Thr Arg Arg
           130                 135                 140
   cca gac ctc aag agc atg gcc ctc agc caa cag aac atg gac att gag     479
   Pro Asp Leu Lys Ser Met Ala Leu Ser Gln Gln Asn Met Asp Ile Glu
       145                 150                 155
   ttg tcc acc ctc tcc ttg agc aac gag ctt ctc ttg gag tcc atc aag     527
   Leu Ser Thr Leu Ser Leu Ser Asn Glu Leu Leu Leu Glu Ser Ile Lys
   160                 165                 170                 175
   act gag agc aag ttg gag aac tac acc aag gtc atg gag atg ctc tcc     575
   Thr Glu Ser Lys Leu Glu Asn Tyr Thr Lys Val Met Glu Met Leu Ser
                   180                 185                 190
   acc ttc aga cca agc ggt gca act cca tac cat gat gcc tac gag aac     623
   Thr Phe Arg Pro Ser Gly Ala Thr Pro Tyr His Asp Ala Tyr Glu Asn
               195                 200                 205
   gtc agg gag gtc atc caa ctt caa gac cct ggt ctt gag caa ctc aac     671
   Val Arg Glu Val Ile Gln Leu Gln Asp Pro Gly Leu Glu Gln Leu Asn
           210                 215                 220
   gct tct cca gcc att gct ggt ttg atg cac cag gca tcc ttg ctc ggt     719
   Ala Ser Pro Ala Ile Ala Gly Leu Met His Gln Ala Ser Leu Leu Gly
       225                 230                 235
   atc aac gcc tcc atc tct cct gag ttg ttc aac atc ttg act gag gag     767
   Ile Asn Ala Ser Ile Ser Pro Glu Leu Phe Asn Ile Leu Thr Glu Glu
   240                 245                 250                 255
   atc act gag ggc aac gct gag gag ttg tac aag aag aac ttc ggc aac     815
   Ile Thr Glu Gly Asn Ala Glu Glu Leu Tyr Lys Lys Asn Phe Gly Asn
                   260                 265                 270
   att gag cca gcc tct ctt gca atg cct gag tac ctc aag agg tac tac     863
   Ile Glu Pro Ala Ser Leu Ala Met Pro Glu Tyr Leu Lys Arg Tyr Tyr
               275                 280                 285
   aac ttg tct gat gag gag ctt tct caa ttc att ggc aag gct tcc aac     911
   Asn Leu Ser Asp Glu Glu Leu Ser Gln Phe Ile Gly Lys Ala Ser Asn
           290                 295                 300
   ttc ggt caa cag gag tac agc aac aac cag ctc atc act cca gtt gtg     959
   Phe Gly Gln Gln Glu Tyr Ser Asn Asn Gln Leu Ile Thr Pro Val Val
       305                 310                 315
   aac tcc tct gat ggc act gtg aag gtc tac cgc atc aca cgt gag tac      1007
   Asn Ser Ser Asp Gly Thr Val Lys Val Tyr Arg Ile Thr Arg Glu Tyr
   320                 325                 330                 335
   acc aca aac gcc tac caa atg gat gtt gag ttg ttc cca ttc ggt ggt      1055
   Thr Thr Asn Ala Tyr Gln Met Asp Val Glu Leu Phe Pro Phe Gly Gly
                   340                 345                 350
   gag aac tac aga ctt gac tac aag ttc aag aac ttc tac aac gcc tcc      1103
   Glu Asn Tyr Arg Leu Asp Tyr Lys Phe Lys Asn Phe Tyr Asn Ala Ser
               355                 360                 365
   tac ctc tcc atc aag ttg aac gac aag agg gag ctt gtc agg act gag      1151
   Tyr Leu Ser Ile Lys Leu Asn Asp Lys Arg Glu Leu Val Arg Thr Glu
           370                 375                 380
   ggt gct cct caa gtg aac att gag tac tct gcc aac atc acc ctc aac      1199
   Gly Ala Pro Gln Val Asn Ile Glu Tyr Ser Ala Asn Ile Thr Leu Asn
       385                 390                 395
   aca gct gac atc tct caa cca ttc gag att ggt ttg acc aga gtc ctt      1247
   Thr Ala Asp Ile Ser Gln Pro Phe Glu Ile Gly Leu Thr Arg Val Leu
   400                 405                 410                 415
   ccc tct ggc tcc tgg gcc tac gct gca gcc aag ttc act gtt gag gag      1295
   Pro Ser Gly Ser Trp Ala Tyr Ala Ala Ala Lys Phe Thr Val Glu Glu
                   420                 425                 430
   tac aac cag tac tct ttc ctc ttg aag ctc aac aag gca att cgt ctc      1343
   Tyr Asn Gln Tyr Ser Phe Leu Leu Lys Leu Asn Lys Ala Ile Arg Leu
               435                 440                 445
   agc aga gcc act gag ttg tct ccc acc atc ttg gag ggc att gtg agg      1391
   Ser Arg Ala Thr Glu Leu Ser Pro Thr Ile Leu Glu Gly Ile Val Arg
           450                 455                 460
   tct gtc aac ctt caa ctt gac atc aac act gat gtg ctt ggc aag gtc      1439
   Ser Val Asn Leu Gln Leu Asp Ile Asn Thr Asp Val Leu Gly Lys Val
       465                 470                 475
   ttc ctc acc aag tac tac atg caa cgc tac gcc atc cat gct gag act      1487
   Phe Leu Thr Lys Tyr Tyr Met Gln Arg Tyr Ala Ile His Ala Glu Thr
   480                 485                 490                 495
   gca ctc atc ctc tgc aac gca ccc atc tct caa cgc tcc tac gac aac      1535
   Ala Leu Ile Leu Cys Asn Ala Pro Ile Ser Gln Arg Ser Tyr Asp Asn
                   500                 505                 510
   cag cct tcc cag ttc gac agg ctc ttc aac act cct ctc ttg aac ggc      1583
   Gln Pro Ser Gln Phe Asp Arg Leu Phe Asn Thr Pro Leu Leu Asn Gly
               515                 520                 525
   cag tac ttc tcc act ggt gat gag gag att gac ctc aac tct ggc tcc      1631
   Gln Tyr Phe Ser Thr Gly Asp Glu Glu Ile Asp Leu Asn Ser Gly Ser
           530                 535                 540
   aca ggt gac tgg aga aag acc atc ttg aag agg gcc ttc aac att gat      1679
   Thr Gly Asp Trp Arg Lys Thr Ile Leu Lys Arg Ala Phe Asn Ile Asp
       545                 550                 555
   gat gtc tct ctc ttc cgt ctc ttg aag atc aca gat cac gac aac aag      1727
   Asp Val Ser Leu Phe Arg Leu Leu Lys Ile Thr Asp His Asp Asn Lys
   560                 565                 570                 575
   gat ggc aag atc aag aac aac ttg aag aac ctt tcc aac ctc tac att      1775
   Asp Gly Lys Ile Lys Asn Asn Leu Lys Asn Leu Ser Asn Leu Tyr Ile
                   580                 585                 590
   ggc aag ttg ctt gca gac atc cac caa ctc acc att gat gag ttg gac      1823
   Gly Lys Leu Leu Ala Asp Ile His Gln Leu Thr Ile Asp Glu Leu Asp
               595                 600                 605
   ctc ttg ctc att gca gtc ggt gag ggc aag acc aac ctc tct gca atc      1871
   Leu Leu Leu Ile Ala Val Gly Glu Gly Lys Thr Asn Leu Ser Ala Ile
           610                 615                 620
   tct gac aag cag ttg gca acc ctc atc agg aag ttg aac acc atc acc      1919
   Ser Asp Lys Gln Leu Ala Thr Leu Ile Arg Lys Leu Asn Thr Ile Thr
       625                 630                 635
   tcc tgg ctt cac acc cag aag tgg tct gtc ttc caa ctc ttc atc atg      1967
   Ser Trp Leu His Thr Gln Lys Trp Ser Val Phe Gln Leu Phe Ile Met
   640                 645                 650                 655
   acc agc acc tcc tac aac aag acc ctc act cct gag atc aag aac ctc      2015
   Thr Ser Thr Ser Tyr Asn Lys Thr Leu Thr Pro Glu Ile Lys Asn Leu
                   660                 665                 670
   ttg gac aca gtc tac cac ggt ctc caa ggc ttc gac aag gac aag gct      2063
   Leu Asp Thr Val Tyr His Gly Leu Gln Gly Phe Asp Lys Asp Lys Ala
               675                 680                 685
   gac ttg ctt cat gtc atg gct ccc tac att gca gcc acc ctc caa ctc      2111
   Asp Leu Leu His Val Met Ala Pro Tyr Ile Ala Ala Thr Leu Gln Leu
           690                 695                 700
   tcc tct gag aac gtg gct cac tct gtc ttg ctc tgg gct gac aag ctc      2159
   Ser Ser Glu Asn Val Ala His Ser Val Leu Leu Trp Ala Asp Lys Leu
       705                 710                 715
   caa cct ggt gat ggt gcc atg act gct gag aag ttc tgg gac tgg ctc      2207
   Gln Pro Gly Asp Gly Ala Met Thr Ala Glu Lys Phe Trp Asp Trp Leu
   720                 725                 730                 735
   aac acc aag tac aca cca ggc tcc tct gag gct gtt gag act caa gag      2255
   Asn Thr Lys Tyr Thr Pro Gly Ser Ser Glu Ala Val Glu Thr Gln Glu
                   740                 745                 750
   cac att gtg caa tac tgc cag gct ctt gca cag ttg gag atg gtc tac      2303
   His Ile Val Gln Tyr Cys Gln Ala Leu Ala Gln Leu Glu Met Val Tyr
               755                 760                 765
   cac tcc act ggc atc aac gag aac gct ttc aga ctc ttc gtc acc aag      2351
   His Ser Thr Gly Ile Asn Glu Asn Ala Phe Arg Leu Phe Val Thr Lys
           770                 775                 780
   cct gag atg ttc ggt gct gcc aca ggt gct gca cct gct cat gat gct      2399
   Pro Glu Met Phe Gly Ala Ala Thr Gly Ala Ala Pro Ala His Asp Ala
       785                 790                 795
   ctc tcc ctc atc atg ttg acc agg ttc gct gac tgg gtc aac gct ctt      2447
   Leu Ser Leu Ile Met Leu Thr Arg Phe Ala Asp Trp Val Asn Ala Leu
   800                 805                 810                 815
   ggt gag aag gct tcc tct gtc ttg gct gcc ttc gag gcc aac tcc ctc     2495
   Gly Glu Lys Ala Ser Ser Val Leu Ala Ala Phe Glu Ala Asn Ser Leu
                   820                 825                 830
   act gct gag caa ctt gct gat gcc atg aac ctt gat gcc aac ctc ttg     2543
   Thr Ala Glu Gln Leu Ala Asp Ala Met Asn Leu Asp Ala Asn Leu Leu
               835                 840                 845
   ctc caa gct tcc att caa gct cag aac cac caa cac ctc cca cct gtc     2591
   Leu Gln Ala Ser Ile Gln Ala Gln Asn His Gln His Leu Pro Pro Val
           850                 855                 860
   act cca gag aac gct ttc tcc tgc tgg acc tcc atc aac acc atc ctc     2639
   Thr Pro Glu Asn Ala Phe Ser Cys Trp Thr Ser Ile Asn Thr Ile Leu
       865                 870                 875
   caa tgg gtc aac gtg gct cag caa ctc aac gtg gct cca caa ggt gtc     2687
   Gln Trp Val Asn Val Ala Gln Gln Leu Asn Val Ala Pro Gln Gly Val
   880                 885                 890                 895
   tct gct ttg gtc ggt ctt gac tac atc cag tcc atg aag gag aca cca     2735
   Ser Ala Leu Val Gly Leu Asp Tyr Ile Gln Ser Met Lys Glu Thr Pro
                   900                 905                 910
   acc tac gct caa tgg gag aac gca gct ggt gtc ttg act gct ggt ctc     2783
   Thr Tyr Ala Gln Trp Glu Asn Ala Ala Gly Val Leu Thr Ala Gly Leu
               915                 920                 925
   aac tcc caa cag gcc aac acc ctc cat gct ttc ttg gat gag tct cgc     2831
   Asn Ser Gln Gln Ala Asn Thr Leu His Ala Phe Leu Asp Glu Ser Arg
           930                 935                 940
   tct gct gcc ctc tcc acc tac tac atc agg caa gtc gcc aag gca gct     2879
   Ser Ala Ala Leu Ser Thr Tyr Tyr Ile Arg Gln Val Ala Lys Ala Ala
       945                 950                 955
   gct gcc atc aag tct cgc gat gac ctc tac caa tac ctc ctc att gac     2927
   Ala Ala Ile Lys Ser Arg Asp Asp Leu Tyr Gln Tyr Leu Leu Ile Asp
   960                 965                 970                 975
   aac cag gtc tct gct gcc atc aag acc acc agg atc gct gag gcc atc     2975
   Asn Gln Val Ser Ala Ala Ile Lys Thr Thr Arg Ile Ala Glu Ala Ile
                   980                 985                 990
   gct tcc atc caa ctc tac gtc aac cgc gct ctt gag aac gtt gag gag     3023
   Ala Ser Ile Gln Leu Tyr Val Asn Arg Ala Leu Glu Asn Val Glu Glu
               995                 1000                1005
   aac gcc aac tct ggt gtc atc tct cgc caa ttc ttc atc gac tgg gac     3071
   Asn Ala Asn Ser Gly Val Ile Ser Arg Gln Phe Phe Ile Asp Trp Asp
           1010                1015                1020
   aag tac aac aag agg tac tcc acc tgg gct ggt gtc tct caa ctt gtc     3119
   Lys Tyr Asn Lys Arg Tyr Ser Thr Trp Ala Gly Val Ser Gln Leu Val
       1025                1030                1035
   tac tac cca gag aac tac att gac cca acc atg agg att ggt cag acc     3167
   Tyr Tyr Pro Glu Asn Tyr Ile Asp Pro Thr Met Arg Ile Gly Gln Thr
   1040                1045                1050                1055
   aag atg atg gat gct ctc ttg caa tct gtc tcc caa agc caa ctc aac     3215
   Lys Met Met Asp Ala Leu Leu Gln Ser Val Ser Gln Ser Gln Leu Asn
                   1060                1065                1070
   gct gac act gtg gag gat gcc ttc atg agc tac ctc acc tcc ttc gag     3263
   Ala Asp Thr Val Glu Asp Ala Phe Met Ser Tyr Leu Thr Ser Phe Glu
               1075                1080                1085
   caa gtt gcc aac ctc aag gtc atc tct gct tac cat gac aac atc aac     3311
   Gln Val Ala Asn Leu Lys Val Ile Ser Ala Tyr His Asp Asn Ile Asn
           1090                1095                1100
   aac gac caa ggt ctc acc tac ttc att ggt ctc tct gag act gat gct     3359
   Asn Asp Gln Gly Leu Thr Tyr Phe Ile Gly Leu Ser Glu Thr Asp Ala
       1105                1110                1115
   ggt gag tac tac tgg aga tcc gtg gac cac agc aag ttc aac gat ggc     3407
   Gly Glu Tyr Tyr Trp Arg Ser Val Asp His Ser Lys Phe Asn Asp Gly
   1120                1125                1130                1135
   aag ttc gct gca aac gct tgg tct gag tgg cac aag att gac tgc cct     3455
   Lys Phe Ala Ala Asn Ala Trp Ser Glu Trp His Lys Ile Asp Cys Pro
                   1140                1145                1150
   atc aac cca tac aag tcc acc atc aga cct gtc atc tac aag agc cgc     3503
   Ile Asn Pro Tyr Lys Ser Thr Ile Arg Pro Val Ile Tyr Lys Ser Arg
               1155                1160                1165
   ctc tac ttg ctc tgg ctt gag cag aag gag atc acc aag caa act ggc     3551
   Leu Tyr Leu Leu Trp Leu Glu Gln Lys Glu Ile Thr Lys Gln Thr Gly
           1170                1175                1180
   aac tcc aag gat ggt tac caa act gag act gac tac cgc tac gag ttg     3599
   Asn Ser Lys Asp Gly Tyr Gln Thr Glu Thr Asp Tyr Arg Tyr Glu Leu
       1185                1190                1195
   aag ttg gct cac atc cgc tac gat ggt acc tgg aac act cca atc acc     3647
   Lys Leu Ala His Ile Arg Tyr Asp Gly Thr Trp Asn Thr Pro Ile Thr
   1200                1205                1210                1215
   ttc gat gtc aac aag aag atc agc gag ttg aag ttg gag aag aac cgt     3695
   Phe Asp Val Asn Lys Lys Ile Ser Glu Leu Lys Leu Glu Lys Asn Arg
                   1220                1225                1230
   gct cct ggt ctc tac tgc gct ggt tac caa ggt gag gac acc ctc ttg     3743
   Ala Pro Gly Leu Tyr Cys Ala Gly Tyr Gln Gly Glu Asp Thr Leu Leu
               1235                1240                1245
   gtc atg ttc tac aac cag caa gac acc ctt gac tcc tac aag aac gct     3791
   Val Met Phe Tyr Asn Gln Gln Asp Thr Leu Asp Ser Tyr Lys Asn Ala
           1250                1255                1260
   tcc atg caa ggt ctc tac atc ttc gct gac atg gct tcc aag gac atg     3839
   Ser Met Gln Gly Leu Tyr Ile Phe Ala Asp Met Ala Ser Lys Asp Met
       1265                1270                1275
   act cca gag caa agc aac gtc tac cgt gac aac tcc tac caa cag ttc     3887
   Thr Pro Glu Gln Ser Asn Val Tyr Arg Asp Asn Ser Tyr Gln Gln Phe
   1280                1285                1290                1295
   gac acc aac aac gtc agg cgt gtc aac aac aga tac gct gag gac tac    3935
   Asp Thr Asn Asn Val Arg Arg Val Asn Asn Arg Tyr Ala Glu Asp Tyr
                   1300                1305                1310
   gag atc cca agc tct gtc agc tct cgc aag gac tac ggc tgg ggt gac    3983
   Glu Ile Pro Ser Ser Val Ser Ser Arg Lys Asp Tyr Gly Trp Gly Asp
               1315                1320                1325
   tac tac ctc agc atg gtg tac aac ggt gac atc cca acc atc aac tac    4031
   Tyr Tyr Leu Ser Met Val Tyr Asn Gly Asp Ile Pro Thr Ile Asn Tyr
           1330                1335                1340
   aag gct gcc tct tcc gac ctc aaa atc tac atc agc cca aag ctc agg    4079
   Lys Ala Ala Ser Ser Asp Leu Lys Ile Tyr Ile Ser Pro Lys Leu Arg
       1345                1350                1355
   atc atc cac aac ggc tac gag ggt cag aag agg aac cag tgc aac ttg    4127
   Ile Ile His Asn Gly Tyr Glu Gly Gln Lys Arg Asn Gln Cys Asn Leu
   1360                1365                1370                1375
   atg aac aag tac ggc aag ttg ggt gac aag ttc att gtc tac acc tct    4175
   Met Asn Lys Tyr Gly Lys Leu Gly Asp Lys Phe Ile Val Tyr Thr Ser
                   1380                1385                1390
   ctt ggt gtc aac cca aac aac agc tcc aac aag ctc atg ttc tac cca    4223
   Leu Gly Val Asn Pro Asn Asn Ser Ser Asn Lys Leu Met Phe Tyr Pro
               1395                1400                1405
   gtc tac caa tac tct ggc aac acc tct ggt ctc aac cag ggt aga ctc    4271
   Val Tyr Gln Tyr Ser Gly Asn Thr Ser Gly Leu Asn Gln Gly Arg Leu
           1410                1415                1420
   ttg ttc cac agg gac acc acc tac cca agc aag gtg gag gct tgg att    4319
   Leu Phe His Arg Asp Thr Thr Tyr Pro Ser Lys Val Glu Ala Trp Ile
       1425                1430                1435
   cct ggt gcc aag agg tcc ctc acc aac cag aac gct gcc att ggt gat    4367
   Pro Gly Ala Lys Arg Ser Leu Thr Asn Gln Asn Ala Ala Ile Gly Asp
   1440                1445                1450                1455
   gac tac gcc aca gac tcc ctc aac aag cct gat gac ctc aag cag tac    4415
   Asp Tyr Ala Thr Asp Ser Leu Asn Lys Pro Asp Asp Leu Lys Gln Tyr
                   1460                1465                1470
   atc ttc atg act gac tcc aag ggc aca gcc act gat gtc tct ggt cca    4463
   Ile Phe Met Thr Asp Ser Lys Gly Thr Ala Thr Asp Val Ser Gly Pro
               1475                1480                1485
   gtg gag atc aac act gca atc agc cca gcc aag gtc caa atc att gtc    4511
   Val Glu Ile Asn Thr Ala Ile Ser Pro Ala Lys Val Gln Ile Ile Val
           1490                1495                1500
   aag gct ggt ggc aag gag caa acc ttc aca gct gac aag gat gtc tcc    4559
   Lys Ala Gly Gly Lys Glu Gln Thr Phe Thr Ala Asp Lys Asp Val Ser
       1505                1510                1515
   atc cag cca agc cca tcc ttc gat gag atg aac tac caa ttc aac gct    4607
   Ile Gln Pro Ser Pro Ser Phe Asp Glu Met Asn Tyr Gln Phe Asn Ala
   1520                1525                1530                1535
   ctt gag att gat ggt tct ggc ctc aac ttc atc aac aac tct gct tcc    4655
   Leu Glu Ile Asp Gly Ser Gly Leu Asn Phe Ile Asn Asn Ser Ala Ser
                   1540                1545                1550
   att gat gtc acc ttc act gcc ttc gct gag gat ggc cgc aag ttg ggt     4703
   Ile Asp Val Thr Phe Thr Ala Phe Ala Glu Asp Gly Arg Lys Leu Gly
               1555                1560                1565
   tac gag agc ttc tcc atc cca gtc acc ctt aag gtt tcc act gac aac     4751
   Tyr Glu Ser Phe Ser Ile Pro Val Thr Leu Lys Val Ser Thr Asp Asn
           1570                1575                1580
   gca ctc acc ctt cat cac aac gag aac ggt gct cag tac atg caa tgg     4799
   Ala Leu Thr Leu His His Asn Glu Asn Gly Ala Gln Tyr Met Gln Trp
       1585                1590                1595
   caa agc tac cgc acc agg ttg aac acc ctc ttc gca agg caa ctt gtg     4847
   Gln Ser Tyr Arg Thr Arg Leu Asn Thr Leu Phe Ala Arg Gln Leu Val
   1600                1605                1610                1615
   gcc cgt gcc acc aca ggc att gac acc atc ctc agc atg gag acc cag     4895
   Ala Arg Ala Thr Thr Gly Ile Asp Thr Ile Leu Ser Met Glu Thr Gln
                   1620                1625                1630
   aac atc caa gag cca cag ttg ggc aag ggt ttc tac gcc acc ttc gtc     4943
   Asn Ile Gln Glu Pro Gln Leu Gly Lys Gly Phe Tyr Ala Thr Phe Val
               1635                1640                1645
   atc cca cct tac aac ctc agc act cat ggt gat gag agg tgg ttc aag     4991
   Ile Pro Pro Tyr Asn Leu Ser Thr His Gly Asp Glu Arg Trp Phe Lys
           1650                1655                1660
   ctc tac atc aag cac gtg gtt gac aac aac tcc cac atc atc tac tct     5039
   Leu Tyr Ile Lys His Val Val Asp Asn Asn Ser His Ile Ile Tyr Ser
       1665                1670                1675
   ggt caa ctc act gac acc aac atc aac atc acc ctc ttc atc cca ctt     5087
   Gly Gln Leu Thr Asp Thr Asn Ile Asn Ile Thr Leu Phe Ile Pro Leu
   1680                1685                1690                1695
   gac gat gtc cca ctc aac cag gac tac cat gcc aag gtc tac atg acc     5135
   Asp Asp Val Pro Leu Asn Gln Asp Tyr His Ala Lys Val Tyr Met Thr
                   1700                1705                1710
   ttc aag aag tct cca tct gat ggc acc tgg tgg ggt cca cac ttc gtc     5183
   Phe Lys Lys Ser Pro Ser Asp Gly Thr Trp Trp Gly Pro His Phe Val
               1715                1720                1725
   cgt gat gac aag ggc atc gtc acc atc aac cca aag tcc atc ctc acc     5231
   Arg Asp Asp Lys Gly Ile Val Thr Ile Asn Pro Lys Ser Ile Leu Thr
           1730                1735                1740
   cac ttc gag tct gtc aac gtt ctc aac aac atc tcc tct gag cca atg     5279
   His Phe Glu Ser Val Asn Val Leu Asn Asn Ile Ser Ser Glu Pro Met
       1745                1750                1755
   gac ttc tct ggt gcc aac tcc ctc tac ttc tgg gag ttg ttc tac tac     5327
   Asp Phe Ser Gly Ala Asn Ser Leu Tyr Phe Trp Glu Leu Phe Tyr Tyr
   1760                1765                1770                1775
   aca cca atg ctt gtg gct caa agg ttg ctc cat gag cag aac ttc gat     5375
   Thr Pro Met Leu Val Ala Gln Arg Leu Leu His Glu Gln Asn Phe Asp
                   1780                1785                1790
   gag gcc aac agg tgg ctc aag tac gtc tgg agc cca tct ggt tac att     5423
   Glu Ala Asn Arg Trp Leu Lys Tyr Val Trp Ser Pro Ser Gly Tyr Ile
               1795                1800                1805
   gtg cat ggt caa atc cag aac tac caa tgg aac gtc agg cca ttg ctt     5471
   Val His Gly Gln Ile Gln Asn Tyr Gln Trp Asn Val Arg Pro Leu Leu
           1810                1815                1820
   gag gac acc tcc tgg aac tct gac cca ctt gac tct gtg gac cct gat     5519
   Glu Asp Thr Ser Trp Asn Ser Asp Pro Leu Asp Ser Val Asp Pro Asp
       1825                1830                1835
   gct gtg gct caa cat gac cca atg cac tac aag gtc tcc acc ttc atg     5567
   Ala Val Ala Gln His Asp Pro Met His Tyr Lys Val Ser Thr Phe Met
   1840                1845                1850                1855
   agg acc ttg gac ctc ttg att gcc aga ggt gac cat gct tac cgc caa     5615
   Arg Thr Leu Asp Leu Leu Ile Ala Arg Gly Asp His Ala Tyr Arg Gln
                   1860                1865                1870
   ttg gag agg gac acc ctc aac gag gca aag atg tgg tac atg caa gct     5663
   Leu Glu Arg Asp Thr Leu Asn Glu Ala Lys Met Trp Tyr Met Gln Ala
               1875                1880                1885
   ctc cac ctc ttg ggt gac aag cca tac ctc cca ctc agc acc act tgg     5711
   Leu His Leu Leu Gly Asp Lys Pro Tyr Leu Pro Leu Ser Thr Thr Trp
           1890                1895                1900
   tcc gac cca agg ttg gac cgt gct gct gac atc acc act cag aac gct     5759
   Ser Asp Pro Arg Leu Asp Arg Ala Ala Asp Ile Thr Thr Gln Asn Ala
       1905                1910                1915
   cat gac tct gcc att gtt gct ctc agg cag aac atc cca act cct gct     5807
   His Asp Ser Ala Ile Val Ala Leu Arg Gln Asn Ile Pro Thr Pro Ala
   1920                1925                1930                1935
   cca ctc tcc ctc aga tct gct aac acc ctc act gac ttg ttc ctc cca     5855
   Pro Leu Ser Leu Arg Ser Ala Asn Thr Leu Thr Asp Leu Phe Leu Pro
                   1940                1945                1950
   cag atc aac gag gtc atg atg aac tac tgg caa acc ttg gct caa agg     5903
   Gln Ile Asn Glu Val Met Met Asn Tyr Trp Gln Thr Leu Ala Gln Arg
               1955                1960                1965
   gtc tac aac ctc aga cac aac ctc tcc att gat ggt caa cca ctc tac     5951
   Val Tyr Asn Leu Arg His Asn Leu Ser Ile Asp Gly Gln Pro Leu Tyr
           1970                1975                1980
   ctc cca atc tac gcc aca cca gct gac cca aag gct ctt ctc tct gct     5999
   Leu Pro Ile Tyr Ala Thr Pro Ala Asp Pro Lys Ala Leu Leu Ser Ala
       1985                1990                1995
   gct gtg gct acc agc caa ggt ggt ggc aag ctc cca gag tcc ttc atg     6047
   Ala Val Ala Thr Ser Gln Gly Gly Gly Lys Leu Pro Glu Ser Phe Met
   2000                2005                2010                2015
   tcc ctc tgg agg ttc cca cac atg ttg gag aac gcc cgt ggc atg gtc     6095
   Ser Leu Trp Arg Phe Pro His Met Leu Glu Asn Ala Arg Gly Met Val
                   2020                2025                2030
   tcc caa ctc acc cag ttc ggt tcc acc ctc cag aac atc att gag agg     6143
   Ser Gln Leu Thr Gln Phe Gly Ser Thr Leu Gln Asn Ile Ile Glu Arg
               2035                2040                2045
   caa gat gct gag gct ctc aac gct ttg ctc cag aac cag gca gct gag     6191
   Gln Asp Ala Glu Ala Leu Asn Ala Leu Leu Gln Asn Gln Ala Ala Glu
           2050                2055                2060
   ttg atc ctc acc aac ttg tcc atc caa gac aag acc att gag gag ctt     6239
   Leu Ile Leu Thr Asn Leu Ser Ile Gln Asp Lys Thr Ile Glu Glu Leu
       2065                2070                2075
   gat gct gag aag aca gtc ctt gag aag agc aag gct ggt gcc caa tct     6287
   Asp Ala Glu Lys Thr Val Leu Glu Lys Ser Lys Ala Gly Ala Gln Ser
   2080                2085                2090                2095
   cgc ttc gac tcc tac ggc aag ctc tac gat gag aac atc aac gct ggt     6335
   Arg Phe Asp Ser Tyr Gly Lys Leu Tyr Asp Glu Asn Ile Asn Ala Gly
                   2100                2105                2110
   gag aac cag gcc atg acc ctc agg gct tcc gca gct ggt ctc acc act     6383
   Glu Asn Gln Ala Met Thr Leu Arg Ala Ser Ala Ala Gly Leu Thr Thr
               2115                2120                2125
   gct gtc caa gcc tct cgc ttg gct ggt gca gct gct gac ctc gtt cca     6431
   Ala Val Gln Ala Ser Arg Leu Ala Gly Ala Ala Ala Asp Leu Val Pro
           2130                2135                2140
   aac atc ttc ggt ttc gct ggt ggt ggc tcc aga tgg ggt gcc att gct     6479
   Asn Ile Phe Gly Phe Ala Gly Gly Gly Ser Arg Trp Gly Ala Ile Ala
       2145                2150                2155
   gag gct acc ggt tac gtc atg gag ttc tct gcc aac gtc atg aac act     6527
   Glu Ala Thr Gly Tyr Val Met Glu Phe Ser Ala Asn Val Met Asn Thr
   2160                2165                2170                2175
   gag gct gac aag atc agc caa tct gag acc tac aga agg cgc cgt caa     6575
   Glu Ala Asp Lys Ile Ser Gln Ser Glu Thr Tyr Arg Arg Arg Arg Gln
                   2180                2185                2190
   gag tgg gag atc caa agg aac aac gct gag gca gag ttg aag caa atc     6623
   Glu Trp Glu Ile Gln Arg Asn Asn Ala Glu Ala Glu Leu Lys Gln Ile
               2195                2200                2205
   gat gct caa ctc aag tcc ttg gct gtc aga agg gag gct gct gtc ctc     6671
   Asp Ala Gln Leu Lys Ser Leu Ala Val Arg Arg Glu Ala Ala Val Leu
           2210                2215                2220
   cag aag acc tcc ctc aag acc caa cag gag caa acc cag tcc cag ttg     6719
   Gln Lys Thr Ser Leu Lys Thr Gln Gln Glu Gln Thr Gln Ser Gln Leu
       2225                2230                2235
   gct ttc ctc caa agg aag ttc tcc aac cag gct ctc tac aac tgg ctc     6767
   Ala Phe Leu Gln Arg Lys Phe Ser Asn Gln Ala Leu Tyr Asn Trp Leu
   2240                2245                2250                2255
   aga ggc cgc ttg gct gcc atc tac ttc caa ttc tac gac ctt gct gtg     6815
   Arg Gly Arg Leu Ala Ala Ile Tyr Phe Gln Phe Tyr Asp Leu Ala Val
                   2260                2265                2270
   gcc agg tgc ctc atg gct gag caa gcc tac cgc tgg gag ttg aac gat     6863
   Ala Arg Cys Leu Met Ala Glu Gln Ala Tyr Arg Trp Glu Leu Asn Asp
               2275                2280                2285
   gac tcc gcc agg ttc atc aag cca ggt gct tgg caa ggc acc tac gct     6911
   Asp Ser Ala Arg Phe Ile Lys Pro Gly Ala Trp Gln Gly Thr Tyr Ala
           2290                2295                2300
   ggt ctc ctt gct ggt gag acc ctc atg ctc tcc ttg gct caa atg gag     6959
   Gly Leu Leu Ala Gly Glu Thr Leu Met Leu Ser Leu Ala Gln Met Glu
       2305                2310                2315
   gat gct cac ctc aag agg gac aag agg gct ttg gag gtg gag agg aca     7007
   Asp Ala His Leu Lys Arg Asp Lys Arg Ala Leu Glu Val Glu Arg Thr
   2320                2325                2330                2335
   gtc tcc ctt gct gag gtc tac gct ggt ctc cca aag gac aac ggt cca     7055
   Val Ser Leu Ala Glu Val Tyr Ala Gly Leu Pro Lys Asp Asn Gly Pro
                   2340                2345                2350
   ttc tcc ctt gct caa gag att gac aag ttg gtc agc caa ggt tct ggt     7103
   Phe Ser Leu Ala Gln Glu Ile Asp Lys Leu Val Ser Gln Gly Ser Gly
               2355                2360                2365
   tct gct ggt tct ggt aac aac aac ttg gct ttc ggc gct ggt act gac     7151
   Ser Ala Gly Ser Gly Asn Asn Asn Leu Ala Phe Gly Ala Gly Thr Asp
           2370                2375                2380
   acc aag acc tcc ctc caa gcc tct gtc tcc ttc gct gac ctc aag atc     7199
   Thr Lys Thr Ser Leu Gln Ala Ser Val Ser Phe Ala Asp Leu Lys Ile
       2385                2390                2395
   agg gag gac tac cca gct tcc ctt ggc aag atc agg cgc atc aag caa     7247
   Arg Glu Asp Tyr Pro Ala Ser Leu Gly Lys Ile Arg Arg Ile Lys Gln
   2400                2405                2410                2415
   atc tct gtc acc ctc cca gct ctc ttg ggt cca tac caa gat gtc caa     7295
   Ile Ser Val Thr Leu Pro Ala Leu Leu Gly Pro Tyr Gln Asp Val Gln
                   2420                2425                2430
   gca atc ctc tcc tac ggt gac aag gct ggt ttg gcg aac ggt tgc gag     7343
   Ala Ile Leu Ser Tyr Gly Asp Lys Ala Gly Leu Ala Asn Gly Cys Glu
               2435                2440                2445
   gct ctt gct gtc tct cat ggc atg aac gac tct ggt caa ttc caa ctt     7391
   Ala Leu Ala Val Ser His Gly Met Asn Asp Ser Gly Gln Phe Gln Leu
           2450                2455                2460
   gac ttc aac gat ggc aag ttc ctc cca ttc gag ggc att gcc att gac     7439
   Asp Phe Asn Asp Gly Lys Phe Leu Pro Phe Glu Gly Ile Ala Ile Asp
       2465                2470                2475
   caa ggc acc ctc acc ctc tcc ttc cca aac gct tcc atg cca gag aag     7487
   Gln Gly Thr Leu Thr Leu Ser Phe Pro Asn Ala Ser Met Pro Glu Lys
   2480                2485                2490                2495
   gga aag caa gcc acc atg ctc aag acc ctc aac gat atc atc ctc cac     7535
   Gly Lys Gln Ala Thr Met Leu Lys Thr Leu Asn Asp Ile Ile Leu His
                   2500                2505                2510
   atc agg tac acc atc aag tgagctcgag aggcctgcgg ccgc                  7577
   Ile Arg Tyr Thr Ile Lys
   2515
<210> 4
   <211> 7541
   <212> DNA
   <213> Artificial Sequence
<220>
   <221> CDS
   <222> (3)..(7517)
<220>
   <223> Description of Artificial Sequence:hemicot tcbA
<400> 4
   cc atg gct cag aac tcc ctc agc tcc acc att gac acc atc tgc cag     47
      Met Ala Gln Asn Ser Leu Ser Ser Thr Ile Asp Thr Ile Cys Gln
        1               5                  10                  15
   aag ctt caa ctc acc tgc cca gct gag atc gcc ctc tac cca ttc gac    95
   Lys Leu Gln Leu Thr Cys Pro Ala Glu Ile Ala Leu Tyr Pro Phe Asp
                   20                  25                  30
   acc ttc cgt gag aag acc aga ggc atg gtc aac tgg ggt gag gcc aag    143
   Thr Phe Arg Glu Lys Thr Arg Gly Met Val Asn Trp Gly Glu Ala Lys
               35                  40                  45
   agg atc tac gag att gct caa gct gag caa gac agg aac ctc ctt cat    191
   Arg Ile Tyr Glu Ile Ala Gln Ala Glu Gln Asp Arg Asn Leu Leu His
           50                  55                  60
   gag aag agg atc ttc gcc tac gct aac cca ttg ctc aag aac gct gtc    239
   Glu Lys Arg Ile Phe Ala Tyr Ala Asn Pro Leu Leu Lys Asn Ala Val
       65                  70                  75
   agg ctt ggt acc agg caa atg ttg ggt ttc atc caa ggt tac tct gac    287
   Arg Leu Gly Thr Arg Gln Met Leu Gly Phe Ile Gln Gly Tyr Ser Asp
   80                  85                  90                  95
   ttg ttc ggc aac agg gct gac aac tac gca gct cct ggt tct gtt gct    335
   Leu Phe Gly Asn Arg Ala Asp Asn Tyr Ala Ala Pro Gly Ser Val Ala
                   100                 105                 110
   agc atg ttc agc cca gct gcc tac ctc act gag ttg tac cgt gag gcc    383
   Ser Met Phe Ser Pro Ala Ala Tyr Leu Thr Glu Leu Tyr Arg Glu Ala
               115                 120                 125
   aag aac ctc cat gac agc tcc agc atc tac tac ctt gac aag agg cgc    431
   Lys Asn Leu His Asp Ser Ser Ser Ile Tyr Tyr Leu Asp Lys Arg Arg
           130                 135                 140
   cca gac ctt gct tcc ttg atg ctc tcc cag aag aac atg gat gag gag    479
   Pro Asp Leu Ala Ser Leu Met Leu Ser Gln Lys Asn Met Asp Glu Glu
       145                 150                 155
   atc agc acc ttg gct ctc tcc aac gag ctt tgc ttg gct ggc att gag    527
   Ile Ser Thr Leu Ala Leu Ser Asn Glu Leu Cys Leu Ala Gly Ile Glu
   160                 165                 170                 175
   acc aag act ggc aag tcc caa gat gag gtc atg gac atg ctc tcc acc     575
   Thr Lys Thr Gly Lys Ser Gln Asp Glu Val Met Asp Met Leu Ser Thr
                   180                 185                 190
   tac cgc ctc tct ggt gag act cca tac cac cat gct tac gag act gtc     623
   Tyr Arg Leu Ser Gly Glu Thr Pro Tyr His His Ala Tyr Glu Thr Val
               195                 200                 205
   agg gag att gtc cat gag agg gac cca ggt ttc cgc cac ctc tcc caa     671
   Arg Glu Ile Val His Glu Arg Asp Pro Gly Phe Arg His Leu Ser Gln
           210                 215                 220
   gct ccc att gtg gct gcc aag ttg gac cca gtc acc ctc ttg ggc atc     719
   Ala Pro Ile Val Ala Ala Lys Leu Asp Pro Val Thr Leu Leu Gly Ile
       225                 230                 235
   tcc agc cac atc agc cca gag ttg tac aac ctt ctc att gag gag atc     767
   Ser Ser His Ile Ser Pro Glu Leu Tyr Asn Leu Leu Ile Glu Glu Ile
   240                 245                 250                 255
   cca gag aag gat gag gca gct ttg gac acc ctc tac aag acc aac ttc     815
   Pro Glu Lys Asp Glu Ala Ala Leu Asp Thr Leu Tyr Lys Thr Asn Phe
                   260                 265                 270
   ggt gac atc acc act gct caa ctc atg agc cca tcc tac ttg gcc agg     863
   Gly Asp Ile Thr Thr Ala Gln Leu Met Ser Pro Ser Tyr Leu Ala Arg
               275                 280                 285
   tac tac ggt gtc tct cca gag gac att gct tac gtc acc aca agc ctc     911
   Tyr Tyr Gly Val Ser Pro Glu Asp Ile Ala Tyr Val Thr Thr Ser Leu
           290                 295                 300
   tcc cat gtg ggt tac tcc tct gac atc ctt gtc atc cca ctc gtg gat     959
   Ser His Val Gly Tyr Ser Ser Asp Ile Leu Val Ile Pro Leu Val Asp
       305                 310                 315
   ggt gtg ggc aag atg gag gtt gtc agg gtc acc agg act cca tct gac     1007
   Gly Val Gly Lys Met Glu Val Val Arg Val Thr Arg Thr Pro Ser Asp
   320                 325                 330                 335
   aac tac acc tcc cag acc aac tac att gag ttg tac cca caa ggt ggt     1055
   Asn Tyr Thr Ser Gln Thr Asn Tyr Ile Glu Leu Tyr Pro Gln Gly Gly
                   340                 345                 350
   gac aac tac ctc atc aag tac aac ctc tcc aac tct ttc ggt ttg gat     1103
   Asp Asn Tyr Leu Ile Lys Tyr Asn Leu Ser Asn Ser Phe Gly Leu Asp
               355                 360                 365
   gac ttc tac ctc cag tac aag gat ggt tct gct gac tgg act gag att     1151
   Asp Phe Tyr Leu Gln Tyr Lys Asp Gly Ser Ala Asp Trp Thr Glu Ile
           370                 375                 380
   gct cac aac cca tac cca gac atg gtc atc aac cag aag tac gag tcc     1199
   Ala His Asn Pro Tyr Pro Asp Met Val Ile Asn Gln Lys Tyr Glu Ser
       385                 390                 395
   caa gcc acc atc aag aga tct gac tct gac aac atc ctc tcc att ggt     1247
   Gln Ala Thr Ile Lys Arg Ser Asp Ser Asp Asn Ile Leu Ser Ile Gly
   400                 405                 410                 415
   ctc caa agg tgg cac tct ggt tcc tac aac ttc gct gct gcc aac ttc     1295
   Leu Gln Arg Trp His Ser Gly Ser Tyr Asn Phe Ala Ala Ala Asn Phe
                   420                 425                 430
   aag att gac caa tac tct cca aag gct ttc ctc ttg aag atg aac aag     1343
   Lys Ile Asp Gln Tyr Ser Pro Lys Ala Phe Leu Leu Lys Met Asn Lys
               435                 440                 445
   gcc atc agg ctc ttg aag gcc act ggt ctc tcc ttc gcc acc ctt gag     1391
   Ala Ile Arg Leu Leu Lys Ala Thr Gly Leu Ser Phe Ala Thr Leu Glu
           450                 455                 460
   agg att gtg gac tct gtc aac tcc acc aag tcc atc act gtg gag gtc     1439
   Arg Ile Val Asp Ser Val Asn Ser Thr Lys Ser Ile Thr Val Glu Val
       465                 470                 475
   ctc aac aag gtc tac aga gtc aag ttc tac att gac cgc tac ggc atc     1487
   Leu Asn Lys Val Tyr Arg Val Lys Phe Tyr Ile Asp Arg Tyr Gly Ile
   480                 485                 490                 495
   tct gag gag act gct gcc atc ctt gcc aac atc aac atc tcc cag caa     1535
   Ser Glu Glu Thr Ala Ala Ile Leu Ala Asn Ile Asn Ile Ser Gln Gln
                   500                 505                 510
   gct gtc ggc aac cag ctc tcc caa ttc gag caa ctc ttc aac cac cct     1583
   Ala Val Gly Asn Gln Leu Ser Gln Phe Glu Gln Leu Phe Asn His Pro
               515                 520                 525
   cca ctc aac ggc atc cgc tac gag atc agc gag gac aac tcc aag cac     1631
   Pro Leu Asn Gly Ile Arg Tyr Glu Ile Ser Glu Asp Asn Ser Lys His
           530                 535                 540
   ctc cca aac cca gac ctc aac ctc aag cca gac tcc act ggt gat gac     1679
   Leu Pro Asn Pro Asp Leu Asn Leu Lys Pro Asp Ser Thr Gly Asp Asp
       545                 550                 555
   caa agg aag gct gtc ctc aag agg gct ttc caa gtc aac gct tct gag     1727
   Gln Arg Lys Ala Val Leu Lys Arg Ala Phe Gln Val Asn Ala Ser Glu
   560                 565                 570                 575
   ctt tac caa atg ctc ttg atc act gac agg aag gag gat ggt gtc atc     1775
   Leu Tyr Gln Met Leu Leu Ile Thr Asp Arg Lys Glu Asp Gly Val Ile
                   580                 585                 590
   aag aac aac ttg gag aac ctc tct gac ctc tac ctt gtc tcc ctc ttg     1823
   Lys Asn Asn Leu Glu Asn Leu Ser Asp Leu Tyr Leu Val Ser Leu Leu
               595                 600                 605
   gcc caa atc cac aac ttg acc att gct gag ttg aac atc ctc ttg gtc     1871
   Ala Gln Ile His Asn Leu Thr Ile Ala Glu Leu Asn Ile Leu Leu Val
           610                 615                 620
   atc tgc ggt tac ggt gac acc aac atc tac caa atc act gac gac aac     1919
   Ile Cys Gly Tyr Gly Asp Thr Asn Ile Tyr Gln Ile Thr Asp Asp Asn
       625                 630                 635
   ctt gcc aag att gtg gag acc ctc ttg tgg atc acc caa tgg ctc aag     1967
   Leu Ala Lys Ile Val Glu Thr Leu Leu Trp Ile Thr Gln Trp Leu Lys
   640                 645                 650                 655
   acc cag aag tgg act gtc aca gac ctc ttc ctc atg acc act gcc acc     2015
   Thr Gln Lys Trp Thr Val Thr Asp Leu Phe Leu Met Thr Thr Ala Thr
                       660                 665                 670
   tac tcc acc act ctc act cca gag att tcc aac ctc act gcc acc ctc      2063
   Tyr Ser Thr Thr Leu Thr Pro Glu Ile Ser Asn Leu Thr Ala Thr Leu
               675                 680                 685
   agc tcc acc ctc cac ggc aag gag tcc ctc att ggt gag gac ctc aag      2111
   Ser Ser Thr Leu His Gly Lys Glu Ser Leu Ile Gly Glu Asp Leu Lys
           690                 695                 700
   agg gca atg gct cca tgc ttc acc tct gct ctc cac ctc acc tcc caa      2159
   Arg Ala Met Ala Pro Cys Phe Thr Ser Ala Leu His Leu Thr Ser Gln
       705                 710                 715
   gag gtg gct tac gac ctc ctt ctc tgg att gac caa atc caa cca gct      2207
   Glu Val Ala Tyr Asp Leu Leu Leu Trp Ile Asp Gln Ile Gln Pro Ala
   720                 725                 730                 735
   caa atc act gtg gat ggt ttc tgg gag gag gtc caa acc act cca acc      2255
   Gln Ile Thr Val Asp Gly Phe Trp Glu Glu Val Gln Thr Thr Pro Thr
                   740                 745                 750
   tcc ctc aag gtc atc acc ttc gct caa gtc ttg gct caa ctc tcc ctc      2303
   Ser Leu Lys Val Ile Thr Phe Ala Gln Val Leu Ala Gln Leu Ser Leu
               755                 760                 765
   atc tac aga agg att ggt ctc tct gag act gag ttg tcc ctc att gtc      2351
   Ile Tyr Arg Arg Ile Gly Leu Ser Glu Thr Glu Leu Ser Leu Ile Val
           770                 775                 780
   acc caa tcc agc ctc ttg gtc gct ggc aag tcc atc ctt gat cat ggt      2399
   Thr Gln Ser Ser Leu Leu Val Ala Gly Lys Ser Ile Leu Asp His Gly
       785                 790                 795
   ctc ttg acc ctc atg gct ctt gag ggt ttc cac acc tgg gtc aac ggt      2447
   Leu Leu Thr Leu Met Ala Leu Glu Gly Phe His Thr Trp Val Asn Gly
   800                 805                 810                 815
   ttg ggt caa cat gct tcc ctc atc ttg gct gca ctc aag gat ggt gct      2495
   Leu Gly Gln His Ala Ser Leu Ile Leu Ala Ala Leu Lys Asp Gly Ala
                   820                 825                 830
   ctc acc gtc acc gat gtg gct caa gcc atg aac aag gag gag tcc ctc      2543
   Leu Thr Val Thr Asp Val Ala Gln Ala Met Asn Lys Glu Glu Ser Leu
               835                 840                 845
   ttg caa atg gct gcc aac cag gtg gag aag gac ctc acc aag ctc acc      2591
   Leu Gln Met Ala Ala Asn Gln Val Glu Lys Asp Leu Thr Lys Leu Thr
           850                 855                 860
   tcc tgg acc caa atc gat gcc atc ctc caa tgg ctc caa atg tcc tct      2639
   Ser Trp Thr Gln Ile Asp Ala Ile Leu Gln Trp Leu Gln Met Ser Ser
       865                 870                 875
   gct ctt gct gtc agc cca ttg gac ctt gct ggc atg atg gct ctc aag      2687
   Ala Leu Ala Val Ser Pro Leu Asp Leu Ala Gly Met Met Ala Leu Lys
   880                 885                 890                 895
   tac ggc att gat cac aac tac gct gcc tgg caa gca gct gcc gct gcc      2735
   Tyr Gly Ile Asp His Asn Tyr Ala Ala Trp Gln Ala Ala Ala Ala Ala
                   900                 905                 910
   ctc atg gct gac cat gcc aac cag gct cag aag aag ttg gat gag acc    2783
   Leu Met Ala Asp His Ala Asn Gln Ala Gln Lys Lys Leu Asp Glu Thr
                   915                 920                 925
   ttc tcc aag gct ctc tgc aac tac tac atc aac gcc gtg gtt gac tct    2831
   Phe Ser Lys Ala Leu Cys Asn Tyr Tyr Ile Asn Ala Val Val Asp Ser
               930                 935                 940
   gct gcc ggt gtc agg gac agg aac ggt ctc tac acc tac ctc ttg att    2879
   Ala Ala Gly Val Arg Asp Arg Asn Gly Leu Tyr Thr Tyr Leu Leu Ile
       945                 950                 955
   gac aac cag gtc tct gct gat gtc atc acc tcc aga att gct gag gcc    2927
   Asp Asn Gln Val Ser Ala Asp Val Ile Thr Ser Arg Ile Ala Glu Ala
   960                 965                 970                 975
   att gct ggc atc caa ctc tac gtc aac agg gct ctc aac agg gat gag    2975
   Ile Ala Gly Ile Gln Leu Tyr Val Asn Arg Ala Leu Asn Arg Asp Glu
                   980                 985                 990
   ggt cag ttg gct tct gat gtc tcc acc agg caa ttc ttc acc gac tgg    3023
   Gly Gln Leu Ala Ser Asp Val Ser Thr Arg Gln Phe Phe Thr Asp Trp
                   995                1000                1005
   gag agg tac aac aag agg tac tcc acc tgg gct ggt gtc tct gag ttg    3071
   Glu Arg Tyr Asn Lys Arg Tyr Ser Thr Trp Ala Gly Val Ser Glu Leu
              1010                1015                1020
   gtc tac tac cca gag aac tac gtg gac cca acc caa agg att ggt cag    3119
   Val Tyr Tyr Pro Glu Asn Tyr Val Asp Pro Thr Gln Arg Ile Gly Gln
          1025                1030                1035
   acc aag atg atg gat gct ttg ctc caa tcc atc aac cag tcc caa ctc    3167
   Thr Lys Met Met Asp Ala Leu Leu Gln Ser Ile Asn Gln Ser Gln Leu
   1040                1045                1050                1055
   aac gct gac act gtg gag gat gct ttc aag acc tac ctc acc tcc ttc    3215
   Asn Ala Asp Thr Val Glu Asp Ala Phe Lys Thr Tyr Leu Thr Ser Phe
                   1060                1065                1070
   gag caa gtg gcc aac ctc aag gtc atc tct gct tac cat gac aac gtc    3263
   Glu Gln Val Ala Asn Leu Lys Val Ile Ser Ala Tyr His Asp Asn Val
               1075                1080                1085
   aac gtg gac caa ggt ctc acc tac ttc att ggc att gac caa gcc gct    3311
   Asn Val Asp Gln Gly Leu Thr Tyr Phe Ile Gly Ile Asp Gln Ala Ala
              1090                1095                1100
   cct ggc acc tac tac tgg agg tct gtg gac cac tcc aag tgc gag aac    3359
   Pro Gly Thr Tyr Tyr Trp Arg Ser Val Asp His Ser Lys Cys Glu Asn
          1105                1110                1115
   ggc aag ttc gct gcc aac gct tgg ggt gag tgg aac aag atc acc tgc    3407
   Gly Lys Phe Ala Ala Asn Ala Trp Gly Glu Trp Asn Lys Ile Thr Cys
   1120                1125                1130                1135
   gct gtc aac cct tgg aag aac atc atc agg cca gtg gtc tac atg tcc    3455
   Ala Val Asn Pro Trp Lys Asn Ile Ile Arg Pro Val Val Tyr Met Ser
                   1140                1145                1150
   aga ctc tac ttg ctc tgg ctt gag caa cag tcc aag aag tct gat gac    3503
   Arg Leu Tyr Leu Leu Trp Leu Glu Gln Gln Ser Lys Lys Ser Asp Asp
               1155                1160                1165
   ggc aag aca act atc tac cag tac aac ctc aag ttg gct cac atc cgc    3551
   Gly Lys Thr Thr Ile Tyr Gln Tyr Asn Leu Lys Leu Ala His Ile Arg
           1170                1175                1180
   tac gat ggt tcc tgg aac act cca ttc acc ttc gat gtc act gag aag    3599
   Tyr Asp Gly Ser Trp Asn Thr Pro Phe Thr Phe Asp Val Thr Glu Lys
       1185                1190                1195
   gtc aag aac tac acc tcc agc act gat gca gct gag tcc ctt ggt ctc    3647
   Val Lys Asn Tyr Thr Ser Ser Thr Asp Ala Ala Glu Ser Leu Gly Leu
   1200                1205                1210                1215
   tac tgc act ggt tac caa ggt gag gac acc ctc ttg gtc atg ttc tac    3695
   Tyr Cys Thr Gly Tyr Gln Gly Glu Asp Thr Leu Leu Val Met Phe Tyr
                   1220                1225                1230
   tcc atg caa tcc agc tac tcc agc tac act gac aac aac gct cca gtc    3743
   Ser Met Gln Ser Ser Tyr Ser Ser Tyr Thr Asp Asn Asn Ala Pro Val
               1235                1240                1245
   act ggt ctc tac atc ttc gct gac atg tcc tct gac aac atg acc aac    3791
   Thr Gly Leu Tyr Ile Phe Ala Asp Met Ser Ser Asp Asn Met Thr Asn
           1250                1255                1260
   gct caa gcc acc aac tac tgg aac aac tcc tac cca caa ttc gac act    3839
   Ala Gln Ala Thr Asn Tyr Trp Asn Asn Ser Tyr Pro Gln Phe Asp Thr
       1265                1270                1275
   gtc atg gct gac cca gac tct gac aac aag aag gtc atc acc agg cgt    3887
   Val Met Ala Asp Pro Asp Ser Asp Asn Lys Lys Val Ile Thr Arg Arg
   1280                1285                1290                1295
   gtc aac aac cgc tac gct gag gac tac gag atc cca agc tct gtc acc    3935
   Val Asn Asn Arg Tyr Ala Glu Asp Tyr Glu Ile Pro Ser Ser Val Thr
                   1300                1305                1310
   tcc aac agc aac tac tcc tgg ggt gac cac tcc ctc acc atg ctc tac    3983
   Ser Asn Ser Asn Tyr Ser Trp Gly Asp His Ser Leu Thr Met Leu Tyr
               1315                1320                1325
   ggt ggc tct gtc cca aac atc acc ttc gag tct gca gct gag gac ctc    4031
   Gly Gly Ser Val Pro Asn Ile Thr Phe Glu Ser Ala Ala Glu Asp Leu
           1330                1335                1340
   agg ctc tcc acc aac atg gct ctc tcc atc att cac aac ggt tac gct    4079
   Arg Leu Ser Thr Asn Met Ala Leu Ser Ile Ile His Asn Gly Tyr Ala
       1345                1350                1355
   ggc acc agg cgc atc caa tgc aac ctc atg aag caa tac gct tcc ctt    4127
   Gly Thr Arg Arg Ile Gln Cys Asn Leu Met Lys Gln Tyr Ala Ser Leu
   1360                1365                1370                1375
   ggt gac aag ttc att atc tac gac tcc agc ttc gat gac gcc aac agg    4175
   Gly Asp Lys Phe Ile Ile Tyr Asp Ser Ser Phe Asp Asp Ala Asn Arg
                   1380                1385                1390
   ttc aac ttg gtc cca ctc ttc aag ttc ggc aag gat gag aac tct gat    4223
   Phe Asn Leu Val Pro Leu Phe Lys Phe Gly Lys Asp Glu Asn Ser Asp
             1395                1400                1405
   gac tcc atc tgc atc tac aac gag aac cca agc tct gag gac aag aag    4271
   Asp Ser Ile Cys Ile Tyr Asn Glu Asn Pro Ser Ser Glu Asp Lys Lys
         1410                1415                1420
   tgg tac ttc agc tcc aag gac gac aac aag act gct gac tac aac ggt    4319
   Trp Tyr Phe Ser Ser Lys Asp Asp Asn Lys Thr Ala Asp Tyr Asn Gly
     1425                1430                1435
   ggc acc caa tgc att gat gct ggc acc tcc aac aag gac ttc tac tac    4367
   Gly Thr Gln Cys Ile Asp Ala Gly Thr Ser Asn Lys Asp Phe Tyr Tyr
   1440                1445                1450                1455
   aac ctc caa gag att gag gtc atc tct gtc act ggt ggc tac tgg tcc    4415
   Asn Leu Gln Glu Ile Glu Val Ile Ser Val Thr Gly Gly Tyr Trp Ser
                 1460                1465                1470
   agc tac aag atc agc aac ccc atc aac atc aac act ggc att gac tct    4463
   Ser Tyr Lys Ile Ser Asn Pro Ile Asn Ile Asn Thr Gly Ile Asp Ser
             1475                1480                1485
   gcc aag gtc aag gtc act gtc aag gct ggt ggc gat gac caa atc ttc    4511
   Ala Lys Val Lys Val Thr Val Lys Ala Gly Gly Asp Asp Gln Ile Phe
         1490                1495                1500
   act gct gac aac tcc acc tac gtc cca cag caa cct gct cca tcc ttc    4559
   Thr Ala Asp Asn Ser Thr Tyr Val Pro Gln Gln Pro Ala Pro Ser Phe
     1505                1510                1515
   gag gag atg atc tac caa ttc aac aac ctc acc att gac tgc aag aac    4607
   Glu Glu Met Ile Tyr Gln Phe Asn Asn Leu Thr Ile Asp Cys Lys Asn
   1520                1525                1530                1535
   ctc aac ttc att gac aac cag gct cac att gag att gac ttc act gcc    4655
   Leu Asn Phe Ile Asp Asn Gln Ala His Ile Glu Ile Asp Phe Thr Ala
                 1540                1545                1550
   aca gct caa gat ggc cgc ttc ttg ggt gct gag acc ttc atc att cca    4703
   Thr Ala Gln Asp Gly Arg Phe Leu Gly Ala Glu Thr Phe Ile Ile Pro
             1555                1560                1565
   gtc acc aag aag gtc ctt ggc act gag aac gtc att gct ctc tac tct    4751
   Val Thr Lys Lys Val Leu Gly Thr Glu Asn Val Ile Ala Leu Tyr Ser
         1570                1575                1580
   gag aac aac ggt gtc cag tac atg caa att ggt gct tac aga acc agg    4799
   Glu Asn Asn Gly Val Gln Tyr Met Gln Ile Gly Ala Tyr Arg Thr Arg
     1585                1590                1595
   ctc aac acc ctc ttc gct caa cag ttg gtc tcc cgt gcc aac aga ggc    4847
   Leu Asn Thr Leu Phe Ala Gln Gln Leu Val Ser Arg Ala Asn Arg Gly
   1600                1605                1610                1615
   att gat gct gtc ctc agc atg gag act cag aac atc caa gag cca caa    4895
   Ile Asp Ala Val Leu Ser Met Glu Thr Gln Asn Ile Gln Glu Pro Gln
                 1620                1625                1630
   ctt ggt gct ggc acc tac gtc caa ctt gtc ttg gac aag tac gat gag    4943
   Leu Gly Ala Gly Thr Tyr Val Gln Leu Val Leu Asp Lys Tyr Asp Glu
               1635                1640                1645
   tcc att cat ggc acc aac aag tcc ttc gcc att gag tac gtg gac atc     4991
   Ser Ile His Gly Thr Asn Lys Ser Phe Ala Ile Glu Tyr Val Asp Ile
           1650                1655                1660
   ttc aag gag aac gac tcc ttc gtc atc tac caa ggt gag ttg tct gag     5039
   Phe Lys Glu Asn Asp Ser Phe Val Ile Tyr Gln Gly Glu Leu Ser Glu
       1665                1670                1675
   acc tcc caa act gtg gtc aag gtc ttc ctc tcc tac ttc att gag gcc     5087
   Thr Ser Gln Thr Val Val Lys Val Phe Leu Ser Tyr Phe Ile Glu Ala
   1680                1685                1690                1695
   acc ggt aac aag aac cac ctc tgg gtc agg gcc aag tac cag aag gag     5135
   Thr Gly Asn Lys Asn His Leu Trp Val Arg Ala Lys Tyr Gln Lys Glu
                   1700                1705                1710
   acc act gac aag atc ctc ttc gac agg act gat gag aag gac cca cat     5183
   Thr Thr Asp Lys Ile Leu Phe Asp Arg Thr Asp Glu Lys Asp Pro His
               1715                1720                1725
   ggt tgg ttc ctc tct gat gac cac aag acc ttc tct ggt ctc agc tct     5231
   Gly Trp Phe Leu Ser Asp Asp His Lys Thr Phe Ser Gly Leu Ser Ser
           1730                1735                1740
   gct caa gct ctc aag aac gac tct gag cca atg gac ttc tct ggt gcc     5279
   Ala Gln Ala Leu Lys Asn Asp Ser Glu Pro Met Asp Phe Ser Gly Ala
       1745                1750                1755
   aac gct ctc tac ttc tgg gag ttg ttc tac tac act cca atg atg atg     5327
   Asn Ala Leu Tyr Phe Trp Glu Leu Phe Tyr Tyr Thr Pro Met Met Met
   1760                1765                1770                1775
   gct cac agg ctc ctt caa gag cag aac ttc gat gct gcc aac cac tgg     5375
   Ala His Arg Leu Leu Gln Glu Gln Asn Phe Asp Ala Ala Asn His Trp
                   1780                1785                1790
   ttc cgc tac gtc tgg agc cca tct ggt tac att gtg gat ggc aag att     5423
   Phe Arg Tyr Val Trp Ser Pro Ser Gly Tyr Ile Val Asp Gly Lys Ile
               1795                1800                1805
   gcc atc tac cac tgg aac gtc agg cca ttg gag gag gac acc tcc tgg     5471
   Ala Ile Tyr His Trp Asn Val Arg Pro Leu Glu Glu Asp Thr Ser Trp
           1810                1815                1820
   aac gct cag caa ctt gac tcc act gac cca gat gct gtg gct caa gat     5519
   Asn Ala Gln Gln Leu Asp Ser Thr Asp Pro Asp Ala Val Ala Gln Asp
       1825                1830                1835
   gac cca atg cac tac aag gtg gcc acc ttc atg gcc acc ttg gac ctt     5567
   Asp Pro Met His Tyr Lys Val Ala Thr Phe Met Ala Thr Leu Asp Leu
   1840                1845                1850                1855
   ctc atg gcc aga ggt gat gct gcc tac cgc caa ttg gag agg gac acc     5615
   Leu Met Ala Arg Gly Asp Ala Ala Tyr Arg Gln Leu Glu Arg Asp Thr
                   1860                1865                1870
   ttg gct gag gcc aag atg tgg tac acc caa gct ctc aac ttg ctg ggt     5663
   Leu Ala Glu Ala Lys Met Trp Tyr Thr Gln Ala Leu Asn Leu Leu Gly
               1875                1880                1885
   gat gag cca caa gtc atg ctc tcc aca acc tgg gcc aac cca acc ttg    5711
   Asp Glu Pro Gln Val Met Leu Ser Thr Thr Trp Ala Asn Pro Thr Leu
             1890                1895                1900
   ggc aac gct gcc tcc aag acc aca caa cag gtc agg caa cag gtc ctc    5759
   Gly Asn Ala Ala Ser Lys Thr Thr Gln Gln Val Arg Gln Gln Val Leu
         1905                1910                1915
   acc caa ctc agg ctc aac tct aga gtc aag act cca ctc ttg ggc act    5807
   Thr Gln Leu Arg Leu Asn Ser Arg Val Lys Thr Pro Leu Leu Gly Thr
   1920                1925                1930                1935
   gcc aac tcc ctc act gct ctc ttc ctc cca caa gag aac tcc aaa ctt    5855
   Ala Asn Ser Leu Thr Ala Leu Phe Leu Pro Gln Glu Asn Ser Lys Leu
                   1940                1945                1950
   aag ggt tac tgg agg acc ctt gct caa cgc atg ttc aac ctc agg cac    5903
   Lys Gly Tyr Trp Arg Thr Leu Ala Gln Arg Met Phe Asn Leu Arg His
               1955                1960                1965
   aac ctc tcc att gat ggt caa cca ctc tcc ttg cca ctc tac gct aag    5951
   Asn Leu Ser Ile Asp Gly Gln Pro Leu Ser Leu Pro Leu Tyr Ala Lys
           1970                1975                1980
   cca gct gac cca aag gct ctc ctt tcc gct gct gtc tcc gca tcc caa    5999
   Pro Ala Asp Pro Lys Ala Leu Leu Ser Ala Ala Val Ser Ala Ser Gln
       1985                1990                1995
   ggt ggt gct gac ctc cca aag gct cca ctc acc atc cac agg ttc cca    6047
   Gly Gly Ala Asp Leu Pro Lys Ala Pro Leu Thr Ile His Arg Phe Pro
   2000                2005                2010                2015
   caa atg ttg gag ggt gcc cgt ggt ctt gtc aac cag ctc atc caa ttc    6095
   Gln Met Leu Glu Gly Ala Arg Gly Leu Val Asn Gln Leu Ile Gln Phe
                   2020                2025                2030
   ggt tcc tct ctc ctt ggt tac tct gag agg caa gat gct gag gcc atg    6143
   Gly Ser Ser Leu Leu Gly Tyr Ser Glu Arg Gln Asp Ala Glu Ala Met
               2035                2040                2045
   tcc caa ctc ttg caa acc cag gct tct gag ttg atc ctc acc tcc atc    6191
   Ser Gln Leu Leu Gln Thr Gln Ala Ser Glu Leu Ile Leu Thr Ser Ile
           2050                2055                2060
   agg atg caa gac aac cag ctt gct gag ttg gac tct gag aag act gct    6239
   Arg Met Gln Asp Asn Gln Leu Ala Glu Leu Asp Ser Glu Lys Thr Ala
       2065                2070                2075
   ctc caa gtc tcc ctt gct ggt gtc caa cag agg ttc gac agc tac tcc    6287
   Leu Gln Val Ser Leu Ala Gly Val Gln Gln Arg Phe Asp Ser Tyr Ser
   2080                2085                2090                2095
   caa ctc tac gag gag aac atc aac gct ggt gag caa agg gct ttg gct    6335
   Gln Leu Tyr Glu Glu Asn Ile Asn Ala Gly Glu Gln Arg Ala Leu Ala
                   2100                2105                2110
   ctc agg tct gag tct gcc att gag tcc caa ggt gct caa atc tcc cgc    6383
   Leu Arg Ser Glu Ser Ala Ile Glu Ser Gln Gly Ala Gln Ile Ser Arg
               2115                2120                2125
   atg gct ggt gct ggc gtg gac atg gct cca aac atc ttc ggt ctt gct    6431
   Met Ala Gly Ala Gly Val Asp Met Ala Pro Asn Ile Phe Gly Leu Ala
           2130                2135                2140
   gat ggt ggc atg cac tac ggt gcc att gct tac gcc att gct gat ggc    6479
   Asp Gly Gly Met His Tyr Gly Ala Ile Ala Tyr Ala Ile Ala Asp Gly
       2145                2150                2155
   att gag ctt tct gct tct gcc aag atg gtt gat gct gag aag gtg gct    6527
   Ile Glu Leu Ser Ala Ser Ala Lys Met Val Asp Ala Glu Lys Val Ala
   2160                2165                2170                2175
   caa tct gaa atc tac cgt cgc aga cgc caa gaa tgg aag atc caa agg    6575
   Gln Ser Glu Ile Tyr Arg Arg Arg Arg Gln Glu Trp Lys Ile Gln Arg
                   2180                2185                2190
   gac aac gct caa gct gag atc aac cag ctc aac gct caa ctt gag tcc    6623
   Asp Asn Ala Gln Ala Glu Ile Asn Gln Leu Asn Ala Gln Leu Glu Ser
               2195                2200                2205
   ctc agc atc agg cgt gag gct gct gag atg cag aag gag tac ctc aag    6671
   Leu Ser Ile Arg Arg Glu Ala Ala Glu Met Gln Lys Glu Tyr Leu Lys
           2210                2215                2220
   acc caa cag gct caa gct cag gct caa ctc acc ttc ctc agg tcc aag    6719
   Thr Gln Gln Ala Gln Ala Gln Ala Gln Leu Thr Phe Leu Arg Ser Lys
       2225                2230                2235
   ttc tcc aac cag gct ctc tac tcc tgg ctc aga ggc cgc ctc tct ggc    6767
   Phe Ser Asn Gln Ala Leu Tyr Ser Trp Leu Arg Gly Arg Leu Ser Gly
   2240                2245                2250                2255
   atc tac ttc caa ttc tac gac ttg gct gtc tcc cgc tgc ctc atg gct    6815
   Ile Tyr Phe Gln Phe Tyr Asp Leu Ala Val Ser Arg Cys Leu Met Ala
                   2260                2265                2270
   gag caa tcc tac caa tgg gag gcc aac gac aac agc atc tcc ttc gtc    6863
   Glu Gln Ser Tyr Gln Trp Glu Ala Asn Asp Asn Ser Ile Ser Phe Val
               2275                2280                2285
   aag cca ggt gct tgg caa ggc acc tac gct ggt ctc ctt tgc ggt gag    6911
   Lys Pro Gly Ala Trp Gln Gly Thr Tyr Ala Gly Leu Leu Cys Gly Glu
           2290                2295                2300
   gct ctc atc cag aac ttg gct caa atg gag gag gct tac ctc aag tgg    6959
   Ala Leu Ile Gln Asn Leu Ala Gln Met Glu Glu Ala Tyr Leu Lys Trp
       2305                2310                2315
   gag tcc aga gct ttg gag gta gag agg act gtc tcc ctt gct gta gtc    7007
   Glu Ser Arg Ala Leu Glu Val Glu Arg Thr Val Ser Leu Ala Val Val
   2320                2325                2330                2335
   tac gac tcc ttg gag ggc aac gac agg ttc aac ctt gct gag caa atc    7055
   Tyr Asp Ser Leu Glu Gly Asn Asp Arg Phe Asn Leu Ala Glu Gln Ile
                   2340                2345                2350
   cca gct ctc ttg gac aag ggt gag ggc act gct ggc acc aag gag aac    7103
   Pro Ala Leu Leu Asp Lys Gly Glu Gly Thr Ala Gly Thr Lys Glu Asn
               2355                2360                2365
   ggt ctc tcc ttg gcc aac gcc atc ctc tct gct tct gtc aag ctc tct    7151
   Gly Leu Ser Leu Ala Asn Ala Ile Leu Ser Ala Ser Val Lys Leu Ser
             2370                2375                2380
   gac ctc aag ttg ggt act gac tac cca gac tcc att gtg ggt tcc aac      7199
   Asp Leu Lys Leu Gly Thr Asp Tyr Pro Asp Ser Ile Val Gly Ser Asn
       2385                2390                2395
   aag gtc aga agg atc aag caa atc tct gtc tcc ctc cca gct ttg gtg      7247
   Lys Val Arg Arg Ile Lys Gln Ile Ser Val Ser Leu Pro Ala Leu Val
   2400                2405                2410                2415
   ggt cca tac caa gat gtc caa gcc atg ctc tcc tac ggt ggc tcc acc      7295
   Gly Pro Tyr Gln Asp Val Gln Ala Met Leu Ser Tyr Gly Gly Ser Thr
                   2420                2425                2430
   caa ctc cca aag ggt tgc tct gct ttg gct gtc tcc cac ggc acc aac      7343
   Gln Leu Pro Lys Gly Cys Ser Ala Leu Ala Val Ser His Gly Thr Asn
               2435                2440                2445
   gac tct ggt caa ttc caa ctt gac ttc aac gat ggc aag tac ctc cca      7391
   Asp Ser Gly Gln Phe Gln Leu Asp Phe Asn Asp Gly Lys Tyr Leu Pro
           2450                2455                2460
   ttc gaa ggc att gct ttg gat gac caa ggc acc ctc aac ctc caa ttc      7439
   Phe Glu Gly Ile Ala Leu Asp Asp Gln Gly Thr Leu Asn Leu Gln Phe
       2465                2470                2475
   cca aac gcc act gac aag cag aag gcc atc ctc caa acc atg tct gac      7487
   Pro Asn Ala Thr Asp Lys Gln Lys Ala Ile Leu Gln Thr Met Ser Asp
   2480                2485                2490                2495
   atc atc ctc cac atc agg tac acc atc agg tgagctcgag aggcctgcgg        7537
   Ile Ile Leu His Ile Arg Tyr Thr Ile Arg
                   2500                2505
   ccgc                                                                 7541
<210> 5
   <211> 63
   <212> DNA
   <213> Artificial Sequence
<220>
   <223> Description of Artificial Sequence:hemicot sequence encoding ER signal from 15 kDa zein from Black Mexican Sweet maize
<220>
   <221> CDS
   <222> (1) .. (63)
<400> 5
   atg gct aag atg gtc att gtg ctt gtg gtc tgc ttg gct ctc tct gct    48
   Met Ala Lys Met Val Ile Val Leu Val Val Cys Leu Ala Leu Ser Ala
     1               5                  10                  15
   gcc tgt gct tca gcc                                                63
   Ala Cys Ala Ser Ala
                20
<210> 6
   <211> 7621
   <212> DNA
   <213> Artificial Sequence
<220>
   <223> Description of Artificial Sequence:hemicot tcdA fused to the modified 15 kDa zein endoplasmic reticulum signal peptide
<220>
   <221> CDS
   <222> (4)..(7614)
<400> 6
   ncc atg gct aag atg gtc att gtg ctt gtg gtc tgc ttg gct ctc tct    48
       Met Ala Lys Met Val Ile Val Leu Val Val Cys Leu Ala Leu Ser
        1               5                  10                  15
   gct gcc tgt gct tca gcc atg aac gag tcc gtc aag gag atc cca gac    96
   Ala Ala Cys Ala Ser Ala Met Asn Glu Ser Val Lys Glu Ile Pro Asp
                   20                  25                  30
   gtc ctc aag tcc caa tgc ggt ttc aac tgc ctc act gac atc tcc cac    144
   Val Leu Lys Ser Gln Cys Gly Phe Asn Cys Leu Thr Asp Ile Ser His
               35                  40                  45
   agc tcc ttc aac gag ttc aga caa caa gtc tct gag cac ctc tcc tgg    192
   Ser Ser Phe Asn Glu Phe Arg Gln Gln Val Ser Glu His Leu Ser Trp
           50                  55                  60
   tcc gag acc cat gac ctc tac cat gac gct cag caa gct cag aag gac    240
   Ser Glu Thr His Asp Leu Tyr His Asp Ala Gln Gln Ala Gln Lys Asp
       65                  70                  75
   aac agg ctc tac gag gct agg atc ctc aag agg gct aac cca caa ctc    288
   Asn Arg Leu Tyr Glu Ala Arg Ile Leu Lys Arg Ala Asn Pro Gln Leu
   80                  85                  90                  95
   cag aac gct gtc cac ctc gcc atc ttg gct cca aac gct gag ttg att    336
   Gln Asn Ala Val His Leu Ala Ile Leu Ala Pro Asn Ala Glu Leu Ile
                   100                 105                 110
   ggt tac aac aac cag ttc tct ggc aga gct agc cag tac gtg gct cct    384
   Gly Tyr Asn Asn Gln Phe Ser Gly Arg Ala Ser Gln Tyr Val Ala Pro
               115                 120                 125
   ggt aca gtc tcc tcc atg ttc agc cca gcc gct tac ctc act gag ttg    432
   Gly Thr Val Ser Ser Met Phe Ser Pro Ala Ala Tyr Leu Thr Glu Leu
           130                 135                 140
   tac cgc gag gct agg aac ctt cat gct tct gac tcc gtc tac tac ttg    480
   Tyr Arg Glu Ala Arg Asn Leu His Ala Ser Asp Ser Val Tyr Tyr Leu
       145                 150                 155
   gac aca cgc aga cca gac ctc aag agc atg gcc ctc agc caa cag aac    528
   Asp Thr Arg Arg Pro Asp Leu Lys Ser Met Ala Leu Ser Gln Gln Asn
   160                 165                 170                 175
   atg gac att gag ttg tcc acc ctc tcc ttg agc aac gag ctt ctc ttg    576
   Met Asp Ile Glu Leu Ser Thr Leu Ser Leu Ser Asn Glu Leu Leu Leu
                   180                 185                 190
   gag tcc atc aag act gag agc aag ttg gag aac tac acc aag gtc atg      624
   Glu Ser Ile Lys Thr Glu Ser Lys Leu Glu Asn Tyr Thr Lys Val Met
               195                 200                 205
   gag atg ctc tcc acc ttc aga cca agc ggt gca act cca tac cat gat      672
   Glu Met Leu Ser Thr Phe Arg Pro Ser Gly Ala Thr Pro Tyr His Asp
           210                 215                 220
   gcc tac gag aac gtc agg gag gtc atc caa ctt caa gac cct ggt ctt      720
   Ala Tyr Glu Asn Val Arg Glu Val Ile Gln Leu Gln Asp Pro Gly Leu
       225                 230                 235
   gag caa ctc aac gct tct cca gcc att gct ggt ttg atg cac cag gca      768
   Glu Gln Leu Asn Ala Ser Pro Ala Ile Ala Gly Leu Met His Gln Ala
   240                 245                 250                 255
   tcc ttg ctc ggt atc aac gcc tcc atc tct cct gag ttg ttc aac atc      816
   Ser Leu Leu Gly Ile Asn Ala Ser Ile Ser Pro Glu Leu Phe Asn Ile
                   260                 265                 270
   ttg act gag gag atc act gag ggc aac gct gag gag ttg tac aag aag      864
   Leu Thr Glu Glu Ile Thr Glu Gly Asn Ala Glu Glu Leu Tyr Lys Lys
               275                 280                 285
   aac ttc ggc aac att gag cca gcc tct ctt gca atg cct gag tac ctc      912
   Asn Phe Gly Asn Ile Glu Pro Ala Ser Leu Ala Met Pro Glu Tyr Leu
           290                 295                 300
   aag agg tac tac aac ttg tct gat gag gag ctt tct caa ttc att ggc      960
   Lys Arg Tyr Tyr Asn Leu Ser Asp Glu Glu Leu Ser Gln Phe Ile Gly
       305                 310                 315
   aag gct tcc aac ttc ggt caa cag gag tac agc aac aac cag ctc atc      1008
   Lys Ala Ser Asn Phe Gly Gln Gln Glu Tyr Ser Asn Asn Gln Leu Ile
   320                 325                 330                 335
   act cca gtt gtg aac tcc tct gat ggc act gtg aag gtc tac cgc atc      1056
   Thr Pro Val Val Asn Ser Ser Asp Gly Thr Val Lys Val Tyr Arg Ile
                   340                 345                 350
   aca cgt gag tac acc aca aac gcc tac caa atg gat gtt gag ttg ttc      1104
   Thr Arg Glu Tyr Thr Thr Asn Ala Tyr Gln Met Asp Val Glu Leu Phe
               355                 360                 365
   cca ttc ggt ggt gag aac tac aga ctt gac tac aag ttc aag aac ttc      1152
   Pro Phe Gly Gly Glu Asn Tyr Arg Leu Asp Tyr Lys Phe Lys Asn Phe
           370                 375                 380
   tac aac gcc tcc tac ctc tcc atc aag ttg aac gac aag agg gag ctt      1200
   Tyr Asn Ala Ser Tyr Leu Ser Ile Lys Leu Asn Asp Lys Arg Glu Leu
       385                 390                 395
   gtc agg act gag ggt gct cct caa gtg aac att gag tac tct gcc aac      1248
   Val Arg Thr Glu Gly Ala Pro Gln Val Asn Ile Glu Tyr Ser Ala Asn
   400                 405                 410                 415
   atc acc ctc aac aca gct gac atc tct caa cca ttc gag att ggt ttg      1296
   Ile Thr Leu Asn Thr Ala Asp Ile Ser Gln Pro Phe Glu Ile Gly Leu
                   420                 425                 430
   acc aga gtc ctt ccc tct ggc tcc tgg gcc tac gct gca gcc aag ttc      1344
   Thr Arg Val Leu Pro Ser Gly Ser Trp Ala Tyr Ala Ala Ala Lys Phe
               435                 440                 445
   act gtt gag gag tac aac cag tac tct ttc ctc ttg aag ctc aac aag      1392
   Thr Val Glu Glu Tyr Asn Gln Tyr Ser Phe Leu Leu Lys Leu Asn Lys
           450                 455                 460
   gca att cgt ctc agc aga gcc act gag ttg tct ccc acc atc ttg gag      1440
   Ala Ile Arg Leu Ser Arg Ala Thr Glu Leu Ser Pro Thr Ile Leu Glu
       465                 470                 475
   ggc att gtg agg tct gtc aac ctt caa ctt gac atc aac act gat gtg      1488
   Gly Ile Val Arg Ser Val Asn Leu Gln Leu Asp Ile Asn Thr Asp Val
   480                 485                 490                 495
   ctt ggc aag gtc ttc ctc acc aag tac tac atg caa cgc tac gcc atc      1536
   Leu Gly Lys Val Phe Leu Thr Lys Tyr Tyr Met Gln Arg Tyr Ala Ile
                   500                 505                 510
   cat gct gag act gca ctc atc ctc tgc aac gca ccc atc tct caa cgc      1584
   His Ala Glu Thr Ala Leu Ile Leu Cys Asn Ala Pro Ile Ser Gln Arg
               515                 520                 525
   tcc tac gac aac cag cct tcc cag ttc gac agg ctc ttc aac act cct      1632
   Ser Tyr Asp Asn Gln Pro Ser Gln Phe Asp Arg Leu Phe Asn Thr Pro
           530                 535                 540
   ctc ttg aac ggc cag tac ttc tcc act ggt gat gag gag att gac ctc      1680
   Leu Leu Asn Gly Gln Tyr Phe Ser Thr Gly Asp Glu Glu Ile Asp Leu
       545                 550                 555
   aac tct ggc tcc aca ggt gac tgg aga aag acc atc ttg aag agg gcc      1728
   Asn Ser Gly Ser Thr Gly Asp Trp Arg Lys Thr Ile Leu Lys Arg Ala
   560                 565                 570                 575
   ttc aac att gat gat gtc tct ctc ttc cgt ctc ttg aag atc aca gat      1776
   Phe Asn Ile Asp Asp Val Ser Leu Phe Arg Leu Leu Lys Ile Thr Asp
                   580                 585                 590
   cac gac aac aag gat ggc aag atc aag aac aac ttg aag aac ctt tcc      1824
   His Asp Asn Lys Asp Gly Lys Ile Lys Asn Asn Leu Lys Asn Leu Ser
               595                 600                 605
   aac ctc tac att ggc aag ttg ctt gca gac atc cac caa ctc acc att      1872
   Asn Leu Tyr Ile Gly Lys Leu Leu Ala Asp Ile His Gln Leu Thr Ile
           610                 615                 620
   gat gag ttg gac ctc ttg ctc att gca gtc ggt gag ggc aag acc aac      1920
   Asp Glu Leu Asp Leu Leu Leu Ile Ala Val Gly Glu Gly Lys Thr Asn
       625                 630                 635
   ctc tct gca atc tct gac aag cag ttg gca acc ctc atc agg aag ttg      1968
   Leu Ser Ala Ile Ser Asp Lys Gln Leu Ala Thr Leu Ile Arg Lys Leu
   640                 645                 650                 655
   aac acc atc acc tcc tgg ctt cac acc cag aag tgg tct gtc ttc caa      2016
   Asn Thr Ile Thr Ser Trp Leu His Thr Gln Lys Trp Ser Val Phe Gln
                   660                 665                 670
   ctc ttc atc atg acc agc acc tcc tac aac aag acc ctc act cct gag     2064
   Leu Phe Ile Met Thr Ser Thr Ser Tyr Asn Lys Thr Leu Thr Pro Glu
               675                 680                 685
   atc aag aac ctc ttg gac aca gtc tac cac ggt ctc caa ggc ttc gac     2112
   Ile Lys Asn Leu Leu Asp Thr Val Tyr His Gly Leu Gln Gly Phe Asp
           690                 695                 700
   aag gac aag gct gac ttg ctt cat gtc atg gct ccc tac att gca gcc     2160
   Lys Asp Lys Ala Asp Leu Leu His Val Met Ala Pro Tyr Ile Ala Ala
       705                 710                 715
   acc ctc caa ctc tcc tct gag aac gtg gct cac tct gtc ttg ctc tgg     2208
   Thr Leu Gln Leu Ser Ser Glu Asn Val Ala His Ser Val Leu Leu Trp
   720                 725                 730                 735
   gct gac aag ctc caa cct ggt gat ggt gcc atg act gct gag aag ttc     2256
   Ala Asp Lys Leu Gln Pro Gly Asp Gly Ala Met Thr Ala Glu Lys Phe
                   740                 745                 750
   tgg gac tgg ctc aac acc aag tac aca cca ggc tcc tct gag gct gtt     2304
   Trp Asp Trp Leu Asn Thr Lys Tyr Thr Pro Gly Ser Ser Glu Ala Val
               755                 760                 765
   gag act caa gag cac att gtg caa tac tgc cag gct ctt gca cag ttg     2352
   Glu Thr Gln Glu His Ile Val Gln Tyr Cys Gln Ala Leu Ala Gln Leu
           770                 775                 780
   gag atg gtc tac cac tcc act ggc atc aac gag aac gct ttc aga ctc     2400
   Glu Met Val Tyr His Ser Thr Gly Ile Asn Glu Asn Ala Phe Arg Leu
       785                 790                 795
   ttc gtc acc aag cct gag atg ttc ggt gct gcc aca ggt gct gca cct     2448
   Phe Val Thr Lys Pro Glu Met Phe Gly Ala Ala Thr Gly Ala Ala Pro
   800                 805                 810                 815
   gct cat gat gct ctc tcc ctc atc atg ttg acc agg ttc gct gac tgg     2496
   Ala His Asp Ala Leu Ser Leu Ile Met Leu Thr Arg Phe Ala Asp Trp
                   820                 825                 830
   gtc aac gct ctt ggt gag aag gct tcc tct gtc ttg gct gcc ttc gag     2544
   Val Asn Ala Leu Gly Glu Lys Ala Ser Ser Val Leu Ala Ala Phe Glu
               835                 840                 845
   gcc aac tcc ctc act gct gag caa ctt gct gat gcc atg aac ctt gat     2592
   Ala Asn Ser Leu Thr Ala Glu Gln Leu Ala Asp Ala Met Asn Leu Asp
           850                 855                 860
   gcc aac ctc ttg ctc caa gct tcc att caa gct cag aac cac caa cac     2640
   Ala Asn Leu Leu Leu Gln Ala Ser Ile Gln Ala Gln Asn His Gln His
       865                 870                 875
   ctc cca cct gtc act cca gag aac gct ttc tcc tgc tgg acc tcc atc     2688
   Leu Pro Pro Val Thr Pro Glu Asn Ala Phe Ser Cys Trp Thr Ser Ile
   880                 885                 890                 895
   aac acc atc ctc caa tgg gtc aac gtg gct cag caa ctc aac gtg gct     2736
   Asn Thr Ile Leu Gln Trp Val Asn Val Ala Gln Gln Leu Asn Val Ala
                   900                 905                 910
   cca caa ggt gtc tct gct ttg gtc ggt ctt gac tac atc cag tcc atg    2784
   Pro Gln Gly Val Ser Ala Leu Val Gly Leu Asp Tyr Ile Gln Ser Met
               915                 920                 925
   aag gag aca cca acc tac gct caa tgg gag aac gca gct ggt gtc ttg    2832
   Lys Glu Thr Pro Thr Tyr Ala Gln Trp Glu Asn Ala Ala Gly Val Leu
           930                 935                 940
   act gct ggt ctc aac tcc caa cag gcc aac acc ctc cat gct ttc ttg    2880
   Thr Ala Gly Leu Asn Ser Gln Gln Ala Asn Thr Leu His Ala Phe Leu
       945                 950                 955
   gat gag tct cgc tct gct gcc ctc tcc acc tac tac atc agg caa gtc    2928
   Asp Glu Ser Arg Ser Ala Ala Leu Ser Thr Tyr Tyr Ile Arg Gln Val
   960                 965                 970                 975
   gcc aag gca gct gct gcc atc aag tct cgc gat gac ctc tac caa tac    2976
   Ala Lys Ala Ala Ala Ala Ile Lys Ser Arg Asp Asp Leu Tyr Gln Tyr
                   980                 985                 990
   ctc ctc att gac aac cag gtc tct gct gcc atc aag acc acc agg atc    3024
   Leu Leu Ile Asp Asn Gln Val Ser Ala Ala Ile Lys Thr Thr Arg Ile
               995                 1000                1005
   gct gag gcc atc gct tcc atc caa ctc tac gtc aac cgc gct ctt gag    3072
   Ala Glu Ala Ile Ala Ser Ile Gln Leu Tyr Val Asn Arg Ala Leu Glu
           1010                1015                1020
   aac gtt gag gag aac gcc aac tct ggt gtc atc tct cgc caa ttc ttc    3120
   Asn Val Glu Glu Asn Ala Asn Ser Gly Val Ile Ser Arg Gln Phe Phe
       1025                1030                1035
   atc gac tgg gac aag tac aac aag agg tac tcc acc tgg gct ggt gtc    3168
   Ile Asp Trp Asp Lys Tyr Asn Lys Arg Tyr Ser Thr Trp Ala Gly Val
   1040                1045                1050                1055
   tct caa ctt gtc tac tac cca gag aac tac att gac cca acc atg agg    3216
   Ser Gln Leu Val Tyr Tyr Pro Glu Asn Tyr Ile Asp Pro Thr Met Arg
                   1060                1065                1070
   att ggt cag acc aag atg atg gat gct ctc ttg caa tct gtc tcc caa    3264
   Ile Gly Gln Thr Lys Met Met Asp Ala Leu Leu Gln Ser Val Ser Gln
               1075                1080                1085
   agc caa ctc aac gct gac act gtg gag gat gcc ttc atg agc tac ctc    3312
   Ser Gln Leu Asn Ala Asp Thr Val Glu Asp Ala Phe Met Ser Tyr Leu
           1090                1095                1100
   acc tcc ttc gag caa gtt gcc aac ctc aag gtc atc tct gct tac cat    3360
   Thr Ser Phe Glu Gln Val Ala Asn Leu Lys Val Ile Ser Ala Tyr His
       1105                1110                1115
   gac aac atc aac aac gac caa ggt ctc acc tac ttc att ggt ctc tct    3408
   Asp Asn Ile Asn Asn Asp Gln Gly Leu Thr Tyr Phe Ile Gly Leu Ser
   1120                1125                1130                1135
   gag act gat gct ggt gag tac tac tgg aga tcc gtg gac cac agc aag    3456
   Glu Thr Asp Ala Gly Glu Tyr Tyr Trp Arg Ser Val Asp His Ser Lys
                   1140                1145                1150
   ttc aac gat ggc aag ttc gct gca aac gct tgg tct gag tgg cac aag    3504
   Phe Asn Asp Gly Lys Phe Ala Ala Asn Ala Trp Ser Glu Trp His Lys
               1155                1160                1165
   att gac tgc cct atc aac cca tac aag tcc acc atc aga cct gtc atc      3552
   Ile Asp Cys Pro Ile Asn Pro Tyr Lys Ser Thr Ile Arg Pro Val Ile
           1170                1175                1180
   tac aag agc cgc ctc tac ttg ctc tgg ctt gag cag aag gag atc acc      3600
   Tyr Lys Ser Arg Leu Tyr Leu Leu Trp Leu Glu Gln Lys Glu Ile Thr
       1185                1190                1195
   aag caa act ggc aac tcc aag gat ggt tac caa act gag act gac tac      3648
   Lys Gln Thr Gly Asn Ser Lys Asp Gly Tyr Gln Thr Glu Thr Asp Tyr
   1200                1205                1210                1215
   cgc tac gag ttg aag ttg gct cac atc cgc tac gat ggt acc tgg aac      3696
   Arg Tyr Glu Leu Lys Leu Ala His Ile Arg Tyr Asp Gly Thr Trp Asn
                   1220                1225                1230
   act cca atc acc ttc gat gtc aac aag aag atc agc gag ttg aag ttg      3744
   Thr Pro Ile Thr Phe Asp Val Asn Lys Lys Ile Ser Glu Leu Lys Leu
               1235                1240                1245
   gag aag aac cgt gct cct ggt ctc tac tgc gct ggt tac caa ggt gag      3792
   Glu Lys Asn Arg Ala Pro Gly Leu Tyr Cys Ala Gly Tyr Gln Gly Glu
           1250                1255                1260
   gac acc ctc ttg gtc atg ttc tac aac cag caa gac acc ctt gac tcc      3840
   Asp Thr Leu Leu Val Met Phe Tyr Asn Gln Gln Asp Thr Leu Asp Ser
       1265                1270                1275
   tac aag aac gct tcc atg caa ggt ctc tac atc ttc gct gac atg gct      3888
   Tyr Lys Asn Ala Ser Met Gln Gly Leu Tyr Ile Phe Ala Asp Met Ala
   1280                1285                1290                1295
   tcc aag gac atg act cca gag caa agc aac gtc tac cgt gac aac tcc      3936
   Ser Lys Asp Met Thr Pro Glu Gln Ser Asn Val Tyr Arg Asp Asn Ser
                   1300                1305                1310
   tac caa cag ttc gac acc aac aac gtc agg cgt gtc aac aac aga tac      3984
   Tyr Gln Gln Phe Asp Thr Asn Asn Val Arg Arg Val Asn Asn Arg Tyr
               1315                1320                1325
   gct gag gac tac gag atc cca agc tct gtc agc tct cgc aag gac tac      4032
   Ala Glu Asp Tyr Glu Ile Pro Ser Ser Val Ser Ser Arg Lys Asp Tyr
           1330                1335                1340
   ggc tgg ggt gac tac tac ctc agc atg gtg tac aac ggt gac atc cca      4080
   Gly Trp Gly Asp Tyr Tyr Leu Ser Met Val Tyr Asn Gly Asp Ile Pro
       1345                1350                1355
   acc atc aac tac aag gct gcc tct tcc gac ctc aaa atc tac atc agc      4128
   Thr Ile Asn Tyr Lys Ala Ala Ser Ser Asp Leu Lys Ile Tyr Ile Ser
   1360                1365                1370                1375
   cca aag ctc agg atc atc cac aac ggc tac gag ggt cag aag agg aac      4176
   Pro Lys Leu Arg Ile Ile His Asn Gly Tyr Glu Gly Gln Lys Arg Asn
                   1380                1385                1390
   cag tgc aac ttg atg aac aag tac ggc aag ttg ggt gac aag ttc att      4224
   Gln Cys Asn Leu Met Asn Lys Tyr Gly Lys Leu Gly Asp Lys Phe Ile
               1395                1400                1405
   gtc tac acc tct ctt ggt gtc aac cca aac aac agc tcc aac aag ctc      4272
   Val Tyr Thr Ser Leu Gly Val Asn Pro Asn Asn Ser Ser Asn Lys Leu
           1410                1415                1420
   atg ttc tac cca gtc tac caa tac tct ggc aac acc tct ggt ctc aac      4320
   Met Phe Tyr Pro Val Tyr Gln Tyr Ser Gly Asn Thr Ser Gly Leu Asn
       1425                1430                1435
   cag ggt aga ctc ttg ttc cac agg gac acc acc tac cca agc aag gtg      4368
   Gln Gly Arg Leu Leu Phe His Arg Asp Thr Thr Tyr Pro Ser Lys Val
   1440                1445                1450                1455
   gag gct tgg att cct ggt gcc aag agg tcc ctc acc aac cag aac gct      4416
   Glu Ala Trp Ile Pro Gly Ala Lys Arg Ser Leu Thr Asn Gln Asn Ala
                   1460                1465                1470
   gcc att ggt gat gac tac gcc aca gac tcc ctc aac aag cct gat gac      4464
   Ala Ile Gly Asp Asp Tyr Ala Thr Asp Ser Leu Asn Lys Pro Asp Asp
               1475                1480                1485
   ctc aag cag tac atc ttc atg act gac tcc aag ggc aca gcc act gat      4512
   Leu Lys Gln Tyr Ile Phe Met Thr Asp Ser Lys Gly Thr Ala Thr Asp
           1490                1495                1500
   gtc tct ggt cca gtg gag atc aac act gca atc agc cca gcc aag gtc      4560
   Val Ser Gly Pro Val Glu Ile Asn Thr Ala Ile Ser Pro Ala Lys Val
       1505                1510                1515
   caa atc att gtc aag gct ggt ggc aag gag caa acc ttc aca gct gac      4608
   Gln Ile Ile Val Lys Ala Gly Gly Lys Glu Gln Thr Phe Thr Ala Asp
   1520                1525                1530                1535
   aag gat gtc tcc atc cag cca agc cca tcc ttc gat gag atg aac tac      4656
   Lys Asp Val Ser Ile Gln Pro Ser Pro Ser Phe Asp Glu Met Asn Tyr
                   1540                1545                1550
   caa ttc aac gct ctt gag att gat ggt tct ggc ctc aac ttc atc aac      4704
   Gln Phe Asn Ala Leu Glu Ile Asp Gly Ser Gly Leu Asn Phe Ile Asn
               1555                1560                1565
   aac tct gct tcc att gat gtc acc ttc act gcc ttc gct gag gat ggc      4752
   Asn Ser Ala Ser Ile Asp Val Thr Phe Thr Ala Phe Ala Glu Asp Gly
           1570                1575                1580
   cgc aag ttg ggt tac gag agc ttc tcc atc cca gtc acc ctt aag gtt      4800
   Arg Lys Leu Gly Tyr Glu Ser Phe Ser Ile Pro Val Thr Leu Lys Val
       1585                1590                1595
   tcc act gac aac gca ctc acc ctt cat cac aac gag aac ggt gct cag      4848
   Ser Thr Asp Asn Ala Leu Thr Leu His His Asn Glu Asn Gly Ala Gln
   1600                1605                1610                1615
   tac atg caa tgg caa agc tac cgc acc agg ttg aac acc ctc ttc gca      4896
   Tyr Met Gln Trp Gln Ser Tyr Arg Thr Arg Leu Asn Thr Leu Phe Ala
                   1620                1625                1630
   agg caa ctt gtg gcc cgt gcc acc aca ggc att gac acc atc ctc agc      4944
   Arg Gln Leu Val Ala Arg Ala Thr Thr Gly Ile Asp Thr Ile Leu Ser
               1635                1640                1645
   atg gag acc cag aac atc caa gag cca cag ttg ggc aag ggt ttc tac     4992
   Met Glu Thr Gln Asn Ile Gln Glu Pro Gln Leu Gly Lys Gly Phe Tyr
                   1650                1655                1660
   gcc acc ttc gtc atc cca cct tac aac ctc agc act cat ggt gat gag     5040
   Ala Thr Phe Val Ile Pro Pro Tyr Asn Leu Ser Thr His Gly Asp Glu
           1665                1670                1675
   agg tgg ttc aag ctc tac atc aag cac gtg gtt gac aac aac tcc cac     5088
   Arg Trp Phe Lys Leu Tyr Ile Lys His Val Val Asp Asn Asn Ser His
   1680                1685                1690                1695
   atc atc tac tct ggt caa ctc act gac acc aac atc aac atc acc ctc     5136
   Ile Ile Tyr Ser Gly Gln Leu Thr Asp Thr Asn Ile Asn Ile Thr Leu
                       1700                1705                1710
   ttc atc cca ctt gac gat gtc cca ctc aac cag gac tac cat gcc aag     5184
   Phe Ile Pro Leu Asp Asp Val Pro Leu Asn Gln Asp Tyr His Ala Lys
               1715                1720                1725
   gtc tac atg acc ttc aag aag tct cca tct gat ggc acc tgg tgg ggt     5232
   Val Tyr Met Thr Phe Lys Lys Ser Pro Ser Asp Gly Thr Trp Trp Gly
           1730                1735                1740
   cca cac ttc gtc cgt gat gac aag ggc atc gtc acc atc aac cca aag     5280
   Pro His Phe Val Arg Asp Asp Lys Gly Ile Val Thr Ile Asn Pro Lys
       1745                1750                1755
   tcc atc ctc acc cac ttc gag tct gtc aac gtt ctc aac aac atc tcc     5328
   Ser Ile Leu Thr His Phe Glu Ser Val Asn Val Leu Asn Asn Ile Ser
   1760                1765                1770                1775
   tct gag cca atg gac ttc tct ggt gcc aac tcc ctc tac ttc tgg gag     5376
   Ser Glu Pro Met Asp Phe Ser Gly Ala Asn Ser Leu Tyr Phe Trp Glu
                       1780                1785                1790
   ttg ttc tac tac aca cca atg ctt gtg gct caa agg ttg ctc cat gag     5424
   Leu Phe Tyr Tyr Thr Pro Met Leu Val Ala Gln Arg Leu Leu His Glu
               1795                1800                1805
   cag aac ttc gat gag gcc aac agg tgg ctc aag tac gtc tgg agc cca     5472
   Gln Asn Phe Asp Glu Ala Asn Arg Trp Leu Lys Tyr Val Trp Ser Pro
           1810                1815                1820
   tct ggt tac att gtg cat ggt caa atc cag aac tac caa tgg aac gtc     5520
   Ser Gly Tyr Ile Val His Gly Gln Ile Gln Asn Tyr Gln Trp Asn Val
       1825                1830                1835
   agg cca ttg ctt gag gac acc tcc tgg aac tct gac cca ctt gac tct     5568
   Arg Pro Leu Leu Glu Asp Thr Ser Trp Asn Ser Asp Pro Leu Asp Ser
   1840                1845                1850                1855
   gtg gac cct gat gct gtg gct caa cat gac cca atg cac tac aag gtc     5616
   Val Asp Pro Asp Ala Val Ala Gln His Asp Pro Met His Tyr Lys Val
                       1860                1865                1870
   tcc acc ttc atg agg acc ttg gac ctc ttg att gcc aga ggt gac cat     5664
   Ser Thr Phe Met Arg Thr Leu Asp Leu Leu Ile Ala Arg Gly Asp His
               1875                1880                1885
   gct tac cgc caa ttg gag agg gac acc ctc aac gag gca aag atg tgg    5712
   Ala Tyr Arg Gln Leu Glu Arg Asp Thr Leu Asn Glu Ala Lys Met Trp
        1890                1895                1900
   tac atg caa gct ctc cac ctc ttg ggt gac aag cca tac ctc cca ctc    5760
   Tyr Met Gln Ala Leu His Leu Leu Gly Asp Lys Pro Tyr Leu Pro Leu
     1905                1910                1915
   agc acc act tgg tcc gac cca agg ttg gac cgt gct gct gac atc acc    5808
   Ser Thr Thr Trp Ser Asp Pro Arg Leu Asp Arg Ala Ala Asp Ile Thr
   1920                1925                1930                1935
   act cag aac gct cat gac tct gcc att gtt gct ctc agg cag aac atc    5856
   Thr Gln Asn Ala His Asp Ser Ala Ile Val Ala Leu Arg Gln Asn Ile
                   1940                1945                1950
   cca act cct gct cca ctc tcc ctc aga tct gct aac acc ctc act gac    5904
   Pro Thr Pro Ala Pro Leu Ser Leu Arg Ser Ala Asn Thr Leu Thr Asp
               1955                1960                1965
   ttg ttc ctc cca cag atc aac gag gtc atg atg aac tac tgg caa acc    5952
   Leu Phe Leu Pro Gln Ile Asn Glu Val Met Met Asn Tyr Trp Gln Thr
        1970                1975                1980
   ttg gct caa agg gtc tac aac ctc aga cac aac ctc tcc att gat ggt    6000
   Leu Ala Gln Arg Val Tyr Asn Leu Arg His Asn Leu Ser Ile Asp Gly
     1985                1990                1995
   caa cca ctc tac ctc cca atc tac gcc aca cca gct gac cca aag gct    6048
   Gln Pro Leu Tyr Leu Pro Ile Tyr Ala Thr Pro Ala Asp Pro Lys Ala
   2000                2005                2010                2015
   ctt ctc tct gct gct gtg gct acc agc caa ggt ggt ggc aag ctc cca    6096
   Leu Leu Ser Ala Ala Val Ala Thr Ser Gln Gly Gly Gly Lys Leu Pro
                   2020                2025                2030
   gag tcc ttc atg tcc ctc tgg agg ttc cca cac atg ttg gag aac gcc    6144
   Glu Ser Phe Met Ser Leu Trp Arg Phe Pro His Met Leu Glu Asn Ala
               2035                2040                2045
   cgt ggc atg gtc tcc caa ctc acc cag ttc ggt tcc acc ctc cag aac    6192
   Arg Gly Met Val Ser Gln Leu Thr Gln Phe Gly Ser Thr Leu Gln Asn
        2050                2055                2060
   atc att gag agg caa gat gct gag gct ctc aac gct ttg ctc cag aac    6240
   Ile Ile Glu Arg Gln Asp Ala Glu Ala Leu Asn Ala Leu Leu Gln Asn
     2065                2070                2075
   cag gca gct gag ttg atc ctc acc aac ttg tcc atc caa gac aag acc    6288
   Gln Ala Ala Glu Leu Ile Leu Thr Asn Leu Ser Ile Gln Asp Lys Thr
   2080                2085                2090                2095
   att gag gag ctt gat gct gag aag aca gtc ctt gag aag agc aag gct    6336
   Ile Glu Glu Leu Asp Ala Glu Lys Thr Val Leu Glu Lys Ser Lys Ala
                   2100                2105                2110
   ggt gcc caa tct cgc ttc gac tcc tac ggc aag ctc tac gat gag aac    6384
   Gly Ala Gln Ser Arg Phe Asp Ser Tyr Gly Lys Leu Tyr Asp Glu Asn
               2115                2120                2125
   atc aac gct ggt gag aac cag gcc atg acc ctc agg gct tcc gca gct    6432
   Ile Asn Ala Gly Glu Asn Gln Ala Met Thr Leu Arg Ala Ser Ala Ala
         2130                2135                2140
   ggt ctc acc act gct gtc caa gcc tct cgc ttg gct ggt gca gct gct     6480
   Gly Leu Thr Thr Ala Val Gln Ala Ser Arg Leu Ala Gly Ala Ala Ala
      2145                2150                2155
   gac ctc gtt cca aac atc ttc ggt ttc gct ggt ggt ggc tcc aga tgg     6528
   Asp Leu Val Pro Asn Ile Phe Gly Phe Ala Gly Gly Gly Ser Arg Trp
   2160                2165                2170                2175
   ggt gcc att gct gag gct acc ggt tac gtc atg gag ttc tct gcc aac     6576
   Gly Ala Ile Ala Glu Ala Thr Gly Tyr Val Met Glu Phe Ser Ala Asn
                   2180                2185                2190
   gtc atg aac act gag gct gac aag atc agc caa tct gag acc tac aga     6624
   Val Met Asn Thr Glu Ala Asp Lys Ile Ser Gln Ser Glu Thr Tyr Arg
               2195                2200                2205
   agg cgc cgt caa gag tgg gag atc caa agg aac aac gct gag gca gag     6672
   Arg Arg Arg Gln Glu Trp Glu Ile Gln Arg Asn Asn Ala Glu Ala Glu
         2210                2215                2220
   ttg aag caa atc gat gct caa ctc aag tcc ttg gct gtc aga agg gag     6720
   Leu Lys Gln Ile Asp Ala Gln Leu Lys Ser Leu Ala Val Arg Arg Glu
      2225                2230                2235
   gct gct gtc ctc cag aag acc tcc ctc aag acc caa cag gag caa acc     6768
   Ala Ala Val Leu Gln Lys Thr Ser Leu Lys Thr Gln Gln Glu Gln Thr
   2240                2245                2250                2255
   cag tcc cag ttg gct ttc ctc caa agg aag ttc tcc aac cag gct ctc     6816
   Gln Ser Gln Leu Ala Phe Leu Gln Arg Lys Phe Ser Asn Gln Ala Leu
                   2260                2265                2270
   tac aac tgg ctc aga ggc cgc ttg gct gcc atc tac ttc caa ttc tac     6864
   Tyr Asn Trp Leu Arg Gly Arg Leu Ala Ala Ile Tyr Phe Gln Phe Tyr
               2275                2280                2285
   gac ctt gct gtg gcc agg tgc ctc atg gct gag caa gcc tac cgc tgg     6912
   Asp Leu Ala Val Ala Arg Cys Leu Met Ala Glu Gln Ala Tyr Arg Trp
         2290                2295                2300
   gag ttg aac gat gac tcc gcc agg ttc atc aag cca ggt gct tgg caa     6960
   Glu Leu Asn Asp Asp Ser Ala Arg Phe Ile Lys Pro Gly Ala Trp Gln
      2305                2310                2315
   ggc acc tac gct ggt ctc ctt gct ggt gag acc ctc atg ctc tcc ttg     7008
   Gly Thr Tyr Ala Gly Leu Leu Ala Gly Glu Thr Leu Met Leu Ser Leu
   2320                2325                2330                2335
   gct caa atg gag gat gct cac ctc aag agg gac aag agg gct ttg gag     7056
   Ala Gln Met Glu Asp Ala His Leu Lys Arg Asp Lys Arg Ala Leu Glu
                   2340                2345                2350
   gtg gag agg aca gtc tcc ctt gct gag gtc tac gct ggt ctc cca aag     7104
   Val Glu Arg Thr Val Ser Leu Ala Glu Val Tyr Ala Gly Leu Pro Lys
               2355                2360                2365
   gac aac ggt cca ttc tcc ctt gct caa gag att gac aag ttg gtc agc     7152
   Asp Asn Gly Pro Phe Ser Leu Ala Gln Glu Ile Asp Lys Leu Val Ser
           2370                2375                2380
   caa ggt tct ggt tct gct ggt tct ggt aac aac aac ttg gct ttc ggc   7200
   Gln Gly Ser Gly Ser Ala Gly Ser Gly Asn Asn Asn Leu Ala Phe Gly
       2385                2390                2395
   gct ggt act gac acc aag acc tcc ctc caa gcc tct gtc tcc ttc gct   7248
   Ala Gly Thr Asp Thr Lys Thr Ser Leu Gln Ala Ser Val Ser Phe Ala
   2400                2405                2410                2415
   gac ctc aag atc agg gag gac tac cca gct tcc ctt ggc aag atc agg   7296
   Asp Leu Lys Ile Arg Glu Asp Tyr Pro Ala Ser Leu Gly Lys Ile Arg
                   2420                2425                2430
   cgc atc aag caa atc tct gtc acc ctc cca gct ctc ttg ggt cca tac   7344
   Arg Ile Lys Gln Ile Ser Val Thr Leu Pro Ala Leu Leu Gly Pro Tyr
               2435                2440                2445
   caa gat gtc caa gca atc ctc tcc tac ggt gac aag gct ggt ttg gcg   7392
   Gln Asp Val Gln Ala Ile Leu Ser Tyr Gly Asp Lys Ala Gly Leu Ala
           2450                2455                2460
   aac ggt tgc gag gct ctt gct gtc tct cat ggc atg aac gac tct ggt   7440
   Asn Gly Cys Glu Ala Leu Ala Val Ser His Gly Met Asn Asp Ser Gly
       2465                2470                2475
   caa ttc caa ctt gac ttc aac gat ggc aag ttc ctc cca ttc gag ggc   7488
   Gln Phe Gln Leu Asp Phe Asn Asp Gly Lys Phe Leu Pro Phe Glu Gly
   2480                2485                2490                2495
   att gcc att gac caa ggc acc ctc acc ctc tcc ttc cca aac gct tcc   7536
   Ile Ala Ile Asp Gln Gly Thr Leu Thr Leu Ser Phe Pro Asn Ala Ser
                   2500                2505                2510
   atg cca gag aag gga aag caa gcc acc atg ctc aag acc ctc aac gat   7584
   Met Pro Glu Lys Gly Lys Gln Ala Thr Met Leu Lys Thr Leu Asn Asp
               2515                2520                2525
   atc atc ctc cac atc agg tac acc atc aag tgagctc                   7621
   Ile Ile Leu His Ile Arg Tyr Thr Ile Lys
           2530                2535

## Description

### BACKGROUND OF THE INVENTION

As reported in WO98/08932, protein toxins from the genus *Photorhabdus* have been shown to have oral toxicity against insects. The toxin complex produced by *Photorhabdus luminescens* (W-14), for example, has been shown to contain ten to fourteen proteins, and it is known that these are produced by expression of genes from four distinct genomic regions: *tca, tcb, tcc,* and *tcd.* WO98/08932 discloses nucleotide sequences for the native toxin genes.

Of the separate toxins isolated from *Photorhabdus luminescens* (W-14), those designated Toxin A and Toxin B are especially potent against target insect species of interest, for example corn rootworm. Toxin A is comprised of two different subunits. The native gene tcdA (SEQ ID NO:1) encodes protoxin TcdA (see SEQ ID NO:1). As determined by mass spectrometry, TcdA is processed by one or more proteases to provide Toxin A. More specifically, TcdA is an approximately 282.9 kDA protein (2516 aa) that is processed to provide TcdAii, an approximately 208.2 kDA (1849 aa) protein encoded by nucleotides 265-5811 of SEQ ID NO:1, and TcdAiii, an approximately 63.5 kDA (579 aa) protein encoded by nucleotides 5812-7551 of SEQ ID NO:1.

Toxin B is similarly comprised of two different subunits. The native gene *tcbA* (SEQ ID NO:2) encodes protoxin TcbA (see SEQ ID NO:2). As determined by mass spectrometry, TcbA is processed by one or more proteases to provide Toxin B. More specifically, TcbA is an approximately 280.6 kDA (2504 aa) protein that is processed to provide TcbAii, an approximately 207.7 kDA (1844 aa) protein encoded by nucleotides 262-5793 of SEQ ID NO:2 and TcbAiii, an approximately 62.9 kDA (573 aa) protein encoded by nucleotides 5794-7512 of SEQ ID NO:2. The native *tcdA* and *tcbA* genes are not well suited for high level expression in plants. They encode multiple destabilization sequences, mRNA splice sites, polyA addition sites and other possibly detrimental sequence motifs. In addition, the codon compositions are not like those of plant genes. W098/08932 gives general guidance on how the toxin genes could be reengineered to more efficiently expressed in the cytoplasm of plants, and describes how plants can be transformed to incorporate the *Photorhabdus* toxin genes into their genomes.

WO97/13402 discloses synthetic DNA sequences which are optimized for expression in plants, particularly maize, and which encode a *Bacillus thuringiensis* protein that is toxic to specific insects, along with methods for the engineering of any synthetic insecticidal gene in maize.

### SUMMARY OF THE INVENTION

In a first aspect of the present invention, there is provided an isolated nucleic acid of SEQ ID No. 3. In second and third aspects of the present invention, there is provided a transgenic monocot cell and a transgenic dicot cell having a genome comprising SEQ ID No. 3. In a. fourth aspect of the present invention, there is provided a transgenic plant with a genome comprising a nucleic acid of SEQ ID No. 3 that imparts insect resistance. Preferably the genome in the cell or plant further comprises SEQ ID No. 4. The plant is preferably rice, maize or tobacco.

In a preferred embodiment, the invention provides novel polynucleotide sequences that encode TcdA. The specification also discloses polynucleotide sequences that encode TcbA. The novel sequences have base compositions that differ substantially from the native genes, making them more similar to plant genes. The new sequences are suitable for use for high expression in both monocots and dicots, and this feature is designated by referring to the sequences as the "hemicot" criteria, which is set forth in detail hereinafter. Other important features of the sequences are that potentially deleterious sequences have been eliminated, and unique restriction sites have been built in to enable adding or changing expression elements, organellar targeting signals, engineered protease sites and the like, if desired.

In a particularly preferred embodiment, the invention provides polynucleotide sequences that satisfy hemicot criteria and that comprise a sequence encoding an endoplasmic reticulum signal or similar targeting sequence for a cellular organelle in combination with a sequence encoding TcdA

The invention also provides transgenic plants with genomes comprising a novel sequence of claim 1 that imparts functional activity against insects.

### BRIEF DESCRIPTION OF SEQUENCES

SEQ ID NO: 1 is the native *tcdA* DNA sequence together with the corresponding encoded amino acid sequence for TcdA.
SEQ ID NO: 2 is the native *tcbA* DNA sequence together with the corresponding encoded amino acid sequence for TcbA.
SEQ ID NO: 3 is an artificial sequence encoding TcdA that is suitable for expression in monocot and dicot plants.
SEQ ID NO: 4 is an artificial sequence encoding TcbA that is suitable for expression in monocot and dicot plants.
SEQ ID NO: 5 is an artificial hemicot sequence that encodes the 21 amino acid ER signal peptide of 15 kDa zein from Black Mexican Sweet maize.
SEQ ID NO: 6 is an artificial hemicot sequence that encodes for the full-length native TcdA protein (amino acids 22-2537) fused to the modified 15 kDa zein endoplasmic reticulum signal peptide (amino acids 1-21).

### DETAILED DESCRIPTION

The native *Photorhabdus* toxins are protein complexes that are produced and secreted by growing bacteria cells of the genus *Photorhabdus.* Of particular interest are the proteins produced by the species *Photorhabdus luminescens.* The protein complexes have a molecular size of approximately 1,000 kDa and can be separated by SDS-PAGE gel analysis into numerous component proteins. The toxins contain no hemolysin, lipase, type C phospholipase, or nuclease activities. The toxins exhibit significant toxicity upon ingestion by a number of insects.

A unique feature of *Photorhabdus* is its bioluminescence. *Photorhabdus* may be isolated from a variety of sources. One such source is nematodes, more particularly nematodes of the genus *Heterorhabditis.* Another such source is from human clinical samples from wounds, see Farmer et al. 1989 J. Clin. Microbiol. 27 pp. 1594-1600. These saprohytic strains are deposited in the American Type Culture Collection (Rockville, MD) ATCC #s 43948, 43949, 43950, 43951, and 43952, and are incorporated herein by reference. It is possible that other sources could harbor *Photorhabdus* bacteria that produce insecticidal toxins. Such sources in the environment could be either terrestrial or aquatic based.

The genus *Photorhabdus* is taxonomically defined as a member of the Family *Enterobacteriaceae,* although it has certain traits atypical of this family. For example, strains of this genus are nitrate reduction negative, yellow and red pigment producing and bioluminescent. This latter trait is otherwise unknown within the *Enterobacteriaceae. Photorhabdus* has only recently been described as a genus separate from the *Xenorhabdus* (Boemare et al., 1993 Int. J. Syst. Bacteriol. 43, 249-255). This differentiation is based on DNA-DNA hybridization studies, phenotypic differences (e.g., presence (*Photorhabdus*) or absence (*Xenorhabdus*) of catalase and bioluminescence) and the Family of the nematode host (*Xenorhabdus; Steinernematidae, Photorhabdus; Heterorhabditidae*). Comparative, cellular fatty-acid analyses (Janse et al. 1990, Lett. Appl. Microbiol 10, 131-135; Suzuki et al. 1990, J. Gen. Appl. Microbiol., 36, 393-401) support the separation of *Photorhabdus* from *Xenorhabdus.*

Currently, the bacterial genus *Photorhabdus* is comprised of a single defined species, *Photorhabdus luminescens* (ATCC Type strain #29999, Poinar et al., 1977, Nematologica 23, 97-102). A variety of related strains have been described in the literature (e.g., Akhurst et al. 1988 J. Gen. Microbiol., 134, 1835-1845; Boemare et al. 1993 Int. J. Syst. Bacteriol. 43 pp. 249-255; Putz et al. 1990, Appl. Environ. Microbiol., 56, 181-186).

The following toxin producing *Photorhabdus* strains have been deposited:

| strain | accession number | date of deposit |
|---|---|---|
| W-14 | ATCC 55397 | March 5, 1993 |
| WX1 | NRRL B-21710 | April 29, 1997 |
| WX2 | NRRL B-21711 | April 29, 1997 |
| WX3 | NRRL B-21712 | April 29, 1997 |
| WX4 | NRRL B-21713 | April 29, 1997 |
| WX5 | NRRL B-21714 | April 29, 1997 |
| WX6 | NRRL B-21715 | April 29, 1997 |
| WX7 | NRRL B-21716 | April 29, 1997 |
| WX8 | NRRL B-21717 | April 29, 1997 |
| WX9 | NRRL B-21718 | April 29, 1997 |
| WX10 | NRRL B-21719 | April 29, 1997 |
| WX11 | NRRL B-21720 | April 29, 1997 |
| WX12 | NRRL B-21721 | April 29, 1997 |
| WX14 | NRRL B-21722 | April 29, 1997 |
| WX15 | NRRL B-21723 | April 29, 1997 |
| H9 | NRRL B-21727 | April 29, 1997 |
| Hb | NRRL B-21726 | April 29, 1997 |
| Hm | NRRL B-21725 | April 29, 1997 |
| HP88 | NRRL B-21724 | April 29, 1997 |
| NC-1 | NRRL B-21728 | April 29, 1997 |
| W30 | NRRL B-21729 | April 29, 1997 |
| WIR | NRRL B-21730 | April 29, 1997 |
| B2 | NRRL B-21731 | April 29, 1997 |
| ATCC 43948 | ATCC 55878 | November 5, 1996 |
| ATCC 43949 | ATCC 55879 | November 5, 1996 |
| ATCC 43950 | ATCC 55880 | November 5, 1996 |
| ATCC 53951 | ATCC 55881 | November 5, 1996 |
| ATCC 43952 | ATCC 55882 | November 5, 1996 |
| DEPI | NRRL B-21707 | April 29, 1997 |
| DEP2 | NRRL B-21708 | April 29, 1997 |
| DEP3 | NRRL B-21709 | April 29, 1997 |
| P. zealandrica | NRRL B-21683 | April 29, 1997 |
| P. hepialus | NRRL B-21684 | April 29, 1997 |
| HB-Arg | NRRL B-21685 | April 29, 1997 |
| HB Oswego | NRRL B-21686 | April 29, 1997 |
| Hb Lewiston | NRRL B-21687 | April 29, 1997 |
| K-122 | NRRL B-21688 | April 29, 1997 |
| HMGD | NRRL B-21689 | April 29, 1997 |
| Indicus | NRRL B-21690 | April 29, 1997 |
| GD | NRRL B-21691 | April 29, 1997 |
| PWH-5 | NRRL B-21692 | April 29, 1997 |
| Megidis | NRRL B-21693 | April 29, 1997 |
| HF-85 | NRRL B-21694 | April 29, 1997 |
| A. Cows | NRRL B-21695 | April 29, 1997 |
| MP1 | NRRL B-21696 | April 29, 1997 |
| MP2 | NRRL B-21697 | April 29, 1997 |
| MP3 | NRRL B-21698 | April 29, 1997 |
| MP4 | NRRL B-21699 | April 29, 1997 |
| MP5 | NRRL B-21700 | April 29, 1997 |
| GL98 | NRRL B-21701 | April 29, 1997 |
| G1101 | NRRL B-21702 | April 29, 1997 |
| GL138 | NRRL B-21703 | April 29, 1997 |
| GL155 | NRRL B-21704 | April 29, 1997 |
| GL217 | NRRL B-21705 | April 29, 1997 |
| GL257 | NRRL B-21706 | April 29, 1997 |

All strains were deposited in accordance with the terms of the Budapest Treaty. Strains having accession numbers prefaced by "ATTC" were deposited on the indicated date in the American Type Culture Collection, 12301 Parklawn Drive, Rockville, MD 20852 USA. Strains prefaced by "NRRL" were deposited on the indicated date in the Agricultural Research Service Patent Culture Collection (NRRL), National Center for Agricultural Utilization Research, ARS-USDA, 1815 North University St., Peoria IL 61.609 USA.

Several terms that are used herein have a particular meaning and are defined as follows:

By "functional activity" it is meant herein that the protein toxins) function as insect control agents in that the proteins are orally active, or have a toxic effect, or are able to disrupt or deter feeding, which may or may not cause death of the insect. When an insect comes into contact with an effective amount of toxin delivered via transgenic plant expression, formulated protein compositions), sprayable protein compositions), a bait matrix or other delivery system, the results are typically death of the insect, or the insects do not feed upon the source which makes the toxins available to the insects.

By "homolog" it is meant an amino acid sequence that is identified as possessing homology to a reference *Photorhabdus* toxin polypeptide amino acid sequence.

By "homology" it is meant an amino acid sequence that has a similarity index of at least 33% and/or an identity index of at least 26% to a reference *Photorhabdus* toxin polypeptide amino acid sequence, as scored by the GAP algorithm using the B10sum 62 protein scoring matrix Wisconsin Package Version 9.0, Genetics Computer Group GCG), Madison, WI).

By "identity" is meant an amino acid sequence that contains an identical residue at a given position, following alignment with a reference *Photrhabdus* toxin polypeptide amino acid sequence by the GAP algorithm.

By the use of the term *"Photorhabdus* toxin" it is meant any protein produced by a *Photorhabdus* microorganism strain which has functional activity against insects, where the *Photorhabdus* toxin could be formulated as a sprayable composition, expressed by a transgenic plant, formulated as a bait matrix, delivered via baculovirus, or delivered by any other applicable host or delivery system.

By the use of the term "toxic" or "toxicity" as used herein it is meant that the toxins produced by *Photorhabdus* have "functional activity" as defined herein.

By "substantial sequence homology" is meant either: a DNA fragment having a nucleotide sequence sufficiently similar to another DNA fragment to produce a protein having similar biochemical properties; or a polypeptide having an amino acid sequence sufficiently similar to another polypeptide to exhibit similar biochemical properties.

As with other bacterial toxins, the rate of mutation of the bacteria in a population causes many related toxins slightly different in sequence to exist. Toxins of interest here are those which produce protein complexes toxic to a variety of insects upon exposure, as described herein. Preferably, the toxins are active against *Lepidoptera, Coleoptera, Homopotera, Diptera, Hymenoptera, Dictyoptera* and *Acarina.* The inventions herein are intended to capture the protein toxins homologous to protein toxins produced by the strains herein and any derivative strains thereof, as well as any protein toxins produced by *Photorhabdus.* These homologous proteins may differ in sequence, but do not differ in function from those toxins described herein. Homologous toxins are meant to include protein complexes of between 300 kDa to 2,000 kDa and are comprised of at least two 2) subunits, where a subunit is a peptide which may or may not be the same as the other subunit. Various protein subunits have been identified and are taught in the Examples herein. Typically, the protein subunits are between about 18 kDa to about 230 kDa; between about 160 kDa to about 230 kDa; 100 kDa to 160 kDa; about 80 kDa to about 100 kDa; and about 50 kDa to about 80 kDa.

As discussed above, some *Photorhabdus* strains can be isolated from nematodes. Some nematodes, elongated cylindrical parasitic worms of the phylum *Nematoda,* have evolved an ability to exploit insect larvae as a favored growth environment. The insect larvae provide a source of food for growing nematodes and an environment in which to reproduce. One dramatic effect that follows invasion of larvae by certain nematodes is larval death. Larval death results from the presence of, in certain nematodes, bacteria that produce an insecticidal toxin which arrests larval growth and inhibits feeding activity.

Interestingly, it appears that each genus of insect parasitic nematode hosts a particular species of bacterium, uniquely adapted for symbiotic growth with that nematode. In the interim since this research was initiated, the name of the bacterial genus *Xenorhabdus* was reclassified into the *Xenorhabdus* and the *Photorhabdus.* Bacteria of the genus *Photorhabdus* are characterized as being symbionts of *Heterorhabditus* nematodes while *Xenorhabdus* species are symbionts of the *Steinernema* species. This change in nomenclature is reflected in this specification, but in no way should a change in nomenclature alter the scope of the inventions described herein.

The peptides and genes that are disclosed herein are named according to the guidelines recently published in the Journal of Bacteriology "Instructions to Authors" p. i-xii Jan. 1996), which is incorporated herein by reference.

Transformation methods useful in carrying out the invention are well known, and are described, for example, in WO98/08932

### Hemicot tcdA and tcbA

SEQ ID NO: 3 is the nucleotide sequence for an engineered tcdA gene in accordance with the invention. SEQ ID NO: 4 is the nucleotide sequence for an engineered *tcbA* gene.

The following Tables 1 and 2 identify significant features of the engineered tcdA and tcbA genes.

**Table 1 tcdA**

| Feature | nucleotides of SEQ ID NO:3 |
|---|---|
| *Nco*I | 1-6 |
| *Hind*III | 48-53 |
| *Kpn*I | 246-254 |
| sequence encoding TcbAii | 267-5798 |
| *Nhe*I | 333-338 |
| *Bgl*II | 1215-1220 |
| *Cla*I | 2604-2609 |
| *Pst*I | 4015-4020 |
| *Age*I | 5088-5093 |
| *Mun*I | 5598-5603 |
| *Xba*I | 5778-5783 |
| sequence encoding TcbAiii | 5799-7517 |
| *Afl*II | 5853-5858 |
| *Sph*I | 6439-6444 |
| *Sfu*I | 7392-7397 |
| *Sac*I | 7519-7524 |
| *Xho*I | 7522-7527 |
| *Stu*I | 7528-7533 |
| *Not*I | 7533-7538 |

**Table 2 tcbA**

| Feature | nucleotides of SEQ ID NO:4 |
|---|---|
| *Nco*I | 1-6 |
| *Hind*III | 48-53 |
| *Kpn*I | 246-251 |
| sequence encoding TcbAii | 267-5798 |
| *Nhe*I | 333-338 |
| *Bgl*II | 1215-1220 |
| *Cla*I | 2604-2609 |
| *Pst*I | 4015-4020 |
| *Age*I | 5088-5093 |
| *Mun*I | 5598-5603 |
| *Xba*I | 5778-5783 |
| sequence encodingTcbAiii | 5799-7517 |
| *Afl*II | 5853-5858 |
| *Sph*I | 6439-6444 |
| *Sfu*I | 7392-7397 |
| *Sac*I | 7519-7524 |
| *Sfu*I | 7392-7397 |
| *Sac*I | 7519-7524 |
| *Xho*I | 7522-7527 |
| *Stu*I | 7528-7533 |
| *Not*I | 7535-7540 |

It should be noted that the proteins encoded by the plant-optimized *tcdA* (SEQ ID NO:3) and *tcbA* (SEQ ID NO:4) differ from the native proteins by the addition of an Ala residue at position #2. This modification was made to accommodate the *Nco*I site which spans the ATG start codon.

The following Table 3 compares the codon composition of the engineered tcdA gene of SEQ ID NO:3 and engineered tcbA gene of SEQ ID NO:4 with the codon compositions of the native genes, the typical dicot genes, and maize genes.

**Table 3**

| amino acid | codon | % in SEQ ID NO:3 | % in *tcdA* | % in SEQ ID NO:4 | % in *tcbA* | % in dicot | % in maize |
|---|---|---|---|---|---|---|---|
| Ala | GCT | 62 | 21 | 69 | 41 | 42 | 24 |
| | GCC | 26 | 32 | 27 | 17 | 27 | 34 |
| | GCA | 11 | 25 | 4 | 22 | 25 | 18 |
| | GCG | 0 | 21 | 0 | 21 | 6 | 24 |
| Arg | AGG | 48 | 0 | 60 | 2 | 25 | 26 |
| | CGC | 22 | 36 | 18 | 16 | 11 | 24 |
| | AGA | 20 | 11 | 15 | 6 | 30 | 15 |
| | CGT | 11 | 39 | 7 | 57 | 21 | 11 |
| | CGG | 0 | 7 | 0 | 13 | 4 | 15 |
| | CGA | 0 | 8 | 0 | 6 | 8 | 9 |
| Asn | AAC | 100 | 32 | 100 | 33 | 55 | 68 |
| | AAT | 0 | 68 | 0 | 67 | 45 | 32 |
| Asp | GAC | 67 | 22 | 70 | 25 | 42 | 63 |
| | GAT | 33 | 78 | 30 | 75 | 58 | 37 |
| Cys | TGC | 100 | 30 | 100 | 19 | 56 | 68 |
| | TGT | 0 | 70 | 0 | 81 | 44 | 32 |
| End | TGA | 100 | 0 | 100 | 0 | 33 | 59 |
| | TAG | 0 | 0 | 0 | 0 | 19 | 21 |
| | TAA | 0 | 100 | 0 | 100 | 48 | 20 |
| Gln | CAA | 65 | 61 | 74 | 53 | 59 | 38 |
| | CAG | 35 | 39 | 26 | 47 | 41 | 62 |
| Glu | GAG | 100 | 24 | 98 | 36 | 51 | 71 |
| | GAA | 0 | 76 | 2 | 64 | 49 | 29 |
| Gly | GGT | 67 | 37 | 64 | 44 | 33 | 20 |
| | GGC | 32 | 36 | 36 | 22 | 16 | 42 |
| | GGA | 1 | 20 | 0 | 19 | 38 | 19 |
| | GGG | 0 | 8 | 0 | 16 | 12 | 20 |
| His | CAC | 62 | 40 | 72 | 31 | 46 | 62 |
| | CAT | 38 | 60 | 28 | 69 | 54 | 38 |
| Ile | ATC | 73 | 34 | 65 | 24 | 37 | 58 |
| | ATT | 27 | 51 | 35 | 59 | 45 | 28 |
| | ATA | 0 | 15 | 0 | 17 | 18 | 14 |
| Leu | CTC | 54 | 11 | 59 | 7 | 28 | 26 |
| | TTG | 29 | 17 | 25 | 32 | 26 | 15 |
| | CTT | 16 | 9 | 15 | 7 | 19 | 17 |
| | TTA | 0 | 18 | 0 | 19 | 10 | 5 |
| | CTG | 0 | 32 | 0 | 29 | 9 | 29 |
| | CTA | 0 | 13 | 0 | 7 | 8 | 8 |
| Lys | AAG | 99 | 79 | 99 | 75 | 61 | 78 |
| | AAA | 1 | 21 | 1 | 25 | 39 | 22 |
| Met | ATG | 100 | 100 | 100 | 100 | 100 | 100 |
| Phe | TTC | 100 | 42 | 100 | 41 | 55 | 71 |
| | TTT | 0 | 58 | 0 | 59 | 45 | 29 |
| Pro | CCA | 74 | 30 | 91 | 26 | 42 | 26 |
| | CCT | 22 | 28 | 7 | 20 | 32 | 22 |
| | CCC | 4 | 14 | 3 | 7 | 17 | 24 |
| | CCG | 0 | 27 | 0 | 47 | 9 | 28 |
| Ser | TCC | 47 | 19 | 55 | 11 | 18 | 23 |
| | TCT | 35 | 15 | 30 | 15 | 25 | 15 |
| | AGC | 18 | 22 | 15 | 18 | 18 | 23 |
| | AGT | 0 | 20 | 0 | 31 | 14 | 9 |
| | TCG | 0 | 7 | 0 | 8 | 6 | 14 |
| | TCA | 0 | 17 | 0 | 17 | 19 | 16 |
| Thr | ACC | 60 | 41 | 64 | 31 | 30 | 37 |
| | ACT | 28 | 25 | 32 | 34 | 35 | 20 |
| | ACA | 12 | 21 | 4 | 18 | 27 | 21 |
| | ACG | 0 | 13 | 0 | 18 | 8 | 22 |
| Trp | TGG | 100 | 100 | 100 | 100 | 100 | 100 |
| Tyr | TAC | 100 | 24 | 100 | 19 | 57 | 73 |
| | TAT | 0 | 76 | 0 | 81 | 43 | 27 |
| Val | GTC | 69 | 27 | 73 | 11 | 20 | 31 |
| | GTG | 21 | 17 | 22 | 27 | 29 | 39 |
| | GTT | 10 | 34 | 3 | 48 | 39 | 21 |
| | GTA | 0 | 22 | 2 | 14 | 12 | 8 |

### EXAMPLE 1

### Design Of Plant Codon-Biased Genes Encoding W-14 Peptides TcbA and TcdA

### A. Gene Design

The coding strands of the native DNA sequences of the *Photorhabdus* W-14 genes encoding peptides TcbA and TcdA were scanned for the presence of deleterious sequences such as the Shaw/Kamen RNA destabilizing motif ATTTA, intron splice recognition sites, and poly A addition motifs. This was done using the MacVector Sequence Analysis Software (Oxford Molecular Biology Group, Symantec Corp.), using a custom Nucleic Acid Subsequence File. The native sequence was also searched for runs of 4 or more of the same base.

Motif searching of the native W-14 *tcbA* and *tcdA* genes revealed the presence of many potentially deleterious sequences in the protein coding strands, as summarized in Table 4. Not shown, but also present, were many runs of four or more single residues (e.g. the native tcbA gene has 81 runs of four A's).

**Table 4**

| Native Gene | ATTTA | 5' Splice | 3' Splice | Poly A Addition* | RNAP II term. |
|---|---|---|---|---|---|
| *tcbA* | 18 | 7 | 17 | 46 | 0 |
| *tcdA* | 18 | 7 | 13 | 77 | 1 |

| | | | | | |
|---|---|---|---|---|---|
| * Totals of 16 different motifs. | | | | | |

Analyses of eukaryotic genes and plant genes in particular have shown that CG & TA doublets are underrepresented, while the genes are enriched in CT & TG doublets. The sequences of the hemicot biased genes have accordingly been adjusted to encompass these base compositions and to have G+C compositions of about 53%, similar to many plant genes. When compared to the native W-14 *tcbA* and *tcdA* genes, the plant-biased genes have a much more uniform G+C distribution.

Nucleotide changes to remove potentially deleterious sequences were chosen to simultaneously adjust the codon composition of the coding region to more closely reflect that of plant genes. A framework for these changes was provided by the codon bias tables prepared for maize and dicot genes shown in Table 3.

Comparison of codon compositions of the native W-14 genes to maize and dicot genes revealed that the W-14 genes contain a very different preference set of the degenerate codons for the 18 amino acids for which there is a choice (Table 3). For each of 8 amino acids (Phe, Tyr, Cys, Arg, Asn, Lys, Glu, and Gly) in both W-14 genes, the most abundant codon is different from the preferred codons found in either maize or dicot genes. One might expect that translational difficulties would be encountered in efforts to produce in plants proteins (such as TcbA and TcdA) having high relative amounts of these amino acids from mRNAs having large numbers of nonpreferred codons. There is a marked difference in distribution of the codon compositions specifying the other 10 amino acids. For His, Gln, Ile, Val, and Asp, the dicot-preferred codons are found as the most abundant ones in both W-14 genes. For Leu, Thr, Ser, and Ala, the maize preferred codons are the most abundant codon choices found in the *tcdA* gene. In contrast, the *tcbA* gene contains only the CCG (Pro) maize-preferred codon as the highest abundance choice.

In making the codon choices, doublet contents were considered, so that adjacent codons preferably did not form CG or TA doublets (which are underrepresented in eukaryotic genes; 1, 4), while CT or TG doublets (which are enriched in eukaryotic genes ibid.) were created when possible.

Choices were also made to utilize a diversity of codons for Met, Trp, Asn, Asp, Cys, Glu, His, Ile, Lys, Phe, Thr, and Tyr.

The sequences were also designed to encode unique 6-bp recognition sites for restriction enzymes, spaced about every 1200 bp. Finally, an additional codon (GCT; Ala) was inserted at the second position to encode an Nco I recognition site encompassing the ATG (Met) start codon. Additional recognition sites were included after the stop codon to facilitate subsequent cloning steps into expression vectors. These features are set forth above in Tables 1 and 2.

The new tcdA and tcbA genes of SEQ ID NO:3 and SEQ ID NO:4 share 73.5%,and 72.6%% identity, respectively, to their native W-14 counterparts (Wisconsin Genetics Computer Group, GAP algorithm).

### B. Gene Synthesis

The complete synthesis of the plant codon-biased *tcbA* and *tcdA* genes was performed under contract by Operon Technologies, Inc. (OPTI, Alameda, CA). Basically, chemically synthesized oligonucleotides of appropriate sequence were assembled into DNA pieces about 500 bases long. These were joined together end-to-end (presumably by means of appropriately placed restriction enzyme sites) into four larger pieces of roughly 2 kilobase pairs (kbp) each; therefore each comprised about 1/4 of the entire coding region of the particular gene. DNA sequence of the pieces was confirmed at this step. If mistakes in sequence were present, the appropriate oligonucleotides were re-synthesized, and the assembly process was repeated. Once gene fractional parts were sequence verified, they were assembled in pairs to make the gene halves, and again sequence verified. Finally, the two halves were joined, and the sequences of the junctions between the halves was verified. Therefore, each part of the new gene was sequence verified at least twice.

It should be noted that attempts to express the native *tcbA* or *tcdA* genes in standard *Escherichia coli* cloning strains suggests that production of these proteins is lethal. Lethality problems may be encountered if standard cloning vectors having leaky expression from inherent *lacZ* promoters are used to assemble these genes.

### C. Addition Of Endoplasmic Reticulum Targeting Peptide To Tcda Coding Region

It is known to those in the field of plant gene expression that proteins are specifically directed into the endoplasmic reticulum (ER) by means of a short signal peptide which is removed during or after the transport process through the ER membrane. The mature (processed) protein is incorporated into the ER endomembrane or is released into the ER lumen where the transported protein may be uniquely folded (aided by chaperonins), modified by glycosylation, accumulated in the vacuole, or additionally translocated (by secretion). These processes are reviewed by Gomord and Faye [V. Gomord and L. Faye, (1996) Signals and mechanisms involved in intracellular transport of secreted proteins in plants. Plant Physiol. Biochem. 34:165-181] and by Bar-Peled *et al.* [M. Bar-Peled, D. C. Bassham, and N. V. Raikhel, (1996) Transport of proteins in eukaryotic cells: more questions ahead. Plant Molec. Biology 32:223-249]. It is also known that the subcellular recognition mechanisms for an ER signal peptide are evolutionarily somewhat conserved, since the ER signal for a protein normally produced in monocot (maize) cells is recognized and processed normally by dicot (tobacco) cells. This is exemplified by the maize 15 kDa zein ER signal peptide [L. M. Hoffman, D. D. Donaldson, R. Bookland, K. Rashka, and E. M. Herman, (1987) Synthesis and protein body deposition of maize 15-kd zein in transgenic tobacco seeds. EMBO J. 6:3213-3221, and U.S. Patent 5589616]. Further, it is known that the ER signal peptide derived from one protein can direct the translocation of a different protein if it is appropriately attached to the second protein by genetic engineering methods [D. C. Hunt and M. J. Chrispeels, (1991) The signal peptide of a vacuolar protein is necessary and sufficient for the efficient secretion of a cytosolic protein. Plant Physiol. 96:18-25, and Denecke, J., J. Botterman, and R. Deblaere (1990) Protein secretion in plants can occur via a default pathway. Plant Cell 2:51-59]. Therefore, one may expose a protein *in vivo* to different biochemical environments by directing its accumulation in the cytosol (by not providing a signal peptide sequence), or in the ER/vacuole (by provision of an appropriate signal peptide.)

The ER signal peptide of maize 15 kDa zein proteins is known to comprise the first 20 amino acids encoded by the zein coding region. Two examples of such signal peptides the ER signal peptide of 15 kDa zein from A5707 maize, NCBI Accession # M72708, and the ER signal peptide of 15 kDa zein from Black Mexican Sweet maize, NCBI Accession # M13507. There is only a single amino acid difference (Ser vs Cys at residue 17) between these signal peptides.

SEQ ID NO:5 is a modified sequence coding the ER signal peptide of 15 kDa zein from Black Mexican Sweet maize. The modifications embodied in this sequence were made to accommodate the different monocot/dicot codon usages and other sequence motif considerations discussed above in the design of the plant-optimized *tcdA* coding region. The sequence includes an additional Ala residue at position #2 to accommodate the *Nco*I site which spans the ATG start codon.

SEQ ID NO:6 gives a sequence coding for the full-length native TcdA protein (amino acids 22-2537) fused to the modified 15 kDa zein endoplasmic reticulum signal peptide (amino acids 1-21).

### Example 2

### Transformation Of Tobacco With Agrobacterium Carrying Plasmid pDAB2041 Encoding Photorhabdus Toxins

### A. Plasmid pDAB2041

Preparation of tobacco transformation vectors was accomplished in three steps. First, a modified plant-optimized *tcdA* coding region was ligated into a tobacco plant expression cassette plasmid. In this step, the coding region was placed under the transcriptional control of a promoter functional in tobacco plant cells. RNA transcription termination and polyadenylation were mediated by a downstream copy of the terminator region from the *Agrobacterium* nopaline synthase gene. Two plasmids designed to function in this role are pDAB1507 and pDAB2006. In the second step, the complete gene comprised of the promoter, coding region, and terminator region was ligated between the T-DNA borders of an *Agrobacterium* binary vector, pDAB1542. Also positioned between the T-DNA borders was a plant selectable marker gene to allow selection of transformed tobacco plant cells. In the third step, the engineered binary vector plasmid was conjugated from its *E. coli* host strain into a disabled *Agrobacterium tumefaciens* strain capable of transforming tobacco plant cells that regenerate into fertile transgenic plants.

It is a feature of plasmid pDAB1507 that any coding region having an *Nco*I site at its 5' end and a *Sac*I site 3' to the coding region, when cloned into the unique *Nco*I and *Sac*I sites of pDAB1507, is placed under the transcriptional control of an enhanced version of the CaMV 35S promoter. It is also a feature of pDAB1507 that the 5' untranslated leader (UTR) sequence preceding the *Nco*I site comprises a modified version of the 5' UTR of the MSV coat protein gene, into which has been cloned an internally deleted version of the maize Adh1S intron 1. Additionally it is a feature of pDAB1507 that transcription termination and polyadenylation of the mRNA containing the introduced coding region are mediated by termination/Poly A addition sequences derived from the nopaline synthase (Nos) gene. Finally, it is a feature of pDAB1507 that the entire assembly of promoter/coding region/3'UTR can be obtained as a single DNA fragment by cleavage at the flanking *Not*I sites.

It is a feature of plasmid pDAB2006 that any coding region having an *Nco*I site at its 5' end and a SacI site 3' to the coding region, when cloned into the unique *Nco*I and *Sac*I sites of pDAB2006, is placed under the transcriptional control of the CaMV 35S promoter. It is also a feature of pDAB2006 that the 5' untranslated leader (UTR) sequence preceding the *Nco*I site comprises a polylinker. Additionally it is a feature of pDAB2006 that transcription termination and polyadenylation of the mRNA containing, the introduced coding region are mediated by termination/Poly A addition sequences derived from the nopaline synthase (Nos) gene. Finally, it is a feature of pDAB2006 that the entire assembly of promoter/coding region/3'UTR can be obtained as a single DNA fragment by cleavage at the flanking *Not*I sites.

It is a feature of pDAB1542 that any DNA fragment flanked by *Not*I sites can be cloned into the unique *Not*I site of pDAB1542, thus placing the introduced fragment between the T-DNA borders, and adjacent to the neomycin phosphotransferase II (kanamycin resistance) gene. To prepare a plant-expressible gene to produce the non-targeted TcdA protein in tobacco plant cells, DNA of a plasmid (pAOH_4-OPTI) containing the plant-optimized *tcdA* coding region, (SEQ ID No:3) was cleaved with restriction enzymes *Nco*I and *Sac*I, and the large 7550 bp fragment was ligated to similarly-cut DNA of plasmid pDAB1507 to produce plasmid pDAB2040. DNA of pDAB2040 was then digested with *Not*I, and the 8884 bp fragment was ligated to *Not*I digested DNA of pDAB1542 to produce plasmid pDAB2041. This plasmid was then conjugated by triparental mating [Firoozabady, E., D. L. DeBoer, D. J. Merlo, E. L. Halk, L. N. Amerson, K. E. Rashka, and E. E. Murray (1987) Transformation of cotton (Gossypium hirsutum L.) by Agrobacterium tumefaciens and regeneration of transgenic plants. Plant Molec. Biol. 10:105-116] from the host *Escherichia coli* strain (XL1-Blue, Stratagene, La Jolla, CA), into the nontumorigenic *Agrobacterium tumefaciens* strain EHA101S, which is a spontaneous streptomycin-resistant mutant of strain EHA101 (Hood, E. E., G. L. Helmer, R. T. Fraley, and M.-D. Chilton (1986) The hypervirulence of Agrobacterium tumefaciens A281 is encoded in a region of pTiBo542 outside of T-DNA. J. Bacteriol. 168:1291-1301). Strain EHA101S(pDAB2041) was then used to produce transgenic tobacco plants that expressed the TcdA protein.

### B. Plasmid pRK2013

To prepare a plant-expressible gene to produce the endoplasmic reticulum-targeted TcdA protein in tobacco plant cells, DNA of a plasmid (pA0H_4-ER) containing the plant-optimized, ER-targeted *tcdA* coding region, (SEQ ID No:6) was cleaved with restriction enzymes *Nco*I and *Sac*I, and the large 7610 bp fragment was ligated to similarly-cut DNA of plasmid pDAB2006 to produce plasmid pDAB1833. DNA of pDAB1833 was then digested with *Not*I, and the 8822 bp fragment was ligated to *Not*I digested DNA of pDAB1542 to produce plasmid pDAB2052. This plasmid was then conjugated by triparental mating from the host *Escherichia coli* strain (XL1-Blue), into the nontumorigenic *Agrobacterium tumefaciens* strain EHA101S. Strain EHA101S (pDAB2052) was then used to produce transgenic tobacco plants that expressed the TcdA protein containing an amino terminus endoplasmic reticulum targeting peptide.

### C. Transfer of Plasmid pDAB2041 Into Agrobacterium Strain EHA101S

Cultures of *E. coli* carrying the engineered Ti plasmid pDAB2041 (plasmid containing the rebuilt Toxin A gene, *tcdA), E. coli* carrying the plasmid pRK2013, and *Agrobacterium* strain EHA101S were grown overnight, then mixed 1:1:1 on plain LB medium solidified with agar and cultured in the dark at 28°C. Two days later, the lawn of bacteria was scraped up with a loop, suspended in plain LB medium, vortexed, and then diluted 1:10⁴ , 1:10⁵, and 1:10⁶ fold in plain LB liquid medium. Aliquots of these dilutions were spread on selective plates containing medium YEP plus erythromycin (100 mg/L) and streptomycin (250 mg/L) and grown at 28°C. Two days later, single colonies were picked and streaked onto the same medium, then spread to give single colonies. Single colonies were picked again and streaked, then spread for single colonies. Single colonies were picked a third time, grown as streaks, then subjected to a quality analysis involving growth on lactose medium and chromogenic assay with Benedict's reagent. Of ten strains developed in this way, the fastest coloring colony was chosen for further work.

### D. Transformation Of Tobacco With Agrobacterium Carrying Plasmid pDAB2041

Tobacco transformation with *Agrobacterium tumefaciens* was carried out by a method similar, but not identical, to published methods (R Horsch et al, 1988. Plant Molecular Biology Manual, S. Gelvin et al, eds., Kluwer Academic Publishers, Boston). To provide source tissue for the transformation, tobacco seed (*Nicotiana tabacum* cv. Kentucky 160) were surface sterilized and planted on the surface of TOB- , which is a hormone-free Murashige and Skoog medium (T. Murashige and F. Skoog, 1962). A revised medium for rapid growth and bioassays with tobacco tissue culture. Plant Physiol. 75: 473-497) solidified with agar. Plants were grown for 6-8 weeks in a lighted incubator room at 28-30°C and leaves were collected sterilely for use in the transformation protocol. Approximately one cm² pieces were sterilely cut from these leaves, excluding the midrib. Cultures of the *Agrobacterium* strains (EHA101S containing pDAB2041), which had been grown overnight on a rotor at 28°C, were pelleted in a centrifuge and resuspended in sterile Murashige & Skoog salts, adjusted to a final optical density of 0.7 at 600 nm. Leaf pieces were dipped in this bacterial suspension for approximately 30 seconds, then blotted dry on sterile paper towels and placed right side up on medium TOB+ (Murashige and Skoog medium containing 1 mg/L indole acetic acid and 2.5 mg/L benzyladenine) and incubated in the dark at 28°C. Two days later the leaf pieces were moved to medium TOB+ containing 250 mg/L cefotaxime (Agri-Bio, North Miami, Florida) and 100 mg/L kanamycin sulfate (AgriBio) and incubated at 28-30°C in the light. Leaf pieces were moved to fresh TOB+ with cefotaxime and kanamycin twice per week for the first two weeks and once per week thereafter. Leaf pieces which showed regrowth of the *Agrobacterium* strain were moved to medium TOB+ with cefotaxime and kanamycin, plus 100 mg/l carbenicillin (Sigma). Four to six weeks after the leaf pieces were treated with the bacteria, small plants arising from transformed foci were removed from this tissue preparation and planted into medium TOB- containing 250 mg/L cefotaxime and 100 mg/L kanamycin in Magenta GA7 boxes (Magenta Corp., Chicago). These plantlets were grown in a lighted incubator room. After 3-4 weeks the primary transgenic plants had rooted and grown to a size sufficient that leaf samples could be analyzed for expression of protein from the transgene. Twenty-five independent transgenic events were recovered as single plants from the pDAB2041 transformation.

Eight independent lines expressing various levels of transgenic protein from the T-DNA of pDAB2041 were propagated *in vitro* from leaf pieces as follows. Twelve to sixteen approximately one cm² pieces were sterilely cut from leaves of each primary transgenic plant, excluding the midrib and all naturally occurring edges. These leaf pieces were placed on medium TOB+ containing 250 mg/L cefotaxime and 100 mg/L kanamycin, and cultured in the lighted incubator at 28-30°C for 3-4 weeks, at which time small plants could be cut from the proliferating tissue mass. Several small plantlets from each transgenic line were moved into Magenta boxes containing medium TOB- plus cefotaxime and kanamycin and allowed to root and grow. The proliferating tissue mass was further cultured on medium TOB+ with cefotaxime and kanamycin, and additional plants could be cut out and grown up as needed.

Plants were moved into the greenhouse by washing the agar from the roots, transplanting into soil in 5 ½" square pots, placing the pot into a Ziploc bag (DowBrands), placing plain water into the bottom of the bag, and placing in indirect light in a 30°C greenhouse for one week. After one week the bag could be opened; the plants were fertilized and allowed to grow further, until the plants were acclimated and the bag was removed. Plants were grown under ordinary warm greenhouse conditions (30°C, 16 H light). Plants were suitable for sampling four weeks post transplant.

### Example 3

### Chacterization Of Transgenic Tobacco Plants Expressing Photorhabdus Toxin That Confer Insect Control.

### A. Polyclonal Antibody Production

The *E. coli* produced recombinant TcdA protein was purified by a series of column purification. The protein was sent to Berkley Antibody Company (Richmond, CA) for the production of antiserum in a rabbit. Inoculations with the antigen were initiated with 0.5 mg of protein followed by four boosting injections of 0.25 mg each at about three week intervals. The rabbit serum was tested by the standard Western analysis using the recombinant TcdA protein as the antigen and enhanced chemi-luminescens, ECL method (Amersham, Arlington Heights, IL ) The antibodies (PAb-EA₀) were purified using a PURE I antibody purification kit (Sigma, St. Luis, MO). PAb-EA₀ antibodies recognize the full-length TcdA and its processed components.

### B. Expression Of TcdA Protein In Tobacco

Protein was extracted from the leaf tissue of transformed and non-transformed tobacco plants following the procedure described immediately below.

Two leaf disks of 1.4 cm in diameter were harvested from the middle portion of a fully expanded leaf. The disks were placed on a 1.6 x 4 cm piece of 3M Whatman paper. The paper was folded lengthwise and inserted in a flexible straw. Four hundred micro liters of the extraction buffer (9.5 ml of 0.2 M NaH₂PO₄, 15.5 ml of 0.2 M Na₂HPO₄, 2 ml of 0.5 M Na₂EDTA, 100 ml of Triton X100, 1 ml of 10% Sarkosyl, 78 ml of beta-mercaptoethanol, H₂O to bring total volume to 100 ml) was pipetted on to the paper. The straw containing the sample was then passed through a rolling device used for squeezing out the extract 1.5 mL micro centrifuge tube was placed at the other end of the straw to collect the extract. The extract was centrifuged for 10 minutes at 14,000 rpm in an Eppendorf regrigerated microcentrifuge. The supernatant was transferred into a new tube. Protein quantitation analysis was performed using the standard Bio-Rad Protein Analysis protocol (Bio-Rad Laboratories, Hercules, CA). The extract was diluted to 2 mg/ml of total protein using the extraction buffer.

For the detection of transgenic protein, Western blot analysis was performed. Following a standard procedure for protein separation (Laemmli, 1970), 40 µg of protein was loaded in each well of 4-20% gradient polyacrylamide gel (Owl Scientific Co., MA) for electrophoresis. Subsequently, the protein was transferred onto a nitrocellulose membrane using a semi-dry electroblotter (Pharmacia LKB Biotechnology, Piscataway, NJ). The membrane was incubated for one hour in Blotto (5% milk in TBST solution; 25 mM Tris HCL pH 7.4, 136 mM NaCl, 2.7 mM KCl, 0.1% Tween 20). Thereafter , Blotto was replaced by the primary antibody solution (in Blotto). After one hour in the primary antibody, the membrane was washed with TBST for five minutes three times. Then the secondary antibody in Blotto (1:2000 dilution of goat anti-rabbit IgG conjugated to horseradish peroxidase; Bio-Rad Laboratories). was added to the membrane. After one hour of incubation, the membrane was washed with an excess amount of TBST for 10 minutes four times. The protein was visualized by using the enhanced chemi-luminescens, ECL method (Amersham, Arlington Heights, IL ). The differential intensity of the protein bands were measured using densitometer (Molecular Dynamics Inc., Sunnyvale, CA).

To determine the expression of TcdA protein in tobacco transformed with pDAB2041, PAb-EA₀ antibodies were used as the primary antibodies. The expression levels of TcdA protein varied among independent transformation events. The primary plant generated from the event #2041-13 showed the highest level of pre-pro TcdA expression of extractable protein. When the leaf pieces from this plant (#2041-13) were used in *in vitro* propagation, several plants were obtained. Seven of these plants were analyzed for the expression of the TcdA protein. All but one plant produced the full-length TcdA protein as well as some processed peptide components. Using the antibodies specific to Neomycin phosphotransferase, NPT (5 prime-3 prime, Boulder, Co), the expression the selectable marker gene (*npt II*) was detected. Similar results were obtained for #2041-29.

**Table 5**

| Western analysis of plants derived from event #2041-13. | | |
|---|---|---|
| Plant # | TcdA | NPT (selectable marker) |
| 2041-13A | + | not done |
| 2041-13B | + | not done |
| 2041-13-1 | - | + |
| 2041-13-2 | + | + |
| 2041-13-3 | + | + |
| 2041-13-4 | + | + |
| 2041-13-5 | + | + |

### C. Nucleic Acid Analysis of Transgenic Tobacco Lines

Genomic DNA was prepared from a group of 2041 transgenic events. The lines included Magenta box stage 2041-13, and greenhouse stage plants 2041-13-1, 2041-13-2, 2041-13-5, 2041-9, 2041-20A and 2041-20B. A transgenic GUS line (2023) was included as a negative control. Southern analysis of these lines was performed. The genomic tobacco DNA was restricted with the enzyme SstI which should result in a 8.9 kb hybridization product when hybridized to a tcdA gene specific probe. The 8.9 kb hybridization product should consist of the 35T promoter and the *tcdA* coding region. All 2041 plants contained a band of the expected size. Events 2041-9 and -20 appear to be the same line with 5 identical hybridizing bands. Event 2041-13 produced 6 hybridization fragments with the *tcdA* coding region probe. Magenta box and various greenhouse plants of 2041-13 all produced the same hybridization profile. This hybridization pattern was different from that of events 2041-9 and -20.

RNA analysis, using the *tcdA* coding region probe, was performed on the same group of greenhouse 2041 plants. Immunoblot analysis had revealed that plants 2041-9, 2041-20A, 2041-20B, and 2041-13-1 produced no detectable TcdA protein; while 2041-13-2 and 2041-13-5 produced substantial amounts of full-length TcdA. Northern analysis was in agreement with the immunoblot result. A faint RNA signal was detected for plants 2041-9, 2041-20A, 2041-20B, and 2041-13-1. Only faintly visible was a band corresponding to full-length *tcdA* transcript in plant 2041-13.1. In contrast, for plants 2041-13-2 and 2041-13-5 a strong RNA signal was detected, with a substantial amount of full-length size (~8.0 kb) *tcdA* transcript. These data support the observed bioassay activity for this group of plants.

Genomic DNA was prepared from a second functionally active 2041 transgenic event, 2041-29. Southern analysis of this line was performed. A transgenic GUS line (2023) was included as a negative control, DNA of line 2041-9 was included as a positive control.

The genomic tobacco DNAs were restricted with the enzyme SstI which should result in a 8.9 kb hybridization product when hybridized to a *tcdA* gene specific probe. The 8.9 kb hybridization product should consist of the 35T promoter and the *tcdA* coding region. For plant 2041-29-5, three hybridization products larger than 8.9 kb the were detected with the *tcdA* gene specific probe. Immunoblot analysis has demonstrated pre-pro TcdA protein is made by this plant, it is therefore likely that a restriction site was lost during transformation or regeneration, or the 2041-29 genomic DNA was not thoroughly digested.

### D. Tobacco Leaf-Disk Tests With Tobacco Hornworm Exhibiting Insect Control

Leaves were sampled from tobacco plants, *Nicotiana tabaco,* previously transplanted into the greenhouse. A single leaf was sampled from each plant on each test date. Leaves were selected from the zone where younger elongate leaves transition into older ovate leaves. Excised leaves were placed into 12 oz. cups with the petiole submerged in water to maintain turgor, and transported to the laboratory.

Eight, 1.4 cm disks were cut from the center portion of one side of each leaf (right adaxial side up, with distal portion facing away from the observer). Each disk was placed individually into a well of a C-D International 128 well tray (Pitman, NJ.) into which 0.5 ml of a 1.6% aqueous agar solution had been previously pipetted. The solidified agar prevented the leaf disks from drying out. The adaxial surface of the disk was always oriented up.

A single neonate tobacco hornworm, *Manduca sexta,* was placed on each disk and the wells were sealed with vented plastic lids. The assay was held at 27°C and 40% RH. Larval mortality and live-weight data were collected after 3 days. Data were subjected to analysis of variance and Duncan's multiple range test (α = 0.05)(Proc GLM, SAS Institute Inc., Cary, NC.). Data were transformed using a logarithmic function to correct a correlation between the magnitude of the mean and variance.

**Table 6 Results of leaf-disk assays from greenhouse grown tobacco plants with event 2041-13.**

| | | | Weight of Surviving Larvae (mg) & Duncan's Group¹ | | | | |
|---|---|---|---|---|---|---|---|
| TRT | Plant | Plant Age | Pretest | Test 1 | Test 2 | Test 3 | 3 Test Sum. |
| 13 | non-transformed - 2 | young | --- | --- | --- | 18.8 a* | --- |
| 14 | non-transformed - 3 | young | --- | --- | --- | 17.0 ab | --- |
| 16 | non-transformed - 5 | young | --- | --- | --- | 16.4 ab | --- |
| 3 | 2041-13-1 (western-) | young | --- | 17.6 a | 18.2 a | 16.1 ab | 17.3 a |
| 9 | Gus Control | old | 19.3 a | 14.6 a | 16.3 a | 14.5 ab | 15.1 a |
| 10 | non-transformed - 1 | young | --- | 8.3 b | 16.8 a | 13.9 b | 13.0 b |
| 11 | 204120B (western -) | old | --- | 10.0 b* | 13.7 ab | 14.6 ab | 12.9 b |
| 15 | non-transformed - 4 | young | --- | --- | --- | 13.0 bc | --- |
| 8 | 2041-20A (western -) | old | 15.7 a | 8.3 b | 11.3 bc | 9.2 cd | 9.6 c |
| 12 | 2041-9 (western -) | old | 19.5 a | --- | --- | 7.9 d | --- |
| 7 | 2041-13-5 (western +) | young | --- | 6.3 bc | 9.6 cd | 7.2 de | 7.7 d |
| 5 | 2041-13-3 (western +) | young | --- | 6.4 | 6.2 e | 6.8 de** | 6.4 de |
| | | | | bc**** | | | |
| 1 | 2041-13A (western +) | old | 7.2 b | 6.8 bc* | 7.0 de* | 5.4 e | 6.4 de |
| 6 | 2041-13-4 (western +) | young | --- | 4.9 c**** | 5.8 e | 7.6 d | 6.4 de |
| 4 | 2041-13-2 (western +) | young | --- | 5.7 bc | 5.7 e** | 7.5 d | 6.3 de |
| 2 | 2041-13B (western+) | old | --- | 4.7 c** | 5.6 e | 7.2 de | 5.9 e |

| | | | | | | | |
|---|---|---|---|---|---|---|---|
| * Number of stars corresponds to the number of dead larvae per 8 tested. | | | | | | | |

### 1. Data transformed (logarithm) for analysis.

Means followed by the same letter are not significantly different (alpha = 0.05).

**TABLE 7 Results Of Leaf-Disk Assays From Greenhouse Grown Tobacco Plants With Event 2041-29.**

| | MEAN WGT (MG) / Duncan's Group | | | | |
|---|---|---|---|---|---|
| Plant | Test 1 | Test 2 | Test 3 | Test 4 | Four Test Summary |
| 2014-6 GUS 1 | 15.8 a | 16.6a | **5.5bc | *12.9ab | 13.2 a |
| 2014-6 GUS 2 | 14.4 a | *6.6 bc | *13.4a | 15.2a | 12.6 a |
| KY-160 NTC | 13.4 a | 6.7 bc | 7.9b | 8.5bc | 9.1 b |
| 2041-29 4P | *4.9 b | *7.3b | ****6.9b | ******** | 6.3 c |
| 2041-29 7 | *5.9b b | 5.1bc | ***6.7b | ***7.2c | 6.1 c |
| 2041-29 3P | *5.6 b | **7.9b | *****6.5b | ***3.6d | 5.9 c |
| 2041-29 2P | 6.3 b | ****4.7c | ******4.1c | ******4.6d | 5.4 c |

| | | | | | |
|---|---|---|---|---|---|
| * Number of stars corresponds to the number of dead larvae per 8 tested. 1. Data transformed (logarithm) for analysis. Means followed by the same letter are not significantly different (alpha = 0.05). | | | | | |

All event 2041-29 plants significantly depressed THW larval weight gain compared to control plants. Average weight depression was 49%. Statistically significant mortality occurred in THW larvae exposed to foliage from 2041-29 plants. Mortality averaged 37.5% compared to 5.2% in controls.

### E. Isolation and Characterization of Functional Photorhabdus Toxin Protein From Transgenic Plants

Seven grams of transgenic tobacco plants (2041-13) expressing TcdA (Toxin A) gene were homogenized with 10 ml 50 mM Potassium Phosphate buffer, pH 7.0 using a bead beater (Biospec Products, Bartlesville, OK) according to manufacturer's instructions. The homogenate was filtered through four layers of cheese cloth and then centrifuged at 35,000 g for 15 min. The supernant was collected and filtered through 0.22 µm Millipore Express^{™} membrane. It was then applied to a Superdex 200 cloumn (2.6 × 40 cm) which had been equilibrated with 20 mM Tris buffer, pH 8.0 (Buffer A). The protein was eluted in Buffer A at a flow rate of 3 ml/min. Fractions with 3 ml each were collected and subjected to southern corn rootworm (SCR) bioassay. It was found that fractions corresponding to a native molecular weight around 860 kDa had the highest insecticidal activity. Western analysis of the active fraction using a polyclonal antibody specific to Toxin A indicated the presence of full-length TcdA peptide. The active fractions were further combined and applied to a Mono Q 10/10 column which had been equilibrated with Buffer A. Proteins bound to the column were then eluted by a linear gradient of 0 to 1 M NaCl in Buffer A. Fractions with 2 ml each were collected and analyzed by both SCR bioassay and Western using antibody specific to Toxin A. The results again demonstrated the correlation between insecticidal activity and presence of full-length TcdA peptide.

### F. Characterization of Progeny Transgenic Plants

The inheritability of the genetically engineering plants containing the Photorhabdus toxin gene was evaluated by generating F1 progeny. Progeny was generated from 2041-13 event by selfing expression positive plants. The 2041-13 plants in the greenhouse were allowed to self-pollinate. Seed capsules were collected when mature and were allowed to dry and after-ripen on the laboratory bench for two weeks. Seed from plant designated 2041-13A was surface-sterilized and distributed on the surface of medium TOB- without selection, to allow recovery of nonexpressing or nontransgenic progeny as well as expressing and segregating transgenic siblings. Seed was germinated in a C lighted incubator room (16 H light, 28 C). After 1 month, fifty-one seedlings, designated 2041-13A-S1 through S51, were distributed into Magenta boxes containing medium TOB- to grow further. Three weeks later, leaf samples from these Magenta-box grown seedlings were submitted for evaluation of the level of expression of TcdA toxin.

Leaf samples were tested for kanamycin response by placing sterile leaf segments on medium TOB+ containing 100 mg/L kanamycin in the light and scoring for tissue growth and color after two weeks. All leaf pieces showed some positive response, indicating complex segregation.

This group of in vitro grown event 2041-13 progeny seedlings were all transplanted into the greenhouse approximately two months after seeding onto medium, using the following method. After washing the agar from the roots, plants were transplanted into 5 ½ inch square pots in a soil mix containing 75% MetroMix and 25% mineral soil. They were enclosed in a zip-lock bag and plain water added to leave 1-2 inches of water in the bottom of the bag after soil absorption. These bags were closed and placed under a cart in the greenhouse to protect them from direct sunlight. The bags were opened after 5-6 days, and removed after 7 days, when the plants were adapted to soil and were moved to the top of the cart for normal greenhouse culture. Plants were ready to test in insect bioassays at four weeks post transplant.

F1 progeny were evaluated for expression of protein toxin by immunological screen and for biological activity by plant bioassays, as described previously, using tobacco hornworm. There existed a positive correlation between levels of expression protein toxin and degree of growth inhibition and at higher expression levels mortality was observed. The biological activity was observed to be statistical significance with high cofidence levels between populations of non-transformed and transformed expressing protein toxin.

The following table summarizes the results of insect (tobacco hornworm) bioassays conducted with F1 progeny of self-fertilized 2041-13 plants genetically engineered to produce the "204" A toxin. The tests included 6 nonexpressing progeny (protein-negative controls), 45 toxin A expressors, and 4 non-transformed controls (KY-160). Results are from three leaf-disk assays (method previously outlined) where eight disks were used per test. The data were analyzed using analysis of variance and were blocked by test.

The treatment effect for each of these analyses indicated the Pr > F was less than 0.0001. The Toxin A expressors produced significant control of tobacco hornworm compared to each of the control groups based on each of the three measures of efficacy. The two control groups behaved similarly. Statistical analysis using ANOVA and an LSD test with alpha equal to 0.01 (or 1%) showed differences between the 3 groups. The LSD test indicated that the non-expressors and the non-transformed plants were similar in larvae weights but the expressors gave weights significantly lower than either of the other two groups of plants. These data demonstrated that the genetic basis for insect control was inheritable and corresponded to the presence of expressed toxin gene.

**Table 8 Tobacco hornworm results from F1 progeny of self-fertilized 2041-13 tobacco plants.**

| | Mean Value and Duncan's Grouping^{d} | | |
|---|---|---|---|
| Treatment Group | Total Weight (mg)^{a} | Survivor Weight (mg)^{b} | Leaf Area (cm²)^{c} |
| Non-transformed Control | 15.8 a | 15.8 a | 1.2 a |
| Protein-negative Control | 16.4 a | 16.5 a | 1.2 a |
| Toxin A Expressor | 8.1 b | 9.2 b | 4.9 b |

| | | | |
|---|---|---|---|
| ^{a} Average insect weight with dead insects considered to weigh nothing. ^{b} Average insect weight with dead insects excluded from analysis. ^{c} Total leaf area remaining per eight leaf disks. Initial area was approximately 12 cm². ^{d} Means followed by the same letter are not significantly different (alpha = 0.05). | | | |

### Example 4

### Transformation Of Maize With a Vector Carrying Plasmid pDAB1834 Encoding Photorhabdus Toxins

### A. Preparation Of Maize Transformation Vectors Containing Modified Plant-Optimized Tcda Coding Regions: Plasmid Pdab1834

Preparation of maize transformation vectors was accomplished in two steps. First, a modified plant-optimized *tcdA* coding region was ligated into a plant expression cassette plasmid. In this step, the coding region was placed under the transcriptional control of a promoter functional in maize plant cells. RNA transcription termination and polyadenylation were mediated by a downstream copy of the terminator region from the *Agrobacterium* nopaline synthase gene. One plasmid designed to function in this role is pDAB1538. In the second step, the complete gene comprised of the promoter, coding region, and 3' UTR terminator region was ligated to a plant transformation vector that contained a plant expressible selectable marker gene which allowed the selection of transformed maize plant cells amongst a background of nontransformed cells. An example of such a vector is pDAB367.

It is a feature of plasmid pDAB1538 that any coding region having an *Nco*I site at its 5' end and a SacI site 3' to the coding region, when cloned into the unique *Nco*I and *Sac*I sites of pDAB1538, is placed under the transcriptional control of the maize ubiquitinl (ubi1) promoter. It is also a feature of pDAB1538 that the 5' untranslated leader (UTR) sequence preceding the NcoI site comprises a polylinker. Additionally it is a feature of pDAB1538 that transcription termination and polyadenylation of the mRNA containing the introduced coding region are mediated by termination/Poly A addition sequences derived from the nopaline synthase (Nos) gene. Finally, it is a feature of pDAB1538 that the entire assembly of promoter/coding region/3'UTR can be obtained as a single DNA fragment by cleavage at the flanking NotI sites.

It is a feature of pDAB367 that the phosphinothricin acetyl transferase protein, which has as its substrate phosphinothricin and related compounds, is produced in plant cells through transcription of its coding region mediated by the Cauliflower Mosaic Virus 35S promoter and that termination of transcription plus polyadenylation are mediated by the nopaline synthase terminator region. It is further a feature of pDAB367 that any DNA fragment containing flanking *Not*I sites can be cloned into the unique *Not*I site of pDAB367, thus physically linking the introduced DNA fragment to the aforementioned selectable marker gene.

To prepare a maize plant-expressible gene to produce the endoplasmic reticulum-targeted TcdA protein in plant cells, DNA of a plasmid (pAOH_4-ER) containing the plant-optimized, ER-targeted *tcdA* coding region, (SEQ ID No:6) was cleaved with restriction enzymes *Nco*I and *Sac*I, and the large 7610 bp fragment was ligated to similarly-cut DNA of plasmid pDAB1538 to produce plasmid pDAB1832. DNA of pDAB1832 was then digested with *Not*I, and the 9984 bp *Not*I fragment was ligated into the unique *Not*I site of pDAB367 to produce plasmid pDAB1834.

It is a feature of plasmids pDAB1834 that the ubi1 and 35S promoters are encoded on the same DNA strand.

### B. Transformation and Regeneration of Transgenic Maize Isolates

Type II callus cultures were initiated from immature zygotic embryos of the genotype "Hi-II." (Armstrong et al, (1991) Maize Genet. Coop. Newslett., 65: 92-93). Embryos were isolated from greenhouse-grown ears from crosses between Hi-II parent A and Hi-II parent B or F₂ embryos derived from a self- or sib-pollination of a Hi-II plant. Immature embryos (1.5 to 3.5 mm) were cultured on initiation medium consisting of N6 salts and vitamins (Chu et al, (1978) The N6 medium and its application to anther culture of cereal crops. Proc. Symp. Plant Tissue Culture, Peking Press, 43-56), 1.0 mg/L 2,4-D, 25mM L-proline, 100 mg/L casein hydrolysate, 10 mg/L AgNO₃, 2.5 g/L GELRITE (Schweizerhall, South Plainfield, NJ), and 20 g/L sucrose, with a pH of 5.8. After four to six weeks callus was subcultured onto maintenance medium (initiation medium in which AgNO₃ was omitted and L-proline was reduced to 6 mM). Selection for Type II callus took place for ca. 12-16 weeks.

Plasmid pDAB1834 was transformed into embryogenic callus. For blasting, 140 µg of plasmid DNA was precipitated onto 60 mg of alcohol-rinsed, spherical gold particles (1.5 - 3.0 µm diameter, Aldrich Chemical Co., Inc., Milwaukee, WI) by adding 74 µL of 2.5M CaCl₂ H₂O and 30 µL of 0.1M spermidine (free base) to 300 µL of plasmid DNA and H₂O. The solution was immediately vortexed and the DNA-coated gold particles were allowed to settle. The resulting clear supernatant was removed and the gold particles were resuspended in 1 ml of absolute ethanol. This suspension was diluted with absolute ethanol to obtain 15 mg DNA-coated gold/mL.

Approximately 600 mg of embryogenic callus tissue was spread over the surface of Type II callus maintenance medium as described herein lacking casein hydrolysate and L-proline, but supplemented with 0.2 M sorbitol and 0.2 M mannitol as an osmoticum. Following a 4 h pre-treatment, tissue was transferred to culture dishes containing blasting medium (osmotic media solidified with 20 g/L TC agar (*Phyto*Technology Laboratories, LLC, Shawnee Mission, KS) instead of 7 g/L GELRITE. Helium blasting accelerated suspended DNA-coated gold particles towards and into the prepared tissue targets. The device used was an earlier prototype of that described in US Patent 5,141,131 which is incorporated herein by reference. Tissues were covered with a stainless steel screen (104 µm openings)and placed under a partial vacuum of 25 inches of Hg in the device chamber. The DNA-coated gold particles were further diluted 1:1 with absolute ethanol prior to blasting and were accelerated at the callus targets four times using a helium pressure of 1500 psi, with each blast delivering 20 µL of the DNA/gold suspension. Immediately post-blasting, the tissue was transferred to osmotic media for a 16-24 h recovery period. Afterwards, the tissue was divided into small pieces and transferred to selection medium (maintenance medium lacking casein hydrolysate and L-proline but containing 30 mg/L BASTA® (AgrEvo, Berlin, Germany)). Every four weeks for 3 months, tissue pieces were non-selectively transferred to fresh selection medium. After 7 weeks and up to 22 weeks, callus sectors found proliferating against a background of growth-inhibited tissue were removed and isolated. The resulting BASTA^{®}-resistant tissue was subcultured biweekly onto fresh selection medium. Following western analysis, positive transgenic lines were identified and transferred to regeneration media. Western-negative lines underwent subsequent RNA spot blot analysis to identify negative controls for regeneration.

Regeneration was initiated by transferring callus tissue to cytokinin-based induction medium, which consisted of Murashige and Skoog salts, hereinafter MS salts, and vitamins (Murashige and Skoog, (1962) Physiol. Plant. 15: 473-497) 30 g/L sucrose, 100 mg/L *myo-*inositol, 30 g/L mannitol, 5 mg/L 6-benzylaminopurine, hereinafter BAP, 0.025 mg/L 2,4-D, 30 mg/L BASTA®, and 2.5 g/L GELRITE at pH 5.7. The cultures were placed in low light (125 ft-candles) for one week followed by one week in high light (325 ft-candles). Following a two week induction period, tissue was non-selectively transferred to hormone-free regeneration medium, which was identical to the induction medium except that it lacked 2,4-D and BAP, and was kept in high light. Small (1.5-3 cm) plantlets were removed and placed in 150x25 mm culture tubes containing SH medium (SH salts and vitamins (Schenk and Hildebrandt, (1972) Can. J. Bot. 50:199-204), 10 g/L sucrose, 100 mg/L myo-inositol, 5 mL/L FeEDTA, and 2.5 g/L GELRITE, pH 5.8). Plantlets were transferred to 12 cm pots containing approximately 0.25 kg of METRO-MIX 360 (The Scotts Co. Marysville, OH) in the greenhouse as soon as they exhibited growth and developed a sufficient root system. They were grown with a 16 h photoperiod supplemented by a combination of high pressure sodium and metal halide lamps, and were watered as needed with a combination of three independent Peters Excel fertilizer formulations (Grace-Sierra Horticultural Products Company, Milpitas, CA). At the 6-8 leaf stage, plants were transplanted to five gallon pots containing approximately 4 kg METRO-MIX 360, and grown to maturity.

### EXAMPLE 5

### Characterization Of Transgenic Maize Plants Expressing Photorhabdus Toxin That Confer Insect Control.

### A. Insect Bioassays

A single leaf was sampled from each plant in each test. Eight, 1.4 cm disks were cut from the outer portion of each leaf (approximately 30cm long) avoiding the center vein. Each disk was placed individually into a well of a C-D International 128 well tray (Pitman, NJ.) into which 0.5 ml of a 1.6% aqueous agar solution had been previously pipetted. The solidified agar prevented the leaf disks from drying out. The adaxial surface of the disk was always oriented up.

Five neonate southern corn rootworms, Diabrotica undecimpunctata howardi, were placed on each disk and the wells were sealed with vented plastic lids. The assay was held at 27°C and 40% RH. Larval mortality and live-weight data were collected after 3 days. Data were subjected to analysis of variance and Duncan's multiple range test (α = 0.05) (Proc GLM, SAS Institute Inc., Cary, NC.). Weight data were transformed using a logarithmic function to correct a correlation between the magnitude of the mean and variance.

**TABLE 9 Results of Maize Leaf-disk Test vs SCR**

| Treatment | Mean % Kill (Duncan's) | Mean Survival Weight (mg) (Duncan's) |
|---|---|---|
| 1834 - 11 | 68 A | 0.064 A |
| 1834 - 17 | 44 B | 0.098 B |
| 1834 - 15 | 26 BC | 0.127 C |
| HiII control | 13 C | 0.161 C |

| | | |
|---|---|---|
| Note: Means followed by the same letter are not significantly different based on Duncan's multiple range test (alpha=0.05). Insect groups weighing less than 0.1 mg were set to 0.03 mg instead of zero to conduct a more conservative analysis. Mortality (arcsin(sqrt)) and weight (log10) data were transformed for analyses. | | |

The results shown in Table 9 demonstrated that two events expressing TcdA protein were statistically distinct from control lines bioassayed using SCR neonates by mortality and survival weight criteria. These results demonstrated that southern corn rootworm were functionally effected by feeding on maize plants containing and expressing the tcdA gene. Those plants from 1834-11 were used to generate progeny for testing of inheritability of transgene.

### B. PRODUCTION AND PROGENY TEST OF tcdA TRANSGENIC MAIZE

Origin and growth of progeny plants: Sibling plants 1834-11-07 and 1834-11-08, clonally derived by regeneration from the callus of transgenic maize event 1834-11, were transplanted to the greenhouse and pollinated with inbred OQ414. Seeds obtained from these crosses, comprising seed lots 1834-11-07A and 1834-11-08A, were planted in Rootrainers (1 ½ inch x 2 inch x 8 inch deep, product #647, C. Hummert Intl., Earth City, Mo.) filled with Metro-Mix 360 soilless mix (Scotts Terra-Lite, available from Hummert Intl.) and top irrigated with Hoagland's nutrient solution. (Hoagland's solution contains 229 ppm nitrogen as nitrate, 24.6 ppm nitrogen as ammonium, 26 ppm P, 157 ppm K, 187 ppm Ca, 49 ppm Mg. and 30 ppm Na.)

Greenhouse conditions for this trial were: 16 hour days, daylight supplemented by metal halide lamps as needed to achieve a minimum of 600 ?Einsteins/cm² PAR, and ambient temperature 30 C days, 22 C nights.

Leaves were sampled for protein determination approximately one week after planting. Leaf bioassays were conducted 2-3 weeks after planting; root bioassays were initiated approximately 3 weeks post planting.

Protein analysis of progeny plants: Protein was extracted from leaf and root samples harvested from transgenic plants, line 1834-11 progenies, and non-transformed plants. Each sample was placed on a 1.6 x 4 cm piece of 3M Whatman^{™} paper. The paper was folded lengthwise and inserted in a flexible straw. A volume of 350 µl of an extraction buffer (9.5 ml of 0.2 M NaH₂PO₄, 15.5 ml of 0.2 M Na₂HPO₄, 2 ml of 0.5 M Na₂EDTA, 100 ml of Triton X-100, 1 ml of 10% Sarkosyl, 78 ml of beta-mercaptoethanol, H₂O to bring total volume to 100 ml, 50 µg/ml Antipain, 50 µg/ml Leupeptin, 0.1 mM Chymostatin, 5 µg/ml Pepstatin) was pipetted on to the paper. The straw containing the sample was then passed through a rolling device used for squeezing the extract into a 1.5 ml microcentrifuge tube. The extract was centrifuged for 10 minutes at 14,000 rpm in an Eppendorf refrigerated micro-centrifuge. The supernatant was transferred into a new tube. The amount of the total extractable protein was determined using a standard BioRad Protein Analysis protocol (BioRad Laboratories, Hercules, CA).

The presence of the TcdA protein was visualized by Western blot analysis following a standard procedure for protein separation (Laemmli, 1970). A volume of twenty µl of extract was loaded in each well of 4-20% gradient polyacrylamide gel (Owl Scientific Co., MA) for electrophoresis. Subsequently, the protein was transferred onto a nitrocellulose membrane using a semi-dry electroblotter (Pharmacia LKB Biotechnology, Piscataway, NJ). The membrane was incubated for one hour in TBST-M solution (10% milk in TBST solution; 25 mM Tris HCL pH 7.4, 136 mM NaCl, 2.7 mM KCl, 0.1% Tween 20). Thereafter, the primary antibody (Anti-TcdA in TBST-M) was added. After one hour, the membrane was washed with TBST for five minutes, three times. Then the secondary antibody solution (goat anti-rabbit IgG conjugated to horseradish peroxidase; Bio-Rad Laboratories, in TBST-M) was added to the membrane. After one hour of incubation, the membrane was washed with an excess amount of TBST for 10 minutes, four times. The protein was visualized using the Super Signal^{®} West Pico chemiluminescence method (Pierce Chemical Co., Rockford, IL). The protein blot was exposed on a Hyper-film (Amersham, Arlington Heights, IL) and was developed within 3 minutes. The intensity of the protein band was measured using a densitometer (Molecular Dynamics Inc., Sunnyvale, CA) and compared to standards.

Three of six plants from seed lot 1834-11-07A and three of six plants from seed lot 1834-11-08A produced detectable levels of TcdA protein (Table 1). Approximately 3.8 to 13.3 ppm of TcdA were detected in the leaf blades and 4.1 to 8.4 ppm were detected in the leaf tips of the protein-positive plants. The amounts of TcdA protein detected in the roots were slightly lower than those found in the leaves.

Insect bioassays with progeny plants: Plants were selected for bioassay based on results from Western blot analysis. Twelve (12), 6.4 mm diameter leaf discs were cut from the youngest leaf of each 2 week old seedling. Each disc was placed in a well of a 128-well tray (CD International) containing approximately 0.5mL of a solidified 2% agar in water solution. Two neonate southern corn rootworm, *Diabrotica undecimpunctata howardi* (Barber)(SCR); were placed in each well with a leaf disc. Trays were covered with perforated lids and maintained under a controlled environment for 3 days (28 C; 16 hours light:8 hours dark; approx. 60% relative humidity). Living larvae from 4 leaf discs were pooled and weighed producing 3 weight determinations per plant. Average weights were calculated by dividing the pooled weight by the number of survivors. Differences in average weights of SCR fed leaf discs from protein positive and protein negative plants were assessed using analysis of variance on the natural log-transformed average weights (Minitab, v. 12.2, Minitab Inc., State College, PA).

Root bioassays were initiated approximately 1 week after the initiation of the leaf disc bioassays. Approximately 24h prior to eclosion, SCR eggs were suspended in a 0.15% solution of agar in water to a concentration of 100 eggs/ml. Plants were inoculated with SCR eggs by pipetting 2.0 ml of the egg suspension (ie., approximately 200 eggs) just below the soil surface at the base of each plant. Two weeks after inoculation, plants were removed from their Rootrainer pots, their roots washed free of potting mix, and scored for rootworm damage based on a 1 (resistant) to 9 (susceptible) rating system (Welch, 1977). The results of the root ratings were examined using non-parametric tests to determine if the distribution of root ratings from the protein positive plants was the same as the distribution of the ratings from the protein negative plants. Testing was done at the 5% significance level. (StatXact v.3, CYTEL Software Corporation, Cambridge MA)

Results from leaf and root bioassays of tcdA protein positive and protein negative progeny plants are summarized in Table 10. The average weights of SCR larvae fed leaf discs from protein positive plants were significantly lower than those of larvae fed leaf discs from protein negative plants (*F* = 4.6; *d.f.* = 1, 34; *P* ≤ 0.001. The Kolmogorov-Smirnov 2 sample test (p=0.04) and the Wald Wolfowitz runs test (p=0.001) indicated that the protein positive and protein negative root rating distributions were not similar. The Wilcoxon- Mann-Whitney test (p=0.0206) and the Normal Scores test (p=0.206) indicated that the average score for the protein positive plants was lower than the average root rating from the protein negative plants.

**Table 10. Protein analysis and insect bioassay results with progeny of TcdA transgenic maize.**

| Plant Number | TcdA Protein | Leaf Disc Bioassay Avg. Wt. (mg) | Root Bioassay Root Rating (1-9) |
|---|---|---|---|
| 1834-11-07A-30 | PRO- | 0.190 | 8 |
| 1834-11-08A-21 | PRO- | 0.196 | 9 |
| 1834-11-08A-16 | PRO- | 0.195 | 9 |
| 1834-11-08A-14 | PRO- | 0.137 | 9 |
| 1834-11-07A-22 | PRO- | 0.208 | 9 |
| 1834-11-07A-20 | PRO- | 0.175 | 9 |
| 1834-11-07A-26 | PRO+ | 0.118 | 9 |
| 1834-11-08A-17 | PRO+ | 0.132 | 8 |
| 1834-11-07A-14 | PRO+ | 0.110 | 2 |
| 1834-11-07A-11 | PRO+ | 0.106 | 4 |
| 1834-11-08A-28 | PRO+ | 0.129 | 8 |
| 1834-11-08A-27 | PRO+ | 0.108 | 4 |

| | | | |
|---|---|---|---|
| DNA analysis of progeny plants: Leaf samples from 1834-11.7A and 1834-11.8A progeny plants were in conical 50 ml polypropylene tubes and dried in a Labconco Freeze Dry Lyophilizer (Kansas City, MO) for 1-2 days. Lyophilized leaves were then ground in a Tecator Cyclotec 1093 Sample mill grinder (Hoganas, Sweden) and stored at -20C. Genomic DNA was extracted by the following procedure: (1) to a 25 ml Conical tube containing 300-500 mg of ground tissue, 9 ml of CTAB (cetyl trimethylammonium bromide solution) was added, and incubated at 65°C for 1 hour; (2) 4.5 ml of chloroform: octanol (24:1) was added and mixed gently for 5 minutes; (3) samples were centrifuged at 2000 rpm and DNA was precipitated from the supernatant with an equal volume of isopropanol; (4) DNA was collected on a glass hook, washed in ethanol, and dissolved in TE (10 mM Tris.HCl, 0.5 mM EDTA, pH8.0). | | | |

Genomic DNA was digested at 37 °C. for 2 hours in an Eppendorf tube containing the following mixture: 8 µl of 800ug/ml DNA, 2 µl 1 mg/ml BSA (Bovine serum albumin),2 µl 10x buffer, 1 µl *Sac*I*,* 1 µl *Eco*RI, and 6 µl H2O. Digested DNA samples were electrophoresed overnight at 40 mA in a 0.85% SeaKem LE agarose gel(FMC, Rockland, Maine). The gel was blotted onto Millipore Immobilon-Ny+ (Bedford, MA) membrane overnight in 20X SSC (NaCl 175.2 g/l, Na citrate 88 g/l). The probe DNA was cut with BamHI/SacI (NEB, Beverly, MA) from pDAH1551 plasmid, which released a 7356 bp fragment containing the open reading frame of the rebuilt *tcdA* gene. This 7356 bp fragment was labeled with P32 using a Stratagene Prime-it RmT dCTP-Labeling Reactions kit (La Jolla, CA) and used for Southern hybridization. Hybridization was conducted in hybridization buffer (10% polyethylene glycol, 7% SDS [Sodium dodecyl sulfate], 0.6X SSC, 10 mM NaPO₄, 5 mM EDTA, 10 µg/ml denatured salmon sperm) at 60 °C overnight. After hybridization, the membrane was washed with 10X SSC plus 0.1% SDS at 60 °C for 30 min and exposed to X ray film (Hyperfilm® MP, Amershan Life Sciences, Piscataway, NJ) for 1-2 days.

Results summarized indicate that a pattern of 8 hybridizing bands (the size of the expected fragment and larger) cosegregated with protein expression in 50% of all progeny assayed. These results are characteristic of a complex insertion at a single site. All seedlings containing the insert also expressed toxin protein.

### Example 6

### Transformation Of Rice With a Vector Carrying Plasmid pDAB1553 Encoding Photorhabdus Toxins

### A. Plasmid pDAB1553

Plasmid pDAB1553 containing *tcdA* driven by the maize ubiquitin1 promoter and *hpt* (hygromycin phosphotransferase providing resistance to the antibiotic hygromycin) under the control of 35T (a modified 35S promoter), was used for transformation.

Preparation of rice transformation vectors was accomplished in two steps. First, a modified plant-optimized *tcdA* coding region was ligated into a rice plant expression cassette plasmid. In this step, the coding region was placed under the transcriptional control of a promoter functional in plant cells. RNA transcription termination and polyadenylation were mediated by a downstream copy of the terminator region from the *Agrobacterium* nopaline synthase gene. One plasmid designed to function in this role is plasmid pDAB1538 (described in the section on maize transformation vectors). In the second step, the complete gene comprised of the promoter, coding region, and terminator region was ligated to a rice plant transformation vector that contained a plant expressible selectable marker gene which allowed the selection of transformed rice plant cells amongst a background of nontransformed cells. An example of such a vector is pDAB354-Not1.

It is a feature of pDAB354-Not1 that the hygromycin phosphotransferase protein, which has as its substrate hygromycin B and related compounds, is produced in plant cells through transcription of its coding region mediated by the Cauliflower Mosaic Virus 35S promoter and that termination of transcription plus polyadenylation are mediated by the nopaline synthase terminator region. It is further a feature of pDAB354-Not1 that any DNA fragment containing flanking *Not*I sites can be cloned into the unique *Not*I site of pDAB354-Not1, thus physically linking the introduced DNA fragment to the aforementioned selectable marker gene.

To prepare a plant-expressible gene to produce the non-targeted TcdA protein in rice plant cells, DNA of a plasmid (pA0H_4-OPTI) containing the plant-optimized tcdA coding region, (SEQ ID No:3) was cleaved with restriction enzymes *Nco*I and *Sac*I, and the large 7550 bp fragment was ligated to similarly-cut DNA of plasmid pDAB1538 to produce plasmid pDAB1551. DNA of pDAB1551 was then digested with *Not*I, and the large 9933 bp fragment was ligated to *Not*I digested DNA of pDAB354-Not1 to produce plasmid pDAB1553.

It is a feature of plasmid pDAB1553 that the ubi1 and 35S promoters are encoded on the same DNA strand.

### B. Production of Rice transgenics

For initiation of embryogenic callus, mature seeds of a *Japonica* cultivar, Taipei 309 were dehusked and surface-sterilized in 70% ethanol for 2-5 min. followed by a 30-45 min soak in 50% commercial bleach (2.6% sodium hypochlorite) with a few drops of 'Liquinox' soap. The seeds were then rinsed 3 times in sterile distilled water and placed on filter paper before transferring to 'callus induction' medium (i.e., NB). The NB medium consisted of N6 macro elements (Chu, 1978, The N6 medium and its application to anther culture of cereal crops. Proc. Symp. Plant Tissue Culture, Peking Press, p43-56), B5 micro elements and vitamins (Gamborg et al., 1968, Nutrient requirements of suspension cultures of soybean root cells. Exp. Cell Res. 50: 151-158), 300 mg/L casein hydrolysate, 500 mg/L L-proline, 500 mg/L L-glutamine, 30 g/L sucrose, 2 mg/L 2,4-dichloro-phenoxyacetic acid (2,4-D), and 2.5 g/L gelrite (Schweizerhall, NJ) with the pH adjusted to 5.8. The mature seed cultured on 'induction' media were incubated in the dark at 28°C. After 3 weeks of culture, the emerging primary callus induced from the scutellar region of mature embryo was transferred to fresh NB medium for further maintenance.

About 140 µg of plasmid pDAB1553 DNA was precipitated onto 60 mg of 1.0 micron (Bio-Rad) gold particles as described herein.

For helium blasting, actively growing embryogenic callus cultures, 2-4 mm in size, were subjected to a high osmoticum treatment. This treatment included placing of callus on NB medium with 0.2 M mannitol and 0.2 M sorbitol (Vain et al., 1993, Osmoticum treatment enhances particle bombardment-mediated transient and stable transformation of maize. Plant Cell Rep. 12: 84-88) for 4 h before helium blasting. Following osmoticum treatment, callus cultures were transferred to 'blasting' medium (NB+2% agar) and covered with a stainless steel screen (230 micron). The callus cultures were blasted at 2,000 psi helium pressures twice per target. After blasting, callus was transferred back to the media with high osmoticum overnight before placing on selection medium, which consisted NB medium with 30 mg/L hygromycin. After 2 weeks, the cultures were transferred to fresh selection medium with a higher concentration of selection agent, i.e., NB+50mg/L hygromycin (Li et al., 1993, An improved rice transformation system using the biolistic method. Plant Cell Rep. 12: 250-255).

Compact, white-yellow, embryogenic callus cultures, recovered on NB+50 mg/L hygromycin, were regenerated by transferring to 'pre-regeneration' (PR) medium + 50 mg/L hygromycin. The PR medium consisted of NB medium with 2 mg/L benzyl aminopurine (BAP), 1 mg/L naphthalene acetic acid (NAA), and 5 mg/L abscisic acid (ABA). After 2 weeks of culture in the dark, they were transferred to 'regeneration' (RN) medium. The composition of RN medium is NB medium with 3 mg/L BAP, and 0.5 mg/L NAA. The cultures on RN medium were incubated for 2 weeks at 28° C under high fluorescent light (325-ft-candles). The plantlets with 2 cm shoot were transferred to 1/2 MS medium (Murashige and Skoog, 1962, A revised medium for rapid growth and bioassays with tobacco tissue cultures. Physiol. Plant.15:473-497) with 1/2 B5 vitamins, 10 g/L sucrose, 0.05 mg/L NAA, 50 mg/L hygromycin and 2.5 g/L gelrite adjusted to pH 5.8 in magenta boxes. When plantlets were established with well-developed root systems, they were transferred to soil (1 metromix: 1 top soil) and raised in the greenhouse (29/24°C day/night cycle, 50-60% humidity, 12 h photoperiod) until maturity.

### EXAMPLE 7

### Chacterization Of Transgenic Rice Plants Expressing Photorhabdus Toxin That Confer Insect Control.

### A. Insect bioassays

Insect bioassays were performed using leaf discs and shown to be highly effective in controlling Southern corn rootworm. *Diabrotica undecimpunctata howardi* eggs are obtained from French Ag Research and hatched in petri dishes held at 28.5°C and 40% RH. The aerial parts are sampled from the transgenic plants and placed, singly into inverted petri dishes (100x15mm) containing 15ml of 1.6% aqueous agar in the bottom to provide humidity and filter paper in the top to absorb condensation. These preparations are infested with five neonate larvae per dish and held at 28.5°C and 40% RH for 3 days. Mortality and larval weights are recorded. Weight data were transformed using a logarithmic function to correct a correlation between the magnitude of the mean and variance.

**Table 11**

| Treatment | Average Survivor Weight in mg¹ (Duncan's Grouping) | Presence TcdA greenhouse-grown plants (number of +/number of plants tested) |
|---|---|---|
| GUS Control | 0.390 A | - |
| 1553-33 | 0.170 BCD | ++ |
| 1553-44 | 0.167 BCD | +++ |
| 1553-62 | 0.125 CD | +++ |
| 1553-41 | 0.100 D | +++ |

| | | |
|---|---|---|
| Note: Means followed by the same letter are not significantly different based on Duncan's multiple range test (alpha=0.05). | | |

Insect groups weighing less than 0.1 mg were set to 0.03 mg instead of zero to conduct a more conservative analysis. Weight data were transformed (Log10) for analyses. A single replicate was used on each of three test dates. Plants were sampled from magenta boxes.

The results demonstrate that in leaf disc bioassays, several rice events derived by transformation with *tcdA* gene were demonstrated to statistically have a functional affect on corn rootworm neonate.

## Claims

1. An isolated nucleic acid of SEQ ID No. 3.

2. A transgenic monocot cell having a genome comprising SEQ ID No. 3.

3. A transgenic dicot cell having a genome comprising SEQ ID No. 3.

4. A transgenic plant with a genome comprising a nucleic acid of SEQ ID No. 3 that imparts insect resistance.

5. A transgenic plant or cell as claimed in any of one of Claims 2 to 4, wherein said genome further comprises SEQ ID No. 4.

6. A transgenic plant of Claims 4 or 5, wherein the plant is rice.

7. A transgenic plant of Claims 4 or 5, wherein the plant is maize.

8. A transgenic plant of Claims 4 to 5, wherein the plant is tobacco.

## Patentansprüche

1. Isolierte Nukleinsäure der SEQ ID No. 3.

2. Transgene monokotyledone Zelle, die ein Genom besitzt, das die SEQ ID No. 3 umfasst.

3. Transgene dikotyledone Zelle, die ein Genom besitzt, das die SEQ ID No. 3 umfasst.

4. Transgene Pflanze mit einem Genom, das eine Nukleinsäure der SEQ ID No. 3 umfasst, die Insektenresistenz verleiht.

5. Transgene Pflanze oder Zelle nach einem der Ansprüche 2 bis 4, wobei das Genom ferner die SEQ ID No. 4 umfasst.

6. Transgene Pflanze nach Anspruch 4 oder 5, wobei die Pflanze Reis ist.

7. Transgene Pflanze nach Anspruch 4 oder 5, wobei die Pflanze Mais ist.

8. Transgene Pflanze nach Anspruch 4 oder 5, wobei die Pflanze Tabak ist.

## Revendications

1. Acide nucléique isolé de SEQ ID No.3.

2. Cellule monocotylédone transgénique ayant un génome comprenant la séquence SEQ ID No.3.

3. Cellule dicotylédone transgénique ayant un génome comprenant la séquence SEQ ID No.3.

4. plante transgénique dont le génome comprend un acide nucléique de la séquence SEQ ID No.3 qui est impliquée dans la résistance insecticide.

5. Plante ou cellule transgénique telle que revendiquée dans l'une quelconque des revendications 2 à 4, dans laquelle ledit génome comprend en outre la séquence SEQ ID No.4.

6. Plante transgénique selon la revendication 4 ou 5, qui est du riz.

7. Plante transgénique selon la revendication 4 ou 5, qui est du maïs.

8. Plante transgénique selon la revendication 4 ou 5, qui est du tabac.
